# EUROPEAN PATENT APPLICATION

(11) **EP 1 428 893 A2**
(43) Date of publication of application: **16.06.2004**
(21) Application number: 03256411.4
(22) Date of filing: 10.10.2003
(51) Int. Cl.: C12Q 1/68, G01N 33/50, C12N 15/11, A61K 38/02, A61K 39/395, A61P 17/14

(54) **Methods of and compositions for modulating hair growth via p-cadherin modulators**

(30) Priority: 15.10.2002 US 418163 P
(71) Applicant: Sprecher, Eli, 36056 Tivon (IL); Bergman, Reuven, 34406 Haifa (IL)
(72) Inventor: Sprecher, Eli, 36056 Tivon (IL); Bergman, Reuven, 34406 Haifa (IL)
(74) Representative: Smart, Peter John

(57) **Abstract**

A method of identifying a hair growth modulator (i.e. hair growth inhibitor or inducer) which comprises identifying a P-cadherin modulator (i.e. P-cadherin inhibitor or inducer); and testing whether the P-cadherin modulator is functional as a hair growth modulator.

SEQUENCE LISTING

<110> Sprecher, Eli
      Bergman, Reuven

<120> METHODS OF AND COMPOSITIONS FOR MODULATING HAIR GROWTH VIA
      P-CADHERIN MODULATORS

<130> 26466

<160> 75

<170> PatentIn version 3.1

<210> 1

<211> 790

<212> PRT

<213> Homo sapiens

<400> 1

Met Arg Thr Tyr Arg Tyr Phe Leu Leu Leu Phe Trp Val Gly Gln Pro
1               5                   10                  15

Tyr Pro Thr Leu Ser Thr Pro Leu Ser Lys Arg Thr Ser Gly Phe Pro
            20                  25                  30

Ala Lys Lys Arg Ala Leu Glu Leu Ser Gly Asn Ser Lys Asn Glu Leu
        35                  40                  45

Asn Arg Ser Lys Arg Ser Trp Met Trp Asn Gln Phe Phe Leu Leu Glu
    50                  55                  60

Glu Tyr Thr Gly Ser Asp Tyr Gln Tyr Val Gly Lys Leu His Ser Asp
65                  70                  75                  80

Gln Asp Arg Gly Asp Gly Ser Leu Lys Tyr Ile Leu Ser Gly Asp Gly
                85                  90                  95

Ala Gly Asp Leu Phe Ile Ile Asn Glu Asn Thr Gly Asp Ile Gln Ala
            100                 105                 110

Thr Lys Arg Leu Asp Arg Glu Glu Lys Pro Val Tyr Ile Leu Arg Ala
        115                 120                 125

Gln Ala Ile Asn Arg Arg Thr Gly Arg Pro Val Glu Pro Glu Ser Glu
    130                 135                 140

Phe Ile Ile Lys Ile His Asp Ile Asn Asp Asn Glu Pro Ile Phe Thr
145                 150                 155                 160

Lys Glu Val Tyr Thr Ala Thr Val Pro Glu Met Ser Asp Val Gly Thr
                165                 170                 175

Phe Val Val Gln Val Thr Ala Thr Asp Ala Asp Asp Pro Thr Tyr Gly
            180                 185                 190

Asn Ser Ala Lys Val Val Tyr Ser Ile Leu Gln Gly Gln Pro Tyr Phe
        195                 200                 205

Ser Val Glu Ser Glu Thr Gly Ile Ile Lys Thr Ala Leu Leu Asn Met
    210                 215                 220

Asp Arg Glu Asn Arg Glu Gln Tyr Gln Val Val Ile Gln Ala Lys Asp
225                 230                 235                 240

Met Gly Gly Gln Met Gly Gly Leu Ser Gly Thr Thr Thr Val Asn Ile
                245                 250                 255

Thr Leu Thr Asp Val Asn Asp Asn Pro Pro Arg Phe Pro Gln Ser Thr
            260                 265                 270

Tyr Gln Phe Lys Thr Pro Glu Ser Ser Pro Pro Gly Thr Pro Ile Gly
        275                 280                 285

Arg Ile Lys Ala Ser Asp Ala Asp Val Gly Glu Asn Ala Glu Ile Glu
    290                 295                 300

Tyr Ser Ile Thr Asp Gly Glu Gly Leu Asp Met Phe Asp Val Ile Thr
305                 310                 315                 320

Asp Gln Glu Thr Gln Glu Gly Ile Ile Thr Val Lys Lys Leu Leu Asp
                325                 330                 335

Phe Glu Lys Lys Lys Val Tyr Thr Leu Lys Val Glu Ala Ser Asn Pro
            340                 345                 350

Tyr Val Glu Pro Arg Phe Leu Tyr Leu Gly Pro Phe Lys Asp Ser Ala
        355                 360                 365

Thr Val Arg Ile Val Val Glu Asp Val Asp Glu Pro Pro Val Phe Ser
    370                 375                 380

Lys Leu Ala Tyr Ile Leu Gln Ile Arg Glu Asp Ala Gln Ile Asn Thr

385                 390                 395                 400

Thr Ile Gly Ser Val Thr Ala Gln Asp Pro Asp Ala Ala Arg Asn Pro
                405                 410                 415

Val Lys Tyr Ser Val Asp Arg His Thr Asp Met Asp Arg Ile Phe Asn
            420                 425                 430

Ile Asp Ser Gly Asn Gly Ser Ile Phe Thr Ser Lys Leu Leu Asp Arg
        435                 440                 445

Glu Thr Leu Leu Trp His Asn Ile Thr Val Ile Ala Thr Glu Ile Asn
    450                 455                 460

Asn Pro Lys Gln Ser Ser Arg Val Pro Leu Tyr Ile Lys Val Leu Asp
465                 470                 475                 480

Val Asn Asp Asn Ala Pro Glu Phe Ala Glu Phe Tyr Glu Thr Phe Val
                485                 490                 495

Cys Glu Lys Ala Lys Ala Asp Gln Leu Ile Gln Thr Leu His Ala Val
            500                 505                 510

Asp Lys Asp Asp Pro Tyr Ser Gly His Gln Phe Ser Phe Ser Leu Ala
        515                 520                 525

Pro Glu Ala Ala Ser Gly Ser Asn Phe Thr Ile Gln Asp Asn Lys Asp
    530                 535                 540

Asn Thr Ala Gly Ile Leu Thr Arg Lys Asn Gly Tyr Asn Arg His Glu
545                 550                 555                 560

Met Ser Thr Tyr Leu Leu Pro Val Val Ile Ser Asp Asn Asp Tyr Pro
                565                 570                 575

Val Gln Ser Ser Thr Gly Thr Val Thr Val Arg Val Cys Ala Cys Asp
            580                 585                 590

His His Gly Asn Met Gln Ser Cys His Ala Glu Ala Leu Ile His Pro
        595                 600                 605

Thr Gly Leu Ser Thr Gly Ala Leu Val Ala Ile Leu Leu Cys Ile Val
    610                 615                 620

Ile Leu Leu Val Thr Val Val Leu Phe Ala Ala Leu Arg Arg Gln Arg
625                 630                 635                 640

Lys Lys Glu Pro Leu Ile Ile Ser Lys Glu Asp Ile Arg Asp Asn Ile
                645                 650                 655

Val Ser Tyr Asn Asp Glu Gly Gly Gly Glu Glu Asp Thr Gln Ala Phe
            660                 665                 670

Asp Ile Gly Thr Leu Arg Asn Pro Glu Ala Ile Glu Asp Asn Lys Leu
        675                 680                 685

Arg Arg Asp Ile Val Pro Glu Ala Leu Phe Leu Pro Arg Arg Thr Pro
    690                 695                 700

Thr Ala Arg Asp Asn Thr Asp Val Arg Asp Phe Ile Asn Gln Arg Leu
705                 710                 715                 720

Lys Glu Asn Asp Thr Asp Pro Thr Ala Pro Pro Tyr Asp Ser Leu Ala
                725                 730                 735

Thr Tyr Ala Tyr Glu Gly Thr Gly Ser Val Ala Asp Ser Leu Ser Ser
            740                 745                 750

Leu Glu Ser Val Thr Thr Asp Ala Asp Gln Asp Tyr Asp Tyr Leu Ser
        755                 760                 765

Asp Trp Gly Pro Arg Phe Lys Lys Leu Ala Asp Met Tyr Gly Gly Val
    770                 775                 780

Asp Ser Asp Lys Asp Ser
785                 790

<210> 2

<211> 794

<212> PRT

<213> Homo sapiens

<400> 2

<210>  3

<211>  799

<212>  PRT

<213>  Homo sapiens

<400>  3

Met Pro Glu Arg Leu Ala Glu Met Leu Leu Asp Leu Trp Thr Pro Leu
1               5                   10                  15

Ile Ile Leu Trp Ile Thr Leu Pro Pro Cys Ile Tyr Met Ala Pro Met
            20                  25                  30

Asn Gln Ser Gln Val Leu Met Ser Gly Ser Pro Leu Glu Leu Asn Ser
        35                  40                  45

Leu Gly Glu Glu Gln Arg Ile Leu Asn Arg Ser Lys Arg Gly Trp Val
    50                  55                  60

Trp Asn Gln Met Phe Val Leu Glu Glu Phe Ser Gly Pro Glu Pro Ile
65                  70                  75                  80

Leu Val Gly Arg Leu His Thr Asp Leu Asp Pro Gly Ser Lys Lys Ile
                85                  90                  95

Lys Tyr Ile Leu Ser Gly Asp Gly Ala Gly Thr Ile Phe Gln Ile Asn
            100                 105                 110

Asp Val Thr Gly Asp Ile His Ala Ile Lys Arg Leu Asp Arg Glu Glu
        115                 120                 125

Lys Ala Glu Tyr Thr Leu Thr Ala Gln Ala Val Asp Trp Glu Thr Ser
    130                 135                 140

Lys Pro Leu Glu Pro Pro Ser Glu Phe Ile Ile Lys Val Gln Asp Ile
145                 150                 155                 160

Asn Asp Asn Ala Pro Glu Phe Leu Asn Gly Pro Tyr His Ala Thr Val
                165                 170                 175

Pro Glu Met Ser Ile Leu Gly Thr Ser Val Thr Asn Val Thr Ala Thr
            180                 185                 190

Asp Ala Asp Asp Pro Val Tyr Gly Asn Ser Ala Lys Leu Val Tyr Ser
        195                 200                 205

Ile Leu Glu Gly Gln Pro Tyr Phe Ser Ile Glu Pro Glu Thr Ala Ile
    210                 215                 220

Ile Lys Thr Ala Leu Pro Asn Met Asp Arg Glu Ala Lys Glu Glu Tyr
225                 230                 235                 240

Leu Val Val Ile Gln Ala Lys Asp Met Gly Gly His Ser Gly Gly Leu
                245                 250                 255

Ser Gly Thr Thr Thr Leu Thr Val Thr Leu Thr Asp Val Asn Asp Asn
            260                 265                 270

Pro Pro Lys Phe Ala Gln Ser Leu Tyr His Phe Ser Val Pro Glu Asp
        275                 280                 285

Val Val Leu Gly Thr Ala Ile Gly Arg Val Lys Ala Asn Asp Gln Asp
    290                 295                 300

Ile Gly Glu Asn Ala Gln Ser Ser Tyr Asp Ile Ile Asp Gly Asp Gly
305                 310                 315                 320

Thr Ala Leu Phe Glu Ile Thr Ser Asp Ala Gln Ala Gln Asp Gly Ile
                325                 330                 335

Ile Arg Leu Arg Lys Pro Leu Asp Phe Glu Thr Lys Lys Ser Tyr Thr
            340                 345                 350

Leu Lys Val Glu Ala Ala Asn Val His Ile Asp Pro Arg Phe Ser Gly
        355                 360                 365

Arg Gly Pro Phe Lys Asp Thr Ala Thr Val Lys Ile Val Val Glu Asp
    370                 375                 380

Ala Asp Glu Pro Pro Val Phe Ser Ser Pro Thr Tyr Leu Leu Glu Val
385                 390                 395                 400

His Glu Asn Ala Ala Leu Asn Ser Val Ile Gly Gln Val Thr Ala Arg
                405                 410                 415

Asp Pro Asp Ile Thr Ser Ser Pro Ile Arg Phe Ser Ile Asp Arg His
            420                 425                 430

Thr Asp Leu Glu Arg Gln Phe Asn Ile Asn Ala Asp Asp Gly Lys Ile
        435                 440                 445

Thr Leu Ala Thr Pro Leu Asp Arg Glu Leu Ser Val Trp His Asn Ile
    450                 455                 460

Thr Ile Ile Ala Thr Glu Ile Arg Asn His Ser Gln Ile Ser Arg Val
465                 470                 475                 480

Pro Val Ala Ile Lys Val Leu Asp Val Asn Asp Asn Ala Pro Glu Phe
                485                 490                 495

Ala Ser Glu Tyr Glu Ala Phe Leu Cys Glu Asn Gly Lys Pro Gly Gln
            500                 505                 510

Val Ile Gln Thr Val Ser Ala Met Asp Lys Asp Asp Pro Lys Asn Gly
        515                 520                 525

His Tyr Phe Leu Tyr Ser Leu Leu Pro Glu Met Val Asn Asn Pro Asn
    530                 535                 540

Phe Thr Ile Lys Lys Asn Glu Asp Asn Ser Leu Ser Ile Leu Ala Lys
545                 550                 555                 560

His Asn Gly Phe Asn Arg Gln Lys Gln Glu Val Tyr Leu Leu Pro Ile
                565                 570                 575

Ile Ile Ser Asp Ser Gly Asn Pro Pro Leu Ser Ser Thr Ser Thr Leu
            580                 585                 590

Thr Ile Arg Val Cys Gly Cys Ser Asn Asp Gly Val Val Gln Ser Cys
        595                 600                 605

Asn Val Glu Ala Tyr Val Leu Pro Ile Gly Leu Ser Met Gly Ala Leu
    610                 615                 620

Ile Ala Ile Leu Ala Cys Ile Ile Leu Leu Leu Val Ile Val Val Leu
625                 630                 635                 640

Phe Val Thr Leu Arg Arg His Lys Asn Glu Pro Leu Ile Ile Lys Asp
                645                 650                 655

Asp Glu Asp Val Arg Glu Asn Ile Ile Arg Tyr Asp Asp Glu Gly Gly
            660                 665                 670

Gly Glu Glu Asp Thr Glu Ala Phe Asp Ile Ala Thr Leu Gln Asn Pro
        675                 680                 685

Asp Gly Ile Asn Gly Phe Leu Pro Arg Lys Asp Ile Lys Pro Asp Leu
    690                 695                 700

Gln Phe Met Pro Arg Gln Gly Leu Ala Pro Val Pro Asn Gly Val Asp
705                 710                 715                 720

Val Asp Glu Phe Ile Asn Val Arg Leu His Glu Ala Asp Asn Asp Pro
                725                 730                 735

Thr Ala Pro Pro Tyr Asp Ser Ile Gln Ile Tyr Gly Tyr Glu Gly Arg
            740                 745                 750

Gly Ser Val Ala Gly Ser Leu Ser Ser Leu Glu Ser Thr Thr Ser Asp
        755                 760                 765

Ser Asp Gln Asn Phe Asp Tyr Leu Ser Asp Trp Gly Pro Arg Phe Lys
    770                 775                 780

Arg Leu Gly Glu Leu Tyr Ser Val Gly Glu Ser Asp Lys Glu Thr
785                 790                 795

<210> 4

<211> 796

<212> PRT

<213> Homo sapiens

<400> 4

Met Lys Glu Asn Tyr Cys Leu Gln Ala Ala Leu Val Cys Leu Gly Met
1               5                   10                  15

Leu Cys His Ser His Ala Phe Ala Pro Glu Arg Arg Gly His Leu Arg
            20                  25                  30

Pro Ser Phe His Gly His His Glu Lys Gly Lys Glu Gly Gln Val Leu
        35                  40                  45

Gln Arg Ser Lys Arg Gly Trp Val Trp Asn Gln Phe Phe Val Ile Glu
    50                  55                  60

Glu Tyr Thr Gly Pro Asp Pro Val Leu Val Gly Arg Leu His Ser Asp
65                  70                  75                  80

Ile Asp Ser Gly Asp Gly Asn Ile Lys Tyr Ile Leu Ser Gly Glu Gly
                85                  90                  95

Ala Gly Thr Ile Phe Val Ile Asp Asp Lys Ser Gly Asn Ile His Ala
            100                 105                 110

Thr Lys Thr Leu Asp Arg Glu Glu Arg Ala Gln Tyr Thr Leu Met Ala
        115                 120                 125

Gln Ala Val Asp Arg Asp Thr Asn Arg Pro Leu Glu Pro Pro Ser Glu
    130                 135                 140

Phe Ile Val Lys Val Gln Asp Ile Asn Asp Asn Pro Pro Glu Phe Leu
145                 150                 155                 160

His Glu Thr Tyr His Ala Asn Val Pro Glu Arg Ser Asn Val Gly Thr
                165                 170                 175

Ser Val Ile Gln Val Thr Ala Ser Asp Ala Asp Asp Pro Thr Tyr Gly
            180                 185                 190

Asn Ser Ala Lys Leu Val Tyr Ser Ile Leu Glu Gly Gln Pro Tyr Phe
        195                 200                 205

Ser Val Glu Ala Gln Thr Gly Ile Ile Arg Thr Ala Leu Pro Asn Met
    210                 215                 220

Asp Arg Glu Ala Lys Glu Glu Tyr His Val Val Ile Gln Ala Lys Asp
225                 230                 235                 240

Met Gly Gly His Met Gly Gly Leu Ser Gly Thr Thr Lys Val Thr Ile
                245                 250                 255

Thr Leu Thr Asp Val Asn Asp Asn Pro Pro Lys Phe Pro Gln Arg Leu
            260                 265                 270

Tyr Gln Met Ser Val Ser Glu Ala Ala Val Pro Gly Glu Glu Val Gly
        275                 280                 285

Arg Val Lys Ala Lys Asp Pro Asp Ile Gly Glu Asn Gly Leu Val Thr
    290                 295                 300

Tyr Asn Ile Val Asp Gly Asp Gly Met Glu Ser Phe Glu Ile Thr Thr
305                 310                 315                 320

Asp Tyr Glu Thr Gln Glu Gly Val Ile Lys Leu Lys Lys Pro Val Asp
                325                 330                 335

Phe Glu Thr Glu Arg Ala Tyr Ser Leu Lys Val Glu Ala Ala Asn Val
            340                 345                 350

His Ile Asp Pro Lys Phe Ile Ser Asn Gly Pro Phe Lys Asp Thr Val
        355                 360                 365

Thr Val Lys Ile Ser Val Glu Asp Ala Asp Glu Pro Pro Met Phe Leu
    370                 375                 380

Ala Pro Ser Tyr Ile His Glu Val Gln Glu Asn Ala Ala Ala Gly Thr
385                 390                 395                 400

Val Val Gly Arg Val His Ala Lys Asp Pro Asp Ala Ala Asn Ser Pro
                405                 410                 415

Ile Arg Tyr Ser Ile Asp Arg His Thr Asp Leu Asp Arg Phe Phe Thr
            420                 425                 430

Ile Asn Pro Glu Asp Gly Phe Ile Lys Thr Thr Lys Pro Leu Asp Arg
        435                 440                 445

Glu Glu Thr Ala Trp Leu Asn Ile Thr Val Phe Ala Ala Glu Ile His
    450                 455                 460

Asn Arg His Gln Glu Ala Gln Val Pro Val Ala Ile Arg Val Leu Asp
465                 470                 475                 480

Val Asn Asp Asn Ala Pro Lys Phe Ala Ala Pro Tyr Glu Gly Phe Ile
                485                 490                 495

Cys Glu Ser Asp Gln Thr Lys Pro Leu Ser Asn Gln Pro Ile Val Thr
            500                 505                 510

Ile Ser Ala Asp Asp Lys Asp Asp Thr Ala Asn Gly Pro Arg Phe Ile
        515                 520                 525

Phe Ser Leu Pro Pro Glu Ile Ile His Asn Pro Asn Phe Thr Val Arg
    530                 535                 540

Asp Asn Arg Asp Asn Thr Ala Gly Val Tyr Ala Arg Arg Gly Gly Phe
545                 550                 555                 560

Ser Arg Gln Lys Gln Asp Leu Tyr Leu Leu Pro Ile Val Ile Ser Asp
                565                 570                 575

Gly Gly Ile Pro Pro Met Ser Ser Thr Asn Thr Leu Thr Ile Lys Val
            580                 585                 590

Cys Gly Cys Asp Val Asn Gly Ala Leu Leu Ser Cys Asn Ala Glu Ala
        595                 600                 605

Tyr Ile Leu Asn Ala Gly Leu Ser Thr Gly Ala Leu Ile Ala Ile Leu
    610                 615                 620

Ala Cys Ile Val Ile Leu Leu Val Ile Val Val Leu Phe Val Thr Leu
625                 630                 635                 640

Arg Arg Gln Lys Lys Glu Pro Leu Ile Val Phe Glu Glu Glu Asp Val
                645                 650                 655

Arg Glu Asn Ile Ile Thr Tyr Asp Asp Glu Gly Gly Gly Glu Glu Asp
            660                 665                 670

Thr Glu Ala Phe Asp Ile Ala Thr Leu Gln Asn Pro Asp Gly Ile Asn
        675                 680                 685

Gly Phe Ile Pro Arg Lys Asp Ile Lys Pro Glu Tyr Gln Tyr Met Pro
    690                 695                 700

Arg Pro Gly Leu Arg Pro Ala Pro Asn Ser Val Asp Val Asp Asp Phe
705                 710                 715                 720

Ile Asn Thr Arg Ile Gln Glu Ala Asp Asn Asp Pro Thr Ala Pro Pro
                725                 730                 735

Tyr Asp Ser Ile Gln Ile Tyr Gly Tyr Glu Gly Arg Gly Ser Val Ala
            740                 745                 750

Gly Ser Leu Ser Ser Leu Glu Ser Ala Thr Thr Asp Ser Asp Leu Asp
        755                 760                 765

Tyr Asp Tyr Leu Gln Asn Trp Gly Pro Arg Phe Lys Lys Leu Ala Asp
    770                 775                 780

Leu Tyr Gly Ser Lys Asp Thr Phe Asp Asp Asp Ser
785                 790                 795

<210> 5

<211> 784

<212> PRT

<213> Homo sapiens

<400> 5

Met Gln Arg Leu Met Met Leu Leu Ala Thr Ser Gly Ala Cys Leu Gly
1               5                   10                  15

Leu Leu Ala Val Ala Ala Val Ala Ala Ala Gly Ala Asn Pro Ala Gln
            20                  25                  30

Arg Asp Thr His Ser Leu Leu Pro Thr His Arg Arg Gln Lys Arg Asp
        35                  40                  45

Trp Ile Trp Asn Gln Met His Ile Asp Glu Glu Lys Asn Thr Ser Leu
    50                  55                  60

Pro His His Val Gly Lys Ile Lys Ser Ser Val Ser Arg Lys Asn Ala
65                  70                  75                  80

Lys Tyr Leu Leu Lys Gly Glu Tyr Val Gly Lys Val Phe Arg Val Asp
                85                  90                  95

Ala Glu Thr Gly Asp Val Phe Ala Ile Glu Arg Leu Asp Arg Glu Asn
            100                 105                 110

Ile Ser Glu Tyr His Leu Thr Ala Val Ile Val Asp Lys Asp Thr Gly
        115                 120                 125

Glu Asn Leu Glu Thr Pro Ser Ser Phe Thr Ile Lys Val His Asp Val
    130                 135                 140

Asn Asp Asn Trp Pro Val Phe Thr His Arg Leu Phe Asn Ala Ser Val
145                 150                 155                 160

Pro Glu Ser Ser Ala Val Gly Thr Ser Val Ile Ser Val Thr Ala Val
                165                 170                 175

Asp Ala Asp Asp Pro Thr Val Gly Asp His Ala Ser Val Met Tyr Gln
            180                 185                 190

Ile Leu Lys Gly Lys Glu Tyr Phe Ala Ile Asp Asn Ser Gly Arg Ile
        195                 200                 205

Ile Thr Ile Thr Lys Ser Leu Asp Arg Glu Lys Gln Ala Arg Tyr Glu

210                 215                 220

Ile Val Val Glu Ala Arg Asp Ala Gln Gly Leu Arg Gly Asp Ser Gly
225                 230                 235                 240

Thr Ala Thr Val Leu Val Thr Leu Gln Asp Ile Asn Asp Asn Phe Pro
                245                 250                 255

Phe Phe Thr Gln Thr Lys Tyr Thr Phe Val Val Pro Glu Asp Thr Arg
            260                 265                 270

Val Gly Thr Ser Val Gly Ser Leu Phe Val Glu Asp Pro Asp Glu Pro
        275                 280                 285

Gln Asn Arg Met Thr Lys Tyr Ser Ile Leu Arg Gly Asp Tyr Gln Asp
    290                 295                 300

Ala Phe Thr Ile Glu Thr Asn Pro Ala His Asn Glu Gly Ile Ile Lys
305                 310                 315                 320

Pro Met Lys Pro Leu Asp Tyr Glu Tyr Ile Gln Gln Tyr Ser Phe Ile
                325                 330                 335

Val Glu Ala Thr Asp Pro Thr Ile Asp Leu Arg Tyr Met Ser Pro Pro
            340                 345                 350

Ala Gly Asn Arg Ala Gln Val Ile Ile Asn Ile Thr Asp Val Asp Glu
        355                 360                 365

Pro Pro Ile Phe Gln Gln Pro Phe Tyr His Phe Gln Leu Lys Glu Asn
    370                 375                 380

Gln Lys Lys Pro Leu Ile Gly Thr Val Leu Ala Met Asp Pro Asp Ala
385                 390                 395                 400

Ala Arg His Ser Ile Gly Tyr Ser Ile Arg Arg Thr Ser Asp Lys Gly
                405                 410                 415

Gln Phe Phe Arg Val Thr Lys Lys Gly Asp Ile Tyr Asn Glu Lys Glu
            420                 425                 430

Leu Asp Arg Glu Val Tyr Pro Trp Tyr Asn Leu Thr Val Glu Ala Lys
        435                 440                 445

Glu Leu Asp Ser Thr Gly Thr Pro Thr Gly Lys Glu Ser Ile Val Gln
    450                 455                 460

Val His Ile Glu Val Leu Asp Glu Asn Asp Asn Ala Pro Glu Phe Ala
465                 470                 475                 480

Lys Pro Tyr Gln Pro Lys Val Cys Glu Asn Ala Val His Gly Gln Leu
                485                 490                 495

Val Leu Gln Ile Ser Ala Ile Asp Lys Asp Ile Thr Pro Arg Asn Val
            500                 505                 510

Lys Phe Lys Phe Thr Leu Asn Thr Glu Asn Asn Phe Thr Leu Thr Asp
        515                 520                 525

Asn His Asp Asn Thr Ala Asn Ile Thr Val Lys Tyr Gly Gln Phe Asp
    530                 535                 540

Arg Glu His Thr Lys Val His Phe Leu Pro Val Val Ile Ser Asp Asn
545                 550                 555                 560

Gly Met Pro Ser Arg Thr Gly Thr Ser Thr Leu Thr Val Ala Val Cys
                565                 570                 575

Lys Cys Asn Glu Gln Gly Glu Phe Thr Phe Cys Glu Asp Met Ala Ala
            580                 585                 590

Gln Val Gly Val Ser Ile Gln Ala Val Val Ala Ile Leu Leu Cys Ile
        595                 600                 605

Leu Thr Ile Thr Val Ile Thr Leu Leu Ile Phe Leu Arg Arg Arg Leu
    610                 615                 620

Arg Lys Gln Ala Arg Ala His Gly Lys Ser Val Pro Glu Ile His Glu
625                 630                 635                 640

Gln Leu Val Thr Tyr Asp Glu Glu Gly Gly Gly Glu Met Asp Thr Thr
                645                 650                 655

Ser Tyr Asp Val Ser Val Leu Asn Ser Val Arg Arg Gly Gly Ala Lys
            660                 665                 670

Pro Pro Arg Pro Ala Leu Asp Ala Arg Pro Ser Leu Tyr Ala Gln Val
        675                 680                 685

Gln Lys Pro Pro Arg His Ala Pro Gly Ala His Gly Gly Pro Gly Glu
    690                 695                 700

Met Ala Ala Met Ile Glu Val Lys Lys Asp Glu Ala Asp His Asp Gly
705                 710                 715                 720

Asp Gly Pro Pro Tyr Asp Thr Leu His Ile Tyr Gly Tyr Glu Gly Ser
                725                 730                 735

Glu Ser Ile Ala Glu Ser Leu Ser Ser Leu Gly Thr Asp Ser Ser Asp
            740                 745                 750

Ser Asp Val Asp Tyr Asp Phe Leu Asn Asp Trp Gly Pro Arg Phe Lys
        755                 760                 765

Met Leu Ala Glu Leu Tyr Gly Ser Asp Pro Arg Glu Glu Leu Leu Tyr
    770                 775                 780

<210>  6

<211>  829

<212>  PRT

<213>  Homo sapiens

<400>  6

<210>  7

<211>  882

<212>  PRT

<213>  Homo sapiens

<400>  7

<210> 8

<211> 900

<212>  PRT

<213>  Homo sapiens

<400>  8

Met Cys Arg Ile Ala Gly Ala Leu Arg Thr Leu Leu Pro Leu Leu Leu
1               5                   10                  15

Ala Leu Leu Gln Ala Ser Val Glu Ala Ser Gly Glu Ile Ala Leu Cys
            20                  25                  30

Lys Thr Gly Phe Pro Glu Asp Val Tyr Ser Ala Val Leu Ser Lys Asp
        35                  40                  45

Val His Glu Gly Gln Pro Leu Leu Asn Val Lys Phe Ser Asn Cys Asn
    50                  55                  60

Gly Lys Arg Lys Val Gln Tyr Glu Ser Ser Glu Pro Ala Asp Phe Lys
65                  70                  75                  80

Val Asp Glu Asp Gly Met Val Tyr Ala Val Arg Ser Phe Pro Leu Ser
                85                  90                  95

Ser Glu His Ala Lys Phe Leu Ile Tyr Ala Gln Asp Lys Glu Thr Gln
            100                 105                 110

Glu Lys Trp Gln Val Ala Val Lys Leu Ser Leu Lys Pro Thr Leu Thr
        115                 120                 125

Glu Glu Ser Val Lys Glu Ser Ala Glu Val Glu Glu Ile Val Phe Pro
    130                 135                 140

Arg Gln Phe Ser Lys His Ser Gly His Leu Gln Arg Gln Lys Arg Asp
145                 150                 155                 160

Trp Val Ile Pro Pro Ile Asn Leu Pro Glu Asn Ser Arg Gly Pro Phe
                165                 170                 175

Pro Gln Glu Leu Val Arg Ile Arg Ser Asp Arg Asp Lys Asn Leu Ser
            180                 185                 190

Leu Arg Tyr Ser Val Thr Gly Pro Gly Ala Asp Gln Pro Pro Thr Gly
        195                 200                 205

Ile Phe Ile Ile Asn Pro Ile Ser Gly Gln Leu Ser Val Thr Lys Pro
    210                 215                 220

Leu Asp Arg Glu Gln Ile Ala Arg Phe His Leu Arg Ala His Ala Val
225                 230                 235                 240

Asp Ile Asn Gly Asn Gln Val Glu Asn Pro Ile Asp Ile Val Ile Asn
                245                 250                 255

Val Ile Asp Met Asn Asp Asn Arg Pro Glu Phe Leu His Gln Val Trp
            260                 265                 270

Asn Gly Thr Val Pro Glu Gly Ser Lys Pro Gly Thr Tyr Val Met Thr
        275                 280                 285

Val Thr Ala Ile Asp Ala Asp Asp Pro Asn Ala Leu Asn Gly Met Leu
    290                 295                 300

Arg Tyr Arg Ile Val Ser Gln Ala Pro Ser Thr Pro Ser Pro Asn Met
305                 310                 315                 320

Phe Thr Ile Asn Asn Glu Thr Gly Asp Ile Ile Thr Val Ala Ala Gly
                325                 330                 335

Leu Asp Arg Glu Lys Val Gln Gln Tyr Thr Leu Ile Ile Gln Ala Thr
            340                 345                 350

Asp Met Glu Gly Asn Pro Thr Tyr Gly Leu Ser Asn Thr Ala Thr Ala
        355                 360                 365

Val Ile Thr Val Thr Asp Val Asn Asp Asn Pro Pro Glu Phe Thr Ala
    370                 375                 380

Met Thr Phe Tyr Gly Glu Val Pro Glu Asn Arg Val Asp Ile Ile Val
385                 390                 395                 400

Ala Asn Leu Thr Val Thr Asp Lys Asp Gln Pro His Thr Pro Ala Trp
                405                 410                 415

Asn Ala Val Tyr Arg Ile Ser Gly Gly Asp Pro Thr Gly Arg Phe Ala
            420                 425                 430

Ile Gln Thr Asp Pro Asn Ser Asn Asp Gly Leu Val Thr Val Val Lys
        435                 440                 445

Pro Ile Asp Phe Glu Thr Asn Arg Met Phe Val Leu Thr Val Ala Ala
    450                 455                 460

Glu Asn Gln Val Pro Leu Ala Lys Gly Ile Gln His Pro Pro Gln Ser
465                 470                 475                 480

Thr Ala Thr Val Ser Val Thr Val Ile Asp Val Asn Glu Asn Pro Tyr
                485                 490                 495

Phe Ala Pro Asn Pro Lys Ile Ile Arg Gln Glu Glu Gly Leu His Ala
            500                 505                 510

Gly Thr Met Leu Thr Thr Phe Thr Ala Gln Asp Pro Asp Arg Tyr Met
        515                 520                 525

Gln Gln Asn Ile Arg Tyr Thr Lys Leu Ser Asp Pro Ala Asn Trp Leu
    530                 535                 540

Lys Ile Asp Pro Val Asn Gly Gln Ile Thr Thr Ile Ala Val Leu Asp
545                 550                 555                 560

Arg Glu Ser Pro Asn Val Lys Asn Asn Ile Tyr Asn Ala Thr Phe Leu
                565                 570                 575

Ala Ser Asp Asn Gly Ile Pro Pro Met Ser Gly Thr Gly Thr Leu Gln
            580                 585                 590

Ile Tyr Leu Leu Asp Ile Asn Asp Asn Ala Pro Gln Val Leu Pro Gln
        595                 600                 605

Glu Ala Glu Thr Cys Glu Thr Pro Asp Pro Asn Ser Ile Asn Ile Thr
    610                 615                 620

Ala Leu Asp Tyr Asp Ile Asp Pro Asn Ala Gly Pro Phe Ala Phe Asp
625                 630                 635                 640

Leu Pro Leu Ser Pro Val Thr Ile Lys Arg Asn Trp Thr Ile Thr Arg
                645                 650                 655

Leu Asn Gly Asp Phe Ala Gln Leu Asn Leu Lys Ile Lys Phe Leu Glu
            660                 665                 670

Ala Gly Ile Tyr Glu Val Pro Ile Ile Ile Thr Asp Ser Gly Asn Pro
        675                 680                 685

Pro Lys Ser Asn Ile Ser Ile Leu Arg Val Lys Val Cys Gln Cys Asp
    690                 695                 700

Ser Asn Gly Asp Cys Thr Asp Val Asp Arg Ile Val Gly Ala Gly Leu
705                 710                 715                 720

Gly Thr Gly Ala Ile Ile Ala Ile Leu Leu Cys Ile Ile Ile Leu Leu
                725                 730                 735

Ile Leu Val Leu Met Phe Val Val Trp Met Lys Arg Arg Asp Lys Glu
            740                 745                 750

Arg Gln Ala Lys Gln Leu Leu Ile Asp Pro Glu Asp Asp Val Arg Asp
        755                 760                 765

Asn Ile Leu Lys Tyr Asp Glu Glu Gly Gly Gly Glu Glu Asp Gln Asp

770                 775                 780

Tyr Asp Leu Ser Gln Leu Gln Gln Pro Asp Thr Val Glu Pro Asp Ala
785                 790                 795                 800

Ile Lys Pro Val Gly Ile Arg Arg Met Asp Glu Arg Pro Ile His Ala
                805                 810                 815

Glu Pro Gln Tyr Pro Val Arg Ser Ala Ala Pro His Pro Gly Asp Ile
            820                 825                 830

Gly Asp Phe Ile Asn Glu Gly Leu Lys Ala Ala Asp Asn Asp Pro Thr
        835                 840                 845

Ala Pro Pro Tyr Asp Ser Leu Leu Val Phe Asp Tyr Glu Gly Ser Gly
    850                 855                 860

Ser Thr Ala Gly Ser Leu Ser Ser Leu Asn Ser Ser Ser Ser Gly Gly
865                 870                 875                 880

Glu Gln Asp Tyr Asp Tyr Leu Asn Asp Trp Gly Pro Arg Phe Lys Lys
                885                 890                 895

Leu Ala Asp Met
            900

<210>  9

<211>  916

<212>  PRT

<213>  Homo sapiens

<400>  9

<210>  10

<211>  814

<212>  PRT

<213>  Homo sapiens

<400>  10

Met Asp Ala Ala Phe Leu Leu Val Leu Gly Leu Leu Ala Gln Ser Leu
1               5                   10                  15

Cys Leu Ser Leu Gly Val Pro Gly Trp Arg Arg Pro Thr Thr Leu Tyr
            20                  25                  30

Pro Trp Arg Arg Ala Pro Ala Leu Ser Arg Val Arg Arg Ala Trp Val
        35                  40                  45

Ile Pro Pro Ile Ser Val Ser Glu Asn His Lys Arg Leu Pro Tyr Pro
    50                  55                  60

Leu Val Gln Ile Lys Ser Asp Lys Gln Gln Leu Gly Ser Val Ile Tyr
65                  70                  75                  80

Ser Ile Gln Gly Pro Gly Val Asp Glu Glu Pro Arg Gly Val Phe Ser
                85                  90                  95

Ile Asp Lys Phe Thr Gly Lys Val Phe Leu Asn Ala Met Leu Asp Arg
            100                 105                 110

Glu Lys Thr Asp Arg Phe Arg Leu Arg Ala Phe Ala Leu Asp Leu Gly
        115                 120                 125

Gly Ser Thr Leu Glu Asp Pro Thr Asp Leu Glu Ile Val Val Val Asp
    130                 135                 140

Gln Asn Asp Asn Arg Pro Ala Phe Leu Gln Glu Ala Phe Thr Gly Arg
145                 150                 155                 160

Val Leu Glu Gly Ala Val Pro Gly Thr Tyr Val Thr Arg Ala Glu Ala
                165                 170                 175

Thr Asp Ala Asp Asp Pro Glu Thr Asp Asn Ala Ala Leu Arg Phe Ser
            180                 185                 190

Ile Leu Gln Gln Gly Ser Pro Glu Leu Phe Ser Ile Asp Glu Leu Thr
        195                 200                 205

Gly Glu Ile Arg Thr Val Gln Val Gly Leu Asp Arg Glu Val Val Ala
    210                 215                 220

Val Tyr Asn Leu Thr Leu Gln Val Ala Asp Met Ser Gly Asp Gly Leu
225                 230                 235                 240

Thr Ala Thr Ala Ser Ala Ile Ile Thr Leu Asp Asp Ile Asn Asp Asn
                245                 250                 255

Ala Pro Glu Phe Thr Arg Asp Glu Phe Phe Met Glu Ala Ile Glu Ala
            260                 265                 270

Val Ser Gly Val Asp Val Gly Arg Leu Glu Val Glu Asp Arg Asp Leu
        275                 280                 285

Pro Gly Ser Pro Asn Trp Val Ala Arg Phe Thr Ile Leu Glu Gly Asp
    290                 295                 300

Pro Asp Gly Gln Phe Thr Ile Arg Thr Asp Pro Lys Thr Asn Glu Gly
305                 310                 315                 320

Val Leu Ser Ile Val Lys Ala Leu Asp Tyr Glu Ser Cys Glu His Tyr
                325                 330                 335

Glu Leu Lys Val Ser Val Gln Asn Glu Ala Pro Leu Gln Ala Ala Ala
            340                 345                 350

Leu Arg Ala Glu Arg Gly Gln Ala Lys Val Arg Val His Val Gln Asp
        355                 360                 365

Thr Asn Glu Pro Pro Val Phe Gln Glu Asn Pro Leu Arg Thr Ser Leu
    370                 375                 380

Ala Glu Gly Ala Pro Pro Gly Thr Leu Val Ala Thr Phe Ser Ala Arg
385                 390                 395                 400

Asp Pro Asp Thr Glu Gln Leu Gln Arg Leu Ser Tyr Ser Lys Asp Tyr
                405                 410                 415

Asp Pro Glu Asp Trp Leu Gln Val Asp Ala Ala Thr Gly Arg Ile Gln
            420                 425                 430

Thr Gln His Val Leu Ser Pro Ala Ser Pro Phe Leu Lys Gly Gly Trp
        435                 440                 445

Tyr Arg Ala Ile Val Leu Ala Gln Asp Asp Ala Ser Gln Pro Arg Thr
    450                 455                 460

Ala Thr Gly Thr Leu Ser Ile Glu Ile Leu Glu Val Asn Asp His Ala
465                 470                 475                 480

Pro Val Leu Ala Pro Pro Pro Pro Gly Ser Leu Cys Ser Glu Pro His
                485                 490                 495

Gln Gly Pro Gly Leu Leu Leu Gly Ala Thr Asp Glu Asp Leu Pro Pro
            500                 505                 510

His Gly Ala Pro Phe His Phe Gln Leu Ser Pro Arg Leu Pro Glu Leu
        515                 520                 525

Gly Arg Asn Trp Ser Leu Ser Gln Val Asn Val Ser His Ala Arg Leu
    530                 535                 540

Arg Pro Arg His Gln Val Pro Glu Gly Leu His Arg Leu Ser Leu Leu
545                 550                 555                 560

Leu Arg Asp Ser Gly Gln Pro Pro Gln Gln Arg Glu Gln Pro Leu Asn
                565                 570                 575

Val Thr Val Cys Arg Cys Gly Lys Asp Gly Val Cys Leu Pro Gly Ala
            580                 585                 590

Ala Ala Leu Leu Ala Gly Gly Thr Gly Leu Ser Leu Gly Ala Leu Val
        595                 600                 605

Ile Val Leu Ala Ser Ala Leu Leu Leu Leu Val Leu Val Leu Leu Val
    610                 615                 620

Ala Leu Arg Ala Arg Phe Trp Lys Gln Ser Arg Gly Lys Gly Leu Leu
625                 630                 635                 640

His Gly Pro Gln Asp Asp Leu Arg Asp Asn Val Leu Asn Tyr Asp Glu
                645                 650                 655

Gln Gly Gly Gly Glu Glu Asp Gln Asp Ala Tyr Asp Ile Ser Gln Leu
            660                 665                 670

Arg His Pro Thr Ala Leu Ser Leu Pro Leu Gly Pro Pro Pro Leu Arg
        675                 680                 685

Arg Asp Ala Pro Gln Gly Arg Leu His Pro Gln Pro Pro Arg Val Leu
    690                 695                 700

Pro Thr Ser Pro Leu Asp Ile Ala Asp Phe Ile Asn Asp Gly Leu Glu
705                 710                 715                 720

Ala Ala Asp Ser Asp Pro Ser Val Pro Pro Tyr Asp Thr Ala Leu Ile
                725                 730                 735

Tyr Asp Tyr Glu Gly Asp Gly Ser Val Ala Gly Thr Leu Ser Ser Ile
            740                 745                 750

Leu Ser Ser Gln Gly Asp Glu Asp Gln Asp Tyr Asp Tyr Leu Arg Asp
        755                 760                 765

Trp Gly Pro Arg Phe Ala Arg Leu Ala Asp Met Tyr Gly His Pro Cys
    770                 775                 780

Gly Leu Glu Tyr Gly Ala Arg Trp Asp His Gln Ala Arg Glu Gly Leu
785                 790                 795                 800

Ser Pro Gly Ala Leu Leu Pro Arg His Arg Gly Arg Thr Ala
                805                 810

<210> 11

<211> 3205

<212> DNA

<213> Homo sapiens

<400> 11
aaaggggcaa gagctgagcg gaacaccggc ccgccgtcgc ggcagctgct tcacccctct 60

ctctgcagcc atggggctcc ctcgtggacc tctcgcgtct ctcctccttc tccaggtttg 120

ctggctgcag tgcgcggcct ccgagccgtg ccgggcggtc ttcagggagg ctgaagtgac 180

cttggaggcg ggaggcgcgg agcaggagcc cggccaggcg ctggggaaag tattcatggg 240

ctgccctggg caagagccag ctctgtttag cactgataat gatgacttca ctgtgcggaa 300

tggcgagaca gtccaggaaa gaaggtcact gaaggaaagg aatccattga agatcttccc 360

atccaaacgt atcttacgaa gacacaagag agattgggtg gttgctccaa tatctgtccc 420

tgaaaatggc aagggtccct tcccccagag actgaatcag ctcaagtcta ataaagatag 480

agacaccaag atttctacag catcacgggg gccgggggca gacagccccc ctgagggtgt 540

cttcgctgta gagaaggaga caggctggtt gttgttgaat aagccactgg accgggagga 600

gattgccaag tatgagctct ttggccacgc tgtgtcagag aatggtgcct cagtggagga 660

ccccatgaac atctccatca tcgtgaccga ccagaatgac cacaagccca agtttaccca 720

ggacaccttc cgagggagtg tcttagaggg agtcctacca ggtacttctg tgatgcaggt 780

gacagccacg gatgaggatg atgccatcta cacctacaat ggggtggttg cttactccat 840

ccatagccaa gaaccaaagg acccacacga cctcatgttc accattcacc ggagcacagg 900

caccatcagc gtcatctcca gtggcctgga ccgggaaaaa gtccctgagt acacactgac 960
catccaggcc acagacatgg atggggacgg ctccaccacc acggcagtgg cagtagtgga 1020
gatccttgat gccaatgaca atgctcccat gtttgacccc cagaagtacg aggcccatgt 1080
gcctgagaat gcagtgggcc atgaggtgca gaggctgacg gtcactgatc tggacgcccc 1140
caactcacca gcgtggcgtg ccacctacct tatcatgggc ggtgacgacg gggaccattt 1200
taccatcacc acccaccctg agagcaacca gggcatcctg acaaccagga agggtttgga 1260
ttttgaggcc aaaaaccagc acaccctgta cgttgaagtg accaacgagg ccccttttgt 1320
gctgaagctc ccaacctcca cagccaccat agtggtccac gtggaggatg tgaatgaggc 1380
acctgtgttt gtcccaccct ccaaagtcgt tgaggtccag gagggcatcc ccactgggga 1440
gcctgtgtgt gtctacactg cagaagaccc tgacaaggag aatcaaaaga tcagctaccg 1500
catcctgaga gacccagcag ggtggctagc catggaccca gacagtgggc aggtcacagc 1560
tgtgggcacc ctcgaccgtg aggatgagca gtttgtgagg aacaacatct atgaagtcat 1620
ggtcttggcc atggacaatg gaagccctcc caccactggc acgggaaccc ttctgctaac 1680
actgattgat gtcaatgacc atggcccagt ccctgagccc cgtcagatca ccatctgcaa 1740
ccaaagccct gtgcgccagg tgctgaacat cacggacaag gacctgtctc cccacacctc 1800
ccctttccag gcccagctca cagatgactc agacatctac tggacggcag aggtcaacga 1860
ggaaggtgac acagtggtct tgtccctgaa gaagttcctg aagcaggata catatgacgt 1920
gcacctttct ctgtctgacc atggcaacaa agagcagctg acggtgatca gggccactgt 1980
gtgcgactgc catggccatg tcgaaacctg ccctggaccc tggaagggag gtttcatcct 2040
ccctgtgctg ggggctgtcc tggctctgct gttcctcctg ctggtgctgc ttttgttggt 2100
gagaaagaag cggaagatca aggagcccct cctactccca gaagatgaca cccgtgacaa 2160
cgtcttctac tatggcgaag aggggggtgg cgaagaggac caggactatg acatcaccca 2220
gctccaccga ggtctggagg ccaggccgga ggtggttctc cgcaatgacg tggcaccaac 2280
catcatcccg acacccatgt accgtcctcg gccagccaac ccagatgaaa tcggcaactt 2340
tataattgag aacctgaagg cggctaacac agaccccaca gccccgccct acgacaccct 2400
cttggtgttc gactatgagg gcagcggctc cgacgccgcg tccctgagct ccctcacctc 2460
ctccgcctcc gaccaagacc aagattacga ttatctgaac gagtggggca gccgcttcaa 2520
gaagctggca gacatgtacg gtggcgggga ggacgactag gcggcctgcc tgcagggctg 2580
gggaccaaac gtcaggccac agagcatctc caaggggtct cagttccccc ttcagctgag 2640
gacttcggag cttgtcagga agtggccgta gcaacttggc ggagacaggc tatgagtctg 2700
acgttagagt ggttgcttcc ttagcctttc aggatggagg aatgtgggca gtttgacttc 2760
agcactgaaa acctctccac ctgggccagg gttgcctcag aggccaagtt tccagaagcc 2820
tcttacctgc cgtaaaatgc tcaaccctgt gtcctgggcc tgggcctgct gtgactgacc 2880
tacagtggac tttctctctg gaatggaacc ttcttaggcc tcctggtgca acttaatttt 2940

tttttttaat gctatcttca aaacgttaga gaaagttctt caaaagtgca gcccagagct 3000

gctgggccca ctggccgtcc tgcatttctg gtttccagac cccaatgcct cccattcgga 3060

tggatctctg cgtttttata ctgagtgtgc ctaggttgcc ccttattttt tattttccct 3120

gttgcgttgc tatagatgaa gggtgaggac aatcgtgtat atgtactaga acttttttat 3180

taaagaaact tttcccagaa aaaaa 3205

<210> 12

<211> 4758

<212> DNA

<213> Homo sapiens

<400> 12
ggcccgaccc gaccgcaccc ggcgcctgcc ctcgctcggc gtccccggcc agccatgggc 60

ccttggagcc gcagcctctc ggcgctgctg ctgctgctgc aggtctcctc ttggctctgc 120

caggagccgg agccctgcca ccctggcttt gacgccgaga gctacacgtt cacggtgccc 180

cggcgccacc tggagagagg ccgcgtcctg ggcagagtga attttgaaga ttgcaccggt 240

cgacaaagga cagcctattt ttccctcgac acccgattca aagtgggcac agatggtgtg 300

attacagtca aaaggcctct acggtttcat aacccacaga tccatttctt ggtctacgcc 360

tgggactcca cctacagaaa gttttccacc aaagtcacgc tgaatacagt ggggcaccac 420

caccgccccc cgccccatca ggcctccgtt tctggaatcc aagcagaatt gctcacattt 480

cccaactcct ctcctggcct cagaagacag aagagagact gggttattcc tcccatcagc 540

tgcccagaaa atgaaaaagg cccatttcct aaaaacctgg ttcagatcaa atccaacaaa 600

gacaaagaag gcaaggtttt ctacagcatc actggccaag gagctgacac accccctgtt 660

ggtgtcttta ttattgaaag agaaacagga tggctgaagg tgacagagcc tctggataga 720

gaacgcattg ccacatacac tctcttctct cacgctgtgt catccaacgg gaatgcagtt 780

gaggatccaa tggagatttt gatcacggta accgatcaga atgacaacaa gcccgaattc 840

acccaggagg tctttaaggg gtctgtcatg gaaggtgctc ttccaggaac ctctgtgatg 900

gaggtcacag ccacagacgc ggacgatgat gtgaacacct acaatgccgc catcgcttac 960

accatcctca gccaagatcc tgagctccct gacaaaaata tgttcaccat taacaggaac 1020

acaggagtca tcagtgtggt caccactggg ctggaccgag agagtttccc tacgtatacc 1080

ctggtggttc aagctgctga ccttcaaggt gaggggttaa gcacaacagc aacagctgtg 1140

atcacagtca ctgacaccaa cgataatcct ccgatcttca atcccaccac gtacaagggt 1200

caggtgcctg agaacgaggc taacgtcgta atcaccacac tgaaagtgac tgatgctgat 1260

gcccccaata ccccagcgtg ggaggctgta tacaccatat tgaatgatga tggtggacaa 1320

tttgtcgtca ccacaaatcc agtgaacaac gatggcattt tgaaaacagc aaagggcttg 1380
gattttgagg ccaagcagca gtacattcta cacgtagcag tgacgaatgt ggtacctttt 1440
gaggtctctc tcaccacctc cacagccacc gtcaccgtgg atgtgctgga tgtgaatgaa 1500
gcccccatct ttgtgcctcc tgaaaagaga gtggaagtgt ccgaggactt tggcgtgggc 1560
caggaaatca catcctacac tgcccaggag ccagacacat ttatggaaca gaaaataaca 1620
tatcggattt ggagagacac tgccaactgg ctggagatta atccggacac tggtgccatt 1680
tccactcggg ctgagctgga cagggaggat tttgagcacg tgaagaacag cacgtacaca 1740
gccctaatca tagctacaga caatggttct ccagttgcta ctggaacagg gacacttctg 1800
ctgatcctgt ctgatgtgaa tgacaacgcc cccataccag aacctcgaac tatattcttc 1860
tgtgagagga atccaaagcc tcaggtcata aacatcattg atgcagacct tcctcccaat 1920
acatctccct tcacagcaga actaacacac ggggcgagtg ccaactggac cattcagtac 1980
aacgacccaa cccaagaatc tatcattttg aagccaaaga tggccttaga ggtgggtgac 2040
tacaaaatca atctcaagct catggataac cagaataaag accaagtgac caccttagag 2100
gtcagcgtgt gtgactgtga aggggccgcc ggcgtctgta ggaaggcaca gcctgtcgaa 2160
gcaggattgc aaattcctgc cattctgggg attcttggag gaattcttgc tttgctaatt 2220
ctgattctgc tgctcttgct gtttcttcgg aggagagcgg tggtcaaaga gcccttactg 2280
cccccagagg atgacacccg ggacaacgtt tattactatg atgaagaagg aggcggagaa 2340
gaggaccagg actttgactt gagccagctg cacaggggcc tggacgctcg gcctgaagtg 2400
actcgtaacg acgttgcacc aaccctcatg agtgtccccc ggtatcttcc ccgccctgcc 2460
aatcccgatg aaattggaaa ttttattgat gaaaatctga aagcggctga tactgacccc 2520
acagccccgc cttatgattc tctgctcgtg tttgactatg aaggaagcgg ttccgaagct 2580
gctagtctga gctccctgaa ctcctcagag tcagacaaag accaggacta tgactacttg 2640
aacgaatggg gcaatcgctt caagaagctg gctgacatgt acggaggcgg cgaggacgac 2700
taggggactc gagagaggcg ggccccagac ccatgtgctg ggaaatgcag aaatcacgtt 2760
gctggtggtt tttcagctcc cttcccttga gatgagtttc tggggaaaaa aaagagactg 2820
gttagtgatg cagttagtat agctttatac tctctccact ttatagctct aataagtttg 2880
tgttagaaaa gtttcgactt atttcttaaa gctttttttt ttttcccatc actctttaca 2940
tggtggtgat gtccaaaaga tacccaaatt ttaatattcc agaagaacaa ctttagcatc 3000
agaaggttca cccagcacct tgcagatttt cttaaggaat tttgtctcac ttttaaaaag 3060
aaggggagaa gtcagctact ctagttctgt tgttttgtgt atataatttt ttaaaaaaaa 3120
tttgtgtgct tctgctcatt actacactgg tgtgtccctc tgcctttttt ttttttttta 3180
agacagggtc tcattctatc ggccaggctg gagtgcagtg gtgcaatcac agctcactgc 3240
agccttgtcc tcccaggctc aagctatcct tgcacctcag cctcccaagt agctgggacc 3300
acaggcatgc accactacgc atgactaatt ttttaaatat ttgagacggg gtctccctgt 3360

gttacccagg ctggtctcaa actcctgggc tcaagtgatc ctcccatctt ggcctcccag 3420
agtattggga ttacagacat gagccactgc acctgcccag ctccccaact ccctgccatt 3480
ttttaagaga cagtttcgct ccatcgccca ggcctgggat gcagtgatgt gatcatagct 3540
cactgtaacc tcaaactctg gggctcaagc agttctccca ccagcctcct ttttattttt 3600
ttgtacagat ggggtcttgc tatgttgccc aagctggtct taaactcctg gcctcaagca 3660
atccttctgc cttggccccc caaagtgctg ggattgtggg catgagctgc tgtgcccagc 3720
ctccatgttt taatatcaac tctcactcct gaattcagtt gctttgccca agataggagt 3780
tctctgatgc agaaattatt gggctctttt agggtaagaa gtttgtgtct ttgtctggcc 3840
acatcttgac taggtattgt ctactctgaa gacctttaat ggcttccctc tttcatctcc 3900
tgagtatgta acttgcaatg ggcagctatc cagtgacttg ttctgagtaa gtgtgttcat 3960
taatgtttat ttagctctga agcaagagtg atatactcca ggacttagaa tagtgcctaa 4020
agtgctgcag ccaaagacag agcggaacta tgaaaagtgg gcttggagat ggcaggagag 4080
cttgtcattg agcctggcaa tttagcaaac tgatgctgag gatgattgag gtgggtctac 4140
ctcatctctg aaaattctgg aaggaatgga ggagtctcaa catgtgtttc tgacacaaga 4200
tccgtggttt gtactcaaag cccagaatcc ccaagtgcct gcttttgatg atgtctacag 4260
aaaatgctgg ctgagctgaa cacatttgcc caattccagg tgtgcacaga aaaccgagaa 4320
tattcaaaat tccaaatttt ttcttaggag caagaagaaa atgtggccct aaagggggtt 4380
agttgagggg tagggggtag tgaggatctt gatttggatc tctttttatt taaatgtgaa 4440
tttcaacttt tgacaatcaa agaaaagact tttgttgaaa tagctttact gtttctcaag 4500
tgttttggag aaaaaaatca accctgcaat cactttttgg aattgtcttg atttttcggc 4560
agttcaagct atatcgaata tagttctgtg tagagaatgt cactgtagtt ttgagtgtat 4620
acatgtgtgg gtgctgataa ttgtgtattt tctttggggg tggaaaagga aaacaattca 4680
agctgagaaa agtattctca aagatgcatt tttataaatt ttattaaaca attttgttaa 4740
accataaaaa aaaaaaaa 4758

<210> 13

<211> 4122

<212> DNA

<213> Homo sapiens


<400> 13
tttgtcatca gctcgctctc cattggcggg gagcggagag cagcgaagaa gggggtgggg 60
aggggagggg aagggaaggg ggtggaaact gcctggagcc gtttctccgc gccgctgttg 120
gtgctgccgc tgcctcctcc tcctccgccg ccgccgccgc cgccgccgcc tcctccggct 180

cttcgctcgg cccctctccg cctccatgtg ccggatagcg ggagcgctgc ggaccctgct 240
gccgctgctg gcggccctgc ttcaggcgtc tgtagaggct tctggtgaaa tcgcattatg 300
caagactgga tttcctgaag atgtttacag tgcagtctta tcgaaggatg tgcatgaagg 360
acagcctctt ctcaatgtga agtttagcaa ctgcaatgga aaaagaaaag tacaatatga 420
gagcagtgag cctgcagatt ttaaggtgga tgaagatggc atggtgtatg ccgtgagaag 480
ctttccactc tcttctgagc atgccaagtt cctgatatat gcccaagaca aagagaccca 540
ggaaaagtgg caagtggcag taaaattgag cctgaagcca accttaactg aggagtcagt 600
gaaggagtca gcagaagttg aagaaatagt gttcccaaga caattcagta agcacagtgg 660
ccacctacaa aggcagaaga gagactgggt catccctcca atcaacttgc cagaaaactc 720
caggggacct tttcctcaag agcttgtcag gatcaggtct gatagagata aaaacctttc 780
actgcggtac agtgtaactg ggccaggagc tgaccagcct ccaactggta tcttcattat 840
caaccccatc tcgggtcagc tgtcggtgac aaagcccctg gatcgcgagc agatagcccg 900
gtttcatttg agggcacatg cagtagatat taatggaaat caagtggaga accccattga 960
cattgtcatc aatgttattg acatgaatga caacagacct gagttcttac accaggtttg 1020
gaatgggaca gttcctgagg gatcaaagcc tggaacatat gtgatgaccg taacagcaat 1080
tgatgctgac gatcccaatg ccctcaatgg gatgttgagg tacagaatcg tgtctcaggc 1140
tccaagcacc ccttcaccca acatgtttac aatcaacaat gagactggtg acatcatcac 1200
agtggcagct ggacttgatc gagaaaaagt gcaacagtat acgttaataa ttcaagctac 1260
agacatggaa ggcaatccca catatggcct ttcaaacaca gccacggccg tcatcacagt 1320
gacagatgtc aatgacaatc ctccagagtt tactgccatg acgttttatg gtgaagttcc 1380
tgagaacagg gtagacatca tagtagctaa tctaactgtg accgataagg atcaacccca 1440
tacaccagcc tggaacgcag tgtacagaat cagtggcgga gatcctactg gacggttcgc 1500
catccagacc gacccaaaca gcaacgacgg gttagtcacc gtggtcaaac caatcgactt 1560
tgaaacaaat aggatgtttg tccttactgt tgctgcagaa aatcaagtgc cattagccaa 1620
gggaattcag cacccgcctc agtcaactgc aaccgtgtct gttacagtta ttgacgtaaa 1680
tgaaaaccct tattttgccc ccaatcctaa gatcattcgc caagaagaag ggcttcatgc 1740
cggtaccatg ttgacaacat tcactgctca ggacccagat cgatatatgc agcaaaatat 1800
tagatacact aaattatctg atcctgccaa ttggctaaaa atagatcctg tgaatggaca 1860
aataactaca attgctgttt tggaccgaga atcaccaaat gtgaaaaaca atatatataa 1920
tgctactttc cttgcttctg acaatggaat tcctcctatg agtggaacag gaacgctgca 1980
gatctattta cttgatatta atgacaatgc ccctcaagtg ttacctcaag aggcagagac 2040
ttgcgaaact ccagacccca attcaattaa tattacagca cttgattatg acattgatcc 2100
aaatgctgga ccatttgctt ttgatcttcc tttatctcca gtgactatta agagaaattg 2160
gaccatcact cggcttaatg gtgattttgc tcagcttaat ttaaagataa aatttcttga 2220

agctggtatc tatgaagttc ccatcataat cacagattcg ggtaatcctc ccaaatcaaa 2280

tatttccatc ctgcgcgtga aggtttgcca gtgtgactcc aacggggact gcacagatgt 2340

ggacaggatt gtgggtgcgg ggcttggcac cggtgccatc attgccatcc tgctctgcat 2400

catcatcctg cttatccttg tgctgatgtt tgtggtatgg atgaaacgcc gggataaaga 2460

acgccaggcc aaacaacttt taattgatcc agaagatgat gtaagagata atattttaaa 2520

atatgatgaa gaaggtggag gagaagaaga ccaggactat gacttgagcc agctgcagca 2580

gcctgacact gtggagcctg atgccatcaa gcctgtggga atccgacgaa tggatgaaag 2640

acccatccac gctgagcccc agtatccggt ccgatctgca gccccacacc ctggagacat 2700

tggggacttc attaatgagg gccttaaagc ggctgacaat gaccccacag ctccaccata 2760

tgactccctg ttagtgtttg actatgaagg cagtggctcc actgctgggt ccttgagctc 2820

ccttaattcc tcaagtagtg gtggtgagca ggactatgat tacctgaacg actgggggcc 2880

acggttcaag aaacttgctg acatgtatgg tggaggtgat gactgaactt cagggtgaac 2940

ttggtttttg gacaagtaca aacaatttca actgatattc ccaaaaagca ttcagaagct 3000

aggctttaac tttgtagtct actagcacag tgcttgctgg aggctttggc ataggctgca 3060

aaccaatttg ggctcagagg gaatatcagt gatccatact gtttggaaaa acactgagct 3120

cagttacact tgaattttac agtacagaag cactgggatt ttatgtgcct tttgtacctt 3180

ttttcagatt ggaattagtt ttctgtttaa ggctttaatg gtactgattt ctgaaacgat 3240

aagtaaaaga caaaatattt tgtggtggga gcagtaagtt aaaccatgat atgcttcaac 3300

acgcttttgt tacattgcat ttgcttttat taaaatacaa aattaaacaa acaaaaaaac 3360

tcatggagcg attttattat cttgggggat gagaccatga gattggaaaa tgtacattac 3420

ttctagtttt agactttagt ttgttttttt tttttcacta aaatcttaaa acttactcag 3480

ctggttgcaa ataaagggag ttttcatatc accaatttgt agcaaaattg aattttttca 3540

taaactagaa tgttagacac attttggtct taatccatgt acactttttt atttctgtat 3600

ttttccactt cactgtaaaa atagtatgtg tacataatgt tttattggca tagtctatgg 3660

agaagtgcag aaacttcaga acatgtgtat gtattatttg gactatggat tcaggttttt 3720

tgcatgttta tatctttcgt tatggataaa gtatttacaa aacagtgaca tttgattcaa 3780

ttgttgagct gtagttagaa tactcaattt ttaatttttt taattttttt attttttatt 3840

ttctttttgg tttggggagg gagaaaagtt cttagcacaa atgttttaca taatttgtac 3900

caaaaaaaaa aaaaaggaaa ggaaagaaag gggtggcctg acactggtgg cactactaag 3960

tgtgtgtttt ttaaaaaaaa aaatggaaaa aaaaaagctt ttaaactgga gagacttctg 4020

acaacagctt tgcctctgta ttgtgtacca gaatataaat gatacacctc tgaccccagc 4080

gttctgaata aaatgctaat tttggaaaaa aaaaaaaaaa aa 4122

<210> 14

<211>  3063

<212>  DNA

<213>  Homo sapiens

<400>  14
cgccggcggg gaagatgacc gcgggcgccg gcgtgctcct tctgctgctc tcgctctccg      60

gcgcgctccg ggcccataat gaggatctta caactagaga gacctgcaag gctgggttct     120

ctgaagatga ttacacggca ttaatctccc aaaatattct agaaggggaa aagctacttc     180

aagtcaagtt cagcagctgt gtggggacca aggggacaca atatgagacc aacagcatgg     240

acttcaaagt tggggcagat gggacagtct tcgccacccg ggagctgcag gtcccctccg     300

agcaggtggc gttcacggtg actgcatggg acagccagac agcagagaaa tgggacgccg     360

tggtgcggtt gctggtggcc cagacctcgt ccccgcactc tggacacaag ccgcagaaag     420

gaaagaaggt cgtggctctg gacccctctc cgcctccgaa ggacaccctg ctgccgtggc     480

cccagcacca gaacgccaac gggctgaggc ggcgcaaacg ggactgggtc atcccgccca     540

tcaacgtgcc cgagaactcg cgcgggccct tcccgcagca gctcgtgagg atccggtccg     600

acaaagacaa tgacatcccc atccggtaca gcatcacggg agtgggcgcc gaccagcccc     660

ccatggaggt cttcagcatt gactccatgt ccggccggat gtacgtcaca aggcccatgg     720

accgggagga gcacgcctct taccacctcc gagcccacgc tgtggacatg aatggcaaca     780

aggtggagaa ccccatcgac ctgtacatct acgtcatcga catgaatgac aaccgccctg     840

agttcatcaa ccaggtctac aacggctccg tggacgaggg ctccaagcca ggcacctacg     900

tgatgaccgt cacggccaac gatgctgacg acagcaccac ggccaacggg atggtgcggt     960

accggatcgt gacccagacc ccacagagcc cgtcccagaa tatgttcacc atcaacagcg    1020

agactggaga tatcgtcaca gtggcggctg gcctggaccg agagaaagtt cagcagtaca    1080

cagtcatcgt tcaggccaca gatatggaag gaaatctcaa ctatggcctc tcaaacacag    1140

ccacagccat catcacggtg acagatgtga atgacaaccc gccagaattt accgccagca    1200

cgtttgcagg ggaggtcccc gaaaaccgcg tggagaccgt ggtcgcaaac ctcacggtga    1260

tggaccgaga tcagccccac tctccaaact ggaatgccgt ttaccgcatc atcagtgggg    1320

atccatccgg gcacttcagc gtccgcacag accccgtaac caacgagggc atggtcaccg    1380

tggtgaaggc agtcgactac gagctcaaca gagctttcat gctgacagtg atggtgtcca    1440

accaggcgcc cctggccagc ggaatccaga tgtccttcca gtccacggca ggggtgacca    1500

tctccatcat ggacatcaac gaggctccct acttcccctc aaaccacaag ctgatccgcc    1560

tggaggaggg cgtgcccccc ggcaccgtgc tgaccacgtt ttcagctgtg gaccctgacc    1620

ggttcatgca gcaggctgtg agatactcaa agctgtcaga cccagcgagc tggctgcaca    1680

tcaatgccac caacggccag atcaccacgg cggcagtgct ggaccgtgag tccctctaca    1740

ccaaaaacaa cgtctacgag gccaccttcc tggcagctga caatgggata cccccggcca 1800

gcggcaccgg gaccctccag atctatctca ttgacatcaa cgacaacgcc cctgagctgc 1860

tgcccaagga ggcgcagatc tgcgagaagc ccaacctgaa cgccatcaac atcacggcgg 1920

ccgacgctga cgtcgacccc aacatcggcc cctacgtctt cgagctgccc tttgtcccgg 1980

cggccgtgcg gaagaactgg accatcaccc gcctgaacgg tgactatgcc caactcagct 2040

tgcgcatcct gtacctggag gccgggatgt atgacgtccc catcatcgtc acagactctg 2100

gaaaccctcc cctgtccaac acgtccatca tcaaagtcaa ggtgtgccca tgtgatgaca 2160

acggggactg caccaccatt ggcgcagtgg cagcggctgg tctgggcacc ggtgccatcg 2220

tggccatcct catctgcatc ctcatcctgc tgaccatggt cctgctgttt gtcatgtgga 2280

tgaagcggcg agagaaggag cgccacacga agcagctgct cattgacccc gaggacgacg 2340

tccgcgacaa catcctcaag tatgacgagg aaggcggtgg cgaggaggac caggactacg 2400

acctcagcca gctgcagcag ccggaagcca tggggcacgt gccaagcaaa gcccctggcg 2460

tgcgtcgcgt ggatgagcgg ccggtgggcg ctgagcccca gtacccgatc aggcccatgg 2520

tgccgcaccc aggcgacatc ggtgacttca tcaatgaggg actccgcgct gctgacaacg 2580

accccacggc acccccctat gactccctgc tggtcttcga ctacgagggg agcggctcca 2640

ccgcaggctc cgtcagctcc ctgaactcat ccagttccgg ggaccaagac tacgattacc 2700

tcaacgactg ggggcccaga ttcaagaagc tggcggacat gtatggaggt ggtgaagagg 2760

attgactgac ctcgcatctt cggaccgaag tgagagccgt gctcggacgc cggaggagca 2820

ggactgagca gaggcggccg gtcttcccga ctccctgcgg ctgtgtcctt agtgctgtta 2880

ggaggccccc caatccccac gttgagctgt ctagcatgag cacccacccc cacagcgccc 2940

tgcacccggc cgctgcccag caccgcgctg gctggcactg aaggacagca agaggcactc 3000

tgtcttcact tgaatttcct agaacagaag cactgttttt aaaaaaaaaa aaaaaaaaag 3060

aag 3063

<210> 15

<211> 2833

<212> DNA

<213> Homo sapiens

<400> 15
acttgcgctg tcactcagcc tggacgcgct tcttcgggtc gcgggtgcac tccggcccgg 60

ctcccgcctc ggccccgatg gacgccgcgt tcctcctcgt cctcgggctg ttggcccaga 120

gcctctgcct gtctttgggg gttcctggat ggaggaggcc caccaccctg tacccctggc 180

gccgggcgcc tgccctgagc cgcgtgcgga gggcctgggt catccccccg atcagcgtat 240

ccgagaacca caagcgtctc ccctaccccc tggttcagat caagtcggac aagcagcagc 300
tgggcagcgt catctacagc atccagggac ccggcgtgga tgaggagccc cggggcgtct 360
tctctatcga caagttcaca gggaaggtct tcctcaatgc catgctggac cgcgagaaga 420
ctgatcgctt caggctaaga gcgtttgccc tggacctggg aggatccacc ctggaggacc 480
ccacggacct ggagattgta gttgtggatc agaatgacaa ccggccagcc ttcctgcagg 540
aggcgttcac tggccgcgtg ctggagggtg cagtcccagg cacctatgtg accagggcag 600
aggccacaga tgccgacgac cccgagacgg acaacgcagc gctgcggttc tccatcctgc 660
agcagggcag ccccgagctc ttcagcatcg acgagctcac aggagagatc cgcacagtgc 720
aagtggggct ggaccgcgag gtggtcgcgg tgtacaatct gaccctgcag gtggcggaca 780
tgtctggaga cggcctcaca gccactgcct cagccatcat cacccttgat gacatcaatg 840
acaatgcccc cgagttcacc agggatgagt tcttcatgga ggccatagag gccgtcagcg 900
gagtggatgt gggacgcctg gaagtggagg acagggacct gccaggctcc ccaaactggg 960
tggccaggtt caccatcctg gaaggcgacc ccgatgggca gttcaccatc cgcacggacc 1020
ccaagaccaa cgagggtgtt ctgtccattg tgaaggccct ggactatgag agctgtgaac 1080
actacgaact caaagtgtcg gtgcagaatg aggccccgct gcaggcggct gcccttaggg 1140
ctgagcgggg ccaggccaag gtccgcgtgc atgtgcagga caccaacgag ccccccgtgt 1200
tccaggagaa cccacttcgg accagcctag cagagggggc acccccaggc actctggtgg 1260
ccaccttctc tgcccgggac cctgacacag agcagctgca gaggctcagc tactccaagg 1320
actacgaccc ggaagactgg ctgcaagtgg acgcagccac tggccggatc cagacccagc 1380
acgtgctcag cccggcgtcc cccttcctca agggcggctg gtacagagcc atcgtcctgg 1440
cccaggatga cgcctcccag ccccgcaccg ccaccggcac cctgtccatc gagatcctgg 1500
aggtgaacga ccatgcacct gtgctggccc cgccgccgcc gggcagcctg tgcagcgagc 1560
cacaccaagg cccaggcctc ctcctgggcg ccacggatga ggacctgccc ccccacgggg 1620
cccccttcca cttccagctg agccccaggc tcccagagct cggccggaac tggagcctca 1680
gccaggtcaa cgtgagccac gcgcgcctgc ggccgcgaca ccaggtcccc gaaggcctgc 1740
accgcctcag cctgctgctc cgggactcgg ggcagccgcc ccagcagcgc gagcagcctc 1800
tgaacgtgac cgtgtgccgc tgcggcaagg acggcgtctg cctgccgggg gccgcagcgc 1860
tgctggcggg gggcacaggc ctcagcctgg gcgcactggt catcgtgctg gccagcgccc 1920
tcctgctgct ggtgctggtc ctgctcgtgg cactccgggc gcggttctgg aagcagtctc 1980
ggggcaaggg gctgctgcac ggcccccagg acgaccttcg agacaatgtc ctcaactacg 2040
atgagcaagg aggcggggag gaggaccagg acgcctacga catcagccag ctgcgtcacc 2100
cgacagcgct gagcctgcct ctgggaccgc cgccacttcg cagagatgcc ccgcagggcc 2160
gcctgcaccc ccagccaccc cgagtgctgc ccaccagccc cctggacatc gccgacttca 2220
tcaatgatgg cttggaggct gcagatagtg accccagtgt gccgccttac gacacagccc 2280

tcatctatga ctacgagggt gacggctcgg tggcggggac gctgagctcc atcctgtcca 2340

gccagggcga tgaggaccag gactacgact acctcagaga ctgggggccc cgcttcgccc 2400

ggctggcaga catgtatggg cacccgtgcg ggttggagta cggggccaga tgggaccacc 2460

aggccaggga gggtctttct cctggggcac tgctacccag acacagaggc cggacagcct 2520

gaccctgggg cgcaactgga catgccactc cccggcctcg tggcagtgat ggcccctgca 2580

gaggcagcct gaggtcaccg ggcccgaccc ccctgggcct ggggcagcct ccttcctgta 2640

ggcgagggcc caagtctggg ggcagaacct gagtgtggat ggggcggcca ggaagaggcc 2700

ccttcctgcc ggggtgggaa gagtttctct ccatcggccc catgcgggtc acctccctag 2760

tcccaccttt gcctcctacc agtgaacctc atctttgtat gaaagacagc aacctcctgg 2820

gtaaatctga atg 2833

<210> 16

<211> 4521

<212> DNA

<213> Homo sapiens

<400> 16
acttcattca cttgcaaatc agtgtgtgcc cacaagagcc agctctcccg agcccgtaac 60

cttcgcatcc caagagctgc agtttcagcc gcgacagcaa gaacggcaga gccggcgacc 120

gcggcggcgg cggcggcgga ggcaggagca gcctgggcgg gtcgcagggt ctccgcgggc 180

gcaggaaggc gagcagagat atcctctgag agccaagcaa agaacattaa ggaaggaagg 240

aggaatgagg ctggatacgg tgcagtgaaa aaggcacttc caagagtggg gcactcacta 300

cgcacagact cgacggtgcc atcagcatga gaacttaccg ctacttcttg ctgctctttt 360

gggtgggcca gccctaccca actctctcaa ctccactatc aaagaggact agtggtttcc 420

cagcaaagaa aagggccctg gagctctctg gaaacagcaa aaatgagctg aaccgttcaa 480

aaaggagctg gatgtggaat cagttctttc tcctggagga atacacagga tccgattatc 540

agtatgtggg caagttacat tcagaccagg atagaggaga tggatcactt aaatatatcc 600

tttcaggaga tggagcagga gatctcttca ttattaatga aaacacaggc gacatacagg 660

ccaccaagag gctggacagg gaagaaaaac ccgtttacat ccttcgagct caagctataa 720

acagaaggac agggagaccc gtggagcccg agtctgaatt catcatcaag atccatgaca 780

tcaatgacaa tgaaccaata ttcaccaagg aggtttacac agccactgtc cctgaaatgt 840

ctgatgtcgg tacatttgtt gtccaagtca ctgcgacgga tgcagatgat ccaacatatg 900

ggaacagtgc taaagttgtc tacagtattc tacagggaca gccctatttt tcagttgaat 960

cagaaacagg tattatcaag acagctttgc tcaacatgga tcgagaaaac agggagcagt 1020

accaagtggt gattcaagcc aaggatatgg gcggccagat gggaggatta tctgggacca 1080
ccaccgtgaa catcacactg actgatgtca acgacaaccc tccccgattc ccccagagta 1140
cataccagtt taaaactcct gaatcttctc caccggggac accaattggc agaatcaaag 1200
ccagcgacgc tgatgtggga gaaaatgctg aaattgagta cagcatcaca gacggtgagg 1260
ggctggatat gtttgatgtc atcaccgacc aggaaaccca ggaagggatt ataactgtca 1320
aaaagctctt ggactttgaa aagaagaaag tgtataccct taaagtggaa gcctccaatc 1380
cttatgttga gccacgattt ctctacttgg ggcctttcaa agattcagcc acggttagaa 1440
ttgtggtgga ggatgtagat gagccacctg tcttcagcaa actggcctac atcttacaaa 1500
taagagaaga tgctcagata aacaccacaa taggctccgt cacagcccaa gatccagatg 1560
ctgccaggaa tcctgtcaag tactctgtag atcgacacac agatatggac agaatattca 1620
acattgattc tggaaatggt tcgattttta catcgaaact tcttgaccga gaaacactgc 1680
tatggcacaa cattacagtg atagcaacag agatcaataa tccaaagcaa agtagtcgag 1740
tacctctata tattaaagtt ctagatgtca atgacaacgc cccagaattt gctgagttct 1800
atgaaacttt tgtctgtgaa aaagcaaagg cagatcagtt gattcagacc ctgcatgctg 1860
ttgacaagga tgacccttat agtggacacc aattttcgtt ttccttggcc cctgaagcag 1920
ccagtggctc aaactttacc attcaagaca acaaagacaa cacggcggga atcttaactc 1980
ggaaaaatgg ctataataga cacgagatga gcacctatct cttgcctgtg gtcatttcag 2040
acaacgacta cccagttcaa agcagcactg ggacagtgac tgtccgggtc tgtgcatgtg 2100
accaccacgg gaacatgcaa tcctgccatg cggaggcgct catccacccc acgggactga 2160
gcacgggggc tctggttgcc atccttctgt gcatcgtgat cctactagtg acagtggtgc 2220
tgtttgcagc tctgaggcgg cagcgaaaaa aagagccttt gatcatttcc aaagaggaca 2280
tcagagataa cattgtcagt tacaacgacg aaggtggtgg agaggaggac acccaggctt 2340
ttgatatcgg caccctgagg aatcctgaag ccatagagga caacaaatta cgaaggacat 2400
ttgtgcccga agcccttttc ctaccccgac ggactccaac agctcgcgac aacaccgatg 2460
tcagagattt cattaaccaa aggttaaagg aaaatgacac ggaccccact gccccgccat 2520

acgactcctt ggccacttac gcctatgaag gcactggctc cgtggcggat tccctgagct 2580
cgctggagtc agtgaccacg gatgcagatc aagactatga ttaccttagt gactggggac 2640
ctcgattcaa aaagcttgca gatatgtatg gaggagtgga cagtgacaaa gactcctaat 2700
ctgttgcctt tttcattttc caatacgaca ctgaaatatg tgaagtggct atttctttat 2760
atttatccac tactccgtga aggcttctct gttctacccg ttccaaaagc caatggctgc 2820
agtccgtgtg gatccaatgt tagagacttt tttctagtac acttttatga gcttccaagg 2880
ggcaaatttt tattttttag tgcatccagt taaccaagtc agcccaacag gcaggtgccg 2940
gaggggagga cagggaacag tatttccact tgttctcagg gcagcgtgcc cgcttccgct 3000

gtcctggtgt tttactacac tccatgtcag gtcagccaac tgccctaact gtacatttca 3060
caggctaatg ggataaagga ctgtgcttta aagataaaaa tatcatcata gtaaaagaaa 3120
tgagggcata tcggctcaca aagagataaa ctacataggg gtgtttattt gtgtcacaaa 3180
gaatttaaaa taacacttgc ccatgctatt tgttcttcaa gaactttctc tgccatcaac 3240
tactattcaa aacctcaaat ccacccatat gttaaaattc tcattactct taaggaatag 3300
aagcaaatta aacggtaaca tccaaaagca accacaaacc tagtacgact tcattccttc 3360
cactaactca tagtttgtta tatcctagac tagacatgcg aaagtttgcc tttgtaccat 3420
ataaaggggg agggaaatag ctaataatgt taaccaagga aatatatttt accatacatt 3480
taaagttttg gccaccacat gtatcacggg tcacttgaaa ttctttcagc tatcagtagg 3540
ctaatgtcaa aattgtttaa aaattcttga aagaattttc ctgagacaaa ttttaacttc 3600
ttgtctatag ttgtcagtat tattctacta tactgtacat gaaagtagca gtgtgaagta 3660
caataattca tattcttcat atccttctta cacgactaag ttgaattagt aaagttagat 3720
taaataaaac ttaaatctca ctctaggagt tcagtggaga ggttagagcc agccacactt 3780
gaacctaata ccctgccctt gacatctgga aacctctaca tatttatata acgtgataca 3840
tttggataaa caacattgag attatgatga aaacctacat attccatgtt tggaagaccc 3900
ttggaagagg aaaattggat tcccttaaac aaaagtgttt aagattgtaa ttaaaatgat 3960
agttgatttt caaaagcatt aatttttttt cattgttttt aactttgctt tcatgaccat 4020
cctgccatcc ttgactttga actaatgata aagtaatgat ctcaaactat gacagaaaag 4080
taatgtaaaa tccatccaat ctattatttc tctaattatg caattagcct catagttatt 4140
atccagagga cccaactgaa ctgaactaat ccttctggca gattcaaatc gtttatttca 4200
cacgctgttc taatggcact tatcattaga atcttacctt gtgcagtcat cagaaattcc 4260
agcgtactat aatgaaaaca tccttgtttt gaaaacctaa aagacaggct ctgtatatat 4320
atatacttaa gaatatgctg acttcactta ttagtcttag ggatttattt tcaattaata 4380
ttaattttct acaaataatt ttagtgtcat ttccatttgg ggatattgtc atatcagcac 4440
atattttctg tttggaaaca cactgttgtt tagttaagtt ttaaataggt gtattaccca 4500
agaagtaaag atggaaacgt t 4521

<210> 17

<211> 2520

<212> DNA

<213> Homo sapiens

<400> 17
cggtggaggc cacagacacc tcaaacctgg attccacaat tctacgttaa gtgttggagt 60
ttttattact ctgctgtagg aaagcctttg ccaatgctta caaggaactg tttatccctg 120

cttctctggg ttctgtttga tggaggtctc ctaacaccac tacaaccaca gccacagcag 180
actttagcca cagagccaag agaaaatgtt atccatctgc caggacaacg gtcacatttc 240
caacgtgtta aacgtggctg ggtatggaat caattttttg tgctggaaga atacgtgggc 300
tccgagcctc agtatgtggg aaagctccat tccgacttag acaagggaga gggcactgtg 360
aaatacaccc tctcaggaga tggcgctggc accgttttta ccattgatga aaccacaggg 420
gacattcatg caataaggag cctagataga gaagagaaac ctttctacac tcttcgtgct 480
caggctgtgg acatagaaac cagaaagccc ctggagcctg aatcagaatt catcatcaaa 540
gtgcaggata ttaatgataa tgagccaaag tttttggatg gaccttatgt tgctactgtt 600
ccagaaatgt ctcctgtggg tgcatatgta ctccaggtca aggccacaga tgcagatgac 660
ccgacctatg gaaacagtgc cagagtcgtt tacagcattc ttcagggaca accttatttc 720
tctattgatc ccaagacagg tgttattaga acagctttgc caaacatgga cagagaagtc 780
aaagaacaat atcaagtact catccaagcc aaggatatgg gaggacagct tggaggatta 840
gccggaacaa caatagtcaa catcactctc accgatgtca atgacaatcc acctcgattc 900
cccaaaagca tcttccactt gaaagttcct gagtcttccc ctattggttc agctattgga 960
agaataagag ctgtggatcc tgattttgga caaaatgcag aaattgaata caatattgtt 1020
ccaggagatg gggaaatttt gtttgacatc gtcacagatg aggatacaca agagggagtc 1080
atcaaattga aaaagccttt agattttgaa acaaagaagg catacacttt caaagttgag 1140
gcttccaacc ttcaccttga ccaccggttt cactcggcgg gccctttcaa agacacagct 1200
acggtgaaga tcagcgtgct ggacgtagat gagccaccgg ttttcagcaa gccgctctac 1260
accatggagg tttatgaaga cactccggta gggaccatca ttggcgctgt cactgctcaa 1320
gacctggatg taggcagcgg tgctgttagg tacttcatag attggaagag tgatggggac 1380
agctacttta caatagatgg aaatgaagga accatcgcca ctaatgaatt actagacaga 1440
gaaagcactg cgcagtataa tttctccata attgcgagta aagttagtaa ccctttattg 1500
accagcaaag tcaatatact gattaatgtc ttagatgtaa atgaatttcc tccagaaata 1560
tctgtgccat atgagacagc cgtgtgtgaa aatgccaagc caggacagat aattcagata 1620
gtcagtgctg cagaccgaga tctttcacct gctgggcaac aattctcctt tagattatca 1680
cctgaggctg ctatcaaacc aaattttaca gttcgtgact tcagaaacaa cacagcgggg 1740
attgaaaccc gaagaaatgg atacagccgc aggcagcaag agttgtattt cctccctgtt 1800
gtaatagaag acagcagcta ccctgtccag agcagcacaa acacaatgac tattcgagtc 1860
tgtagatgtg actctgatgg caccatcctg tcttgtaatg tggaagcaat ttttctacct 1920
gtaggactta gcactggggc gttgattgca attctactat gcattgttat actcttagcc 1980
atagttgtac tgtatgtagc actgcgaagg cagaagaaaa agcacaccct gatgacctct 2040
aaagaagaca tcagagacaa cgtcatccat tacgatgatg aaggaggtgg ggaggaagat 2100

acccaggctt tcgacatcgg ggctctgaga aacccaaaag tgattgagga gaacaaaatt 2160

cgcagggata taaaaccaga ctctctctgt ttacctcgtc agagaccacc catggaagat 2220

aacacagaca taagggattt cattcatcaa aggctacagg aaaatgatgt agatccaact 2280

gccccaccaa tcgattcact ggccacatat gcctacgaag ggagtgggtc cgtggcagag 2340

tccctcagct ctatagactc tctcaccaca gaagccgacc aggactatga ctatctgaca 2400

gactggggac cccgctttaa agtcttggca gacatgtttg gcgaagaaga gagttataac 2460

cctgataaag tcacttaagg gagtcgtgga ggctaaaata caaccgagag gggagatttt 2520

<210> 18

<211> 2545

<212> DNA

<213> Homo sapiens

<400> 18
   caggaaatgc tcttggatct ctggactcca ttaataatat tatggattac tcttccccct 60
   tgcatttaca tggctccgat gaatcagtct caagttttaa tgagtggatc ccctttggaa 120
   ctaaacagtc tgggtgaaga acagcgaatt ttgaaccgct ccaaaagagg ctgggtttgg 180
   aatcaaatgt ttgtcctgga agagttttct ggacctgaac cgattcttgt tggccggcta 240
   cacacagacc tggatcctgg gagcaaaaaa atcaagtata tcctatcagg tgatggagct 300
   gggaccatat ttcaaataaa tgatgtaact ggagatatcc atgctataaa aagacttgac 360
   cgggaggaaa aggctgagta taccctaaca gctcaagcag tggactggga gacaagcaaa 420
   cctctggagc ctccttctga atttattatt aaagttcaag acatcaatga caatgcacca 480
   gagtttctta atggacccta tcatgctact gtgccagaaa tgtccatttt gggtacatct 540
   gtcactaacg tcactgcgac cgacgctgat gacccagttt atggaaacag tgcaaagttg 600
   gtttatagta tattggaagg gcagccttat ttttccattg agcctgaaac agctattata 660
   aaaactgccc ttcccaacat ggacagagaa gccaaggagg agtacctggt tgttatccaa 720
   gccaaagata tgggtggaca ctctggtggc ctgtctggga ccacgacact tacagtgact 780
   cttactgatg ttaatgacaa tcctccaaaa tttgcacaga gcctgtatca cttctcagta 840
   ccggaagatg tggttcttgg cactgcaata ggaagggtga aggccaatga tcaggatatt 900
   ggtgaaaatg cacagtcatc atatgatatc atcgatggag atggaacagc actttttgaa 960
   atcacttctg atgcccaggc ccaggatggc attataaggc taagaaaacc tctggacttt 1020
   gagaccaaaa aatcctatac gctaaaggat gaggcagcca atgtccatat tgacccacgc 1080
   ttcagtggca gggggccctt taaagacacg gcgacagtca aaatcgtggt tgaagatgct 1140
   gatgagcctc cggtcttctc ttcaccgact tacctacttg aagttcatga aaatgctgct 1200
   ctaaactccg tgattgggca agtgactgct cgtgaccctg atatcacttc cagtcctata 1260
   aggttttcca tcgaccggca cactgacctg gagaggcagt tcaacattaa tgcagacgat 1320
   gggaagataa cgctggcaac accacttgac agagaattaa gtgtatggca caacataaca 1380
   atcattgcta ctgaaattag gaaccacagt cagatatcac gagtacctgt tgctattaaa 1440
   gtgctggatg tcaatgacaa cgcccctgaa ttcgcatccg aatatgaggc atttttatgt 1500
   gaaaatggaa aacccggcca agtcattcaa actgttagcg ccatggacaa agatgatccc 1560
   aaaaacggac attatttctt atacagtctc cttccagaaa tggtcaacaa tccgaatttc 1620
   accatcaaga aaaatgaaga taattccctc agtattttgg caaagcataa tggattcaac 1680
   cgccagaagc aagaagtcta tcttttacca atcataatca gtgatagtgg aaatcctcca 1740
   ctgagcagca ctagcacctt gacaatcagg gtctgtggct gcagcaatga cggtgtcgtc 1800
   cagtcttgca atgtcgaagc ttatgtcctt ccaattggac tcagtatggg cgccttaatt 1860
   gccatattag catgcatcat tttgctgtta gtcatcgtgg tgctgtttgt aactctacgg 1920
   cggcatcaaa aaaatgaacc attaattatc aaagatgatg aagacgttcg agaaaacatc 1980
   attcgctacg atgatgaagg aggaggggag gaggacacag aggcttttga cattgcaact 2040
   ttacaaaatc cagatggaat taatggattt ttaccccgta aggatattaa accagatttg 2100
   cagtttatgc caaggcaagg gcttgctcca gttccaaatg gtgttgatgt cgatgaattt 2160
   ataaatgtaa ggctgcatga ggcagataat gatcccacag ccccgccata tgactccatt 2220
   caaatatatg gctatgaagg ccgagggtca gtggctggct ccctcagctc cttggagtcc 2280
   accacatcag actcagacca gaattttgac tacctcagtg actggggtcc ccgctttaag 2340
   agactgggcg aactctactc tgttggtgaa agtgacaaag aaacttgaca gtggattata 2400
   aataaatcac tggaactgag cattctgtaa tattctaggg tcactcccct tagatacaac 2460
   caatgtggct atttgtttag aggcaagttt agcaccagtc atctataact caaccacatt 2520
   taatgttgac aaaaagataa taaat 2545

<210> 19

<211> 2625

<212> DNA

<213> Homo sapiens

<400> 19
cggcagccct gacgtgatga gctcaaccag cagagacatt ccatcccaag agaggtctgc 60

gtgacgcgtc cgggaggcca ccctcagcaa gaccaccgta cagttggtgg aaggggtgac 120

agctgcattc tcctgtgcct accacgtaac caaaaatgaa ggagaactac tgtttacaag 180

ccgccctggt gtgcctgggc atgctgtgcc acagccatgc ctttgcccca gagcggcggg 240

ggcacctgcg gccctccttc catgggcacc atgagaaggg caaggagggg caggtgctac 300

agcgctccaa gcgtggctgg gtctggaacc agttcttcgt gatagaggag tacaccgggc 360
ctgaccccgt gcttgtgggc aggcttcatt cagatattga ctctggtgat gggaacatta 420
aatacattct ctcaggggaa ggagctggaa ccatttttgt gattgatgac aaatcaggga 480
acattcatgc caccaagacg ttggatcgag aagagagagc ccagtacacg ttgatggctc 540
aggcggtgga cagggacacc aatcggccac tggagccacc gtcggaattc attgtcaagg 600
tccaggacat taatgacaac cctccggagt tcctgcacga gacctatcat gccaacgtgc 660
ctgagaggtc caatgtggga acgtcagtaa tccaggtgac agcttcagat gcagatgacc 720
ccacttatgg aaatagcgcc aagttagtgt acagtatcct cgaaggacaa ccctattttt 780
cggtggaagc acagacaggt atcatcagaa cagccctacc caacatggac agggaggcca 840
aggaggagta ccacgtggtg atccaggcca aggacatggg tggacatatg ggcggactct 900
cagggacaac caaagtgacg atcacactga ccgatgtcaa tgacaaccca ccaaagtttc 960
cgcagaggct ataccagatg tctgtgtcag aagcagccgt ccctggggag gaagtaggaa 1020
gagtgaaagc taaagatcca gacattggag aaaatggctt agtcacatac aatattgttg 1080
atggagatgg tatggaatcg tttgaaatca caacggacta tgaaacacag gagggggtga 1140
taaagctgaa aaagcctgta gattttgaaa ccgaaagagc ctatagcttg aaggtagagg 1200
cagccaacgt gcacatcgac ccgaagttta tcagcaatgg ccctttcaag gacactgtga 1260
ccgtcaagat ctcagtagaa gatgctgatg agccccctat gttcttggcc ccaagttaca 1320
tccacgaagt ccaagaaaat gcagctgctg gcaccgtggt tgggagagtg catgccaaag 1380
accctgatgc tgccaacagc ccgataaggt attccatcga tcgtcacact gacctcgaca 1440
gatttttcac tattaatcca gaggatggtt ttattaaaac tacaaaacct ctggatagag 1500
aggaaacagc ctggctcaac atcactgtct ttgcagcaga aatccacaat cggcatcagg 1560
aagcccaagt cccagtggcc attagggtcc ttgatgtcaa cgataatgct cccaagtttg 1620
ctgcccctta tgaaggtttc atctgtgaga gtgatcagac caagccactt tccaaccagc 1680
caattgttac aattagtgca gatgacaagg atgacacggc caatggacca agatttatct 1740
tcagcctacc ccctgaaatc attcacaatc caaatttcac agtcagagac aaccgagata 1800
acacagcagg cgtgtacgcc cggcgtggag ggttcagtcg gcagaagcag gacttgtacc 1860
ttctgcccat agtgatcagc gatggcggca tcccgcccat gagtagcacc aacaccctca 1920
ccatcaaagt ctgcgggtgc gacgtgaacg gggcactgct ctcctgcaac gcagaggcct 1980
acattctgaa cgccggcctg agcacaggcg ccctgatcgc catcctcgcc tgcatcgtca 2040
ttctcctggt cattgtagta ttgtttgtga ccctgagaag gcaaaagaaa gaaccactca 2100
ttgtctttga ggaagaagat gtccgtgaga acatcattac ttatgatgat gaagggggtg 2160
gggaagaaga cacagaagcc tttgatattg ccaccctcca gaatcctgat ggtatcaatg 2220
gatttatccc ccgcaaagac atcaaacctg agtatcagta catgcctaga cctgggctcc 2280
ggccagcgcc caacagcgtg gatgtcgatg acttcatcaa cacgagaata caggaggcag 2340

acaatgaccc cacggctcct ccttatgact ccattcaaat ctacggttat gaaggcaggg 2400

gctcagtggc cgggtccctg agctccctag agtcggccac cacagattca gacttggact 2460

atgattatct acagaactgg ggacctcgtt ttaagaaact agcagatttg tatggttcca 2520

aagacacttt tgatgacgat tcttaacaat aacgatacaa atttggcctt aagaactgtg 2580

tctggcgttc tcaagaatct agaagatgtg taacaggtat ttttt 2625

<210> 20

<211> 4098

<212> DNA

<213> Homo sapiens

<400> 20
gacggtcggc tgacaggctc cacagagctc cactcacgct caggccctgg acggacaggc 60

agtccaacgg aacagaaaca tccctcagcc ccacaggcac gatctgttcc tcctgggaag 120

atgcagaggc tcatgatgct cctcgccaca tcgggcgcct gcctgggcct gctggcagtg 180

gcagcagtgg cagcagcagg tgctaaccct gcccaacggg acacccacag cctgctgccc 240

acccaccggc gccaaaagag agattggatt tggaaccaga tgcacattga tgaagagaaa 300

aacacctcac ttccccatca tgtaggcaag atcaagtcaa gcgtgagtcg caagaatgcc 360

aagtacctgc tcaaaggaga atatgtgggc aaggtcttcc gggtcgatgc agagacagga 420

gacgtgttcg ccattgagag gctggaccgg gagaatatct cagagtacca cctcactgct 480

gtcattgtgg acaaggacac tggtgaaaac ctggagactc cttccagctt caccatcaaa 540

gttcatgacg tgaacgacaa ctggcctgtg ttcacgcatc ggttgttcaa tgcgtccgtg 600

cctgagtcgt cggctgtggg gacctcagtc atctctgtga cagcagtgga tgcagacgac 660

cccactgtgg gagaccacgc ctctgtcatg taccaaatcc tgaaggggaa agagtatttt 720

gccatcgata attctggacg tattatcaca ataacgaaaa gcttggaccg agagaagcag 780

gccaggtatg agatcgtggt ggaagcgcga gatgcccagg gcctccgggg ggactcgggc 840

acggccaccg tgctggtcac tctgcaagac atcaatgaca acttcccctt cttcacccag 900

accaagtaca catttgtcgt gcctgaagac acccgtgtgg gcacctctgt gggctctctg 960

tttgttgagg acccagatga gccccagaac cggatgacca agtacagcat cttgcggggc 1020

gactaccagg acgctttcac cattgagaca aaccccgccc acaacgaggg catcatcaag 1080

cccatgaagc ctctggatta tgaatacatc cagcaataca gcttcatcgt cgaggccaca 1140

gaccccacca tcgacctccg atacatgagc cctcccgcgg gaaacagagc ccaggtcatt 1200

atcaacatca cagatgtgga cgagcccccc attttccagc agcctttcta ccacttccag 1260

ctgaaggaaa accagaagaa gcctctgatt ggcacagtgc tggccatgga ccctgatgcg 1320

gctaggcata gcattggata ctccatccgc aggaccagtg acaagggcca gttcttccga 1380
gtcacaaaaa agggggacat ttacaatgag aaagaactgg acagagaagt ctacccctgg 1440
tataacctga ctgtggaggc caaagaactg gattccactg gaacccccac aggaaaagaa 1500
tccattgtgc aagtccacat tgaagttttg gatgagaatg acaatgcccc ggagtttgcc 1560
aagccctacc agcccaaagt gtgtgagaac gctgtccatg gccagctggt cctgcagatc 1620
tccgcaatag acaaggacat aacaccacga aacgtgaagt tcaaattcac cttgaatact 1680
gagaacaact ttaccctcac ggataatcac gataacacgg ccaacatcac agtcaagtat 1740
gggcagtttg accgggagca taccaaggtc cacttcctac ccgtggtcat ctcagacaat 1800
gggatgccaa gtcgcacggg caccagcacg ctgaccgtgg ccgtgtgcaa gtgcaacgag 1860
cagggcgagt tcaccttctg cgaggatatg gccgcccagg tgggcgtgag catccaggca 1920
gtggtagcca tcttactctg catcctcacc atcacagtga tcaccctgct catcttcctg 1980
cggcggcggc tccggaagca ggcccgcgcg cacggcaaga gcgtgccgga gatccacgag 2040
cagctggtca cctacgacga ggagggcggc ggcgagatgg acaccaccag ctacgatgtg 2100
tcggtgctca actcggtgcg ccgcggcggg gccaagcccc cgcggcccgc gctggacgcc 2160
cggccttccc tctatgcgca ggtgcagaag ccaccgaggc acgcgcctgg ggcacacgga 2220
gggcccgggg agatggcagc catgatcgag gtgaagaagg acgaggcgga ccacgacggc 2280
gacggccccc cctacgacac gctgcacatc tacggctacg agggctccga gtccatagcc 2340
gagtccctca gctccctggg caccgactca tccgactctg acgtggatta cgacttcctt 2400
aacgactggg gacccaggtt taagatgctg gctgagctgt acggctcgga cccccgggag 2460
gagctgctgt attaggcggc cgaggtcact ctgggcctgg ggacccaaac cccctgcagc 2520
ccaggccagt cagacgccag gcaccacagc ctccaaaaat ggcagtgact ccccagccca 2580
gcaccccttc ctcgtgggtc ccagagacct catcagcctt gggatagcaa actccaggtt 2640
cctgaaatat ccaggaatat atgtcagtga tgactattct caaatgctgg caaatccagg 2700
ctggtgttct gtctgggctc agacatccac ataaccctgt cacccacaga ccgccgtcta 2760
actcaaagac ttcctctggc tccccaaggc tgcaaagcaa aacagactgt gtttaactgc 2820
tgcagggtct tttctagggg tccctgaacg ccctggtaag gctggtgagg tcctggtgcc 2880
tatctgcctg gaggcaaagg cctggacagc ttgacttgtg ggcaggattc ctctgcagcc 2940
cattcccaag ggagactgac catcatgccc tctctcggga gccctagccc tgctccaact 3000
ccatactcca ctccaagtgc cccaccactc cccaacccct ctccaggcct gtcaagaggg 3060
aggaaggggc cccatggcag ctcctgacct tgggtcctga agtgacctca ctggcctgcc 3120
atgccagtaa ctgtgctgta ctgagcactg aaccacattc agggaaatgg cttattaaac 3180
tttgaagcaa ctgtgaattc attctggagg ggcagtggag atcaggagtg acagatcaca 3240
gggtgagggc cacctccaca cccaccccct ctggagaagg cctggaagag ctgagacctt 3300
gctttgagac tcctcagcac ccctccagtt ttgcctgaga aggggcagat gttcccggag 3360

cagaagacgt ctccccttct ctgcctcacc tggtcgccaa tccatgctct ctttcttttc 3420

tctgtctact ccttatccct tggtttagag gaacccaaga tgtggccttt agcaaaactg 3480

gacaatgtcc aaacccactc atgactgcat gacggagccg agccatgtgt ctttacacct 3540

cgctgttgtc acatctcagg gaactgaccc tcaggcacac cttgcagaag gcaaggccct 3600

gccctgccca acctctgtgg tcacccatgc atcttccact ggaacgtttc actgcaaaca 3660

caccttggag aagtggcatc agtcaacaga gaggggcagg gaaggagaca ccaagctcac 3720

ccttcgtcat ggaccgaggt tcccactctg ggcaaagccc ctcacactgc aagggattgt 3780

agataacact gacttgtttg ttttaaccaa taactagctt cttataatga tttttttact 3840

aatgatactt acaagtttct agctctcaca gacatataga ataagggttt ttgcataata 3900

agcaggttgt tatttaggtt aacaatatta attcaggttt tttagttgga aaaacaattc 3960

ctgtaacctt ctattttcta taattgtagt aattgctcta cagataatgt ctatatattg 4020

gccaaactgg tgcatgacaa gtactgtatt tttttatacc taaataaaga aaaatcttta 4080

gcctgggcaa caaaaaaa 4098

<210> 21

<211> 21

<212> DNA

<213> Artificial sequence

<220>

<223> Synthetic oligonucleotide

<400> 21
gagaggtcca cgagggagcc c 21

<210> 22

<211> 20

<212> DNA

<213> Artificial sequence

<220>

<223> Synthetic oligonucleotide

<400> 22
cacggctcgg aggccgcgca 20

<210> 23

<211> 20

<212>  DNA

<213>  Artificial sequence

<220>

<223>  Synthetic oligonucleotide

<400>  23
cgcctccaag gtcacttcag                                                    20

<210>  24

<211>  20

<212>  DNA

<213>  Artificial sequence

<220>

<223>  Synthetic oligonucleotide

<400>  24
cgcctccaag gtcacttcag                                                    20

<210>  25

<211>  20

<212>  DNA

<213>  Artificial sequence

<220>

<223>  Synthetic oligonucleotide

<400>  25
agtgaccttc tttcctggac                                                    20

<210>  26

<211>  20

<212>  DNA

<213>  Artificial sequence

<220>

<223>  Synthetic oligonucleotide

<400>  26
gtttggatgg gaagatcttc                                                    20

<210>  27

<211>  20

<212>  DNA

<213>  Artificial sequence

<220>

<223>  Synthetic oligonucleotide

<400>  27
cttgtgtctt cgtaagatac                                                    20

<210>  28

<211>  20

<212>  DNA

<213>  Artificial sequence

<220>

<223>  Synthetic oligonucleotide

<400>  28
ctgggggaag ggacccttgc                                                    20

<210>  29

<211>  20

<212>  DNA

<213>  Artificial sequence

<220>

<223>  Synthetic oligonucleotide

<400>  29
cttcagcaca aaaggggcct                                                    20

<210>  30

<211>  22

<212>  DNA

<213>  Artificial sequence

<220>

<223>  Synthetic oligonucleotide

<400>  30
caacgacttt ggagggtggg ac                                              22

<210>  31

<211>  21

<212>  DNA

<213>  Artificial sequence

<220>

<223>  Synthetic oligonucleotide

<400>  31
gttgttcctc acaaactgct c                                               21

<210>  32

<211>  21

<212>  DNA

<213>  Artificial sequence

<220>

<223>  Synthetic oligonucleotide

<400>  32
gtggtgggag ggcttccatt g                                               21

<210>  33

<211>  21

<212>  DNA

<213>  Artificial sequence

<220>

<223> . Synthetic oligonucleotide

<400>  33
gatctgacgg ggctcaggga c                                               21

<210>  34

<211>  20

<212>  DNA

<213>  Artificial sequence

<220>

<223>  Synthetic oligonucleotide

<400>  34
catctgtgag ctgggcctgg                                                    20

<210>  35

<211>  21

<212>  DNA

<213>  Artificial sequence

<220>

<223>  Synthetic oligonucleotide

<400>  35
ccttcctcgt tgacctctgc c                                                  21

<210>  36

<211>  22

<212>  DNA

<213>  Artificial sequence

<220>

<223>  Synthetic oligonucleotide

<400>  36
ctttgttgcc atggtcagac ag                                                 22

<210>  37

<211>  20

<212>  DNA

<213>  Artificial sequence

<220>

<223>  Synthetic oligonucleotide

<400>  37
gcagcaccag caggaggaac                                                    20

<210>  38

<211>  21

<212>  DNA

<213>  Artificial sequence

<220>

<223>  Synthetic oligonucleotide

<400>  38
ggttggtgcc acgtcattgc g                                                21

<210>  39

<211>  22

<212>  DNA

<213>  Artificial sequence

<220>

<223>  Synthetic oligonucleotide

<400>  39
gttggctggc cgaggacggt ac                                               22

<210>  40

<211>  10

<212>  PRT

<213>  Artificial sequence

<220>

<223>  Synthetic peptide

<400>  40

Val Pro Glu Asn Gly Lys Gly Pro Phe Pro
1               5                   10

<210>  41

<211>  19

<212>  PRT

<213>  Artificial sequence

<220>

<223>  Synthetic peptide

<400>  41

Gln Glu Pro Lys Asp Pro His Asp Leu Met Phe Thr Ile His Arg Ser
1               5                   10                  15

Thr Gly Thr

<210>  42

<211>  10

<212>  PRT

<213>  Artificial sequence

<220>

<223>  Synthetic peptide

<400>  42

Asp Asn Gly Ser Pro Pro Thr Thr Gly Thr
1               5                   10

<210>  43

<211>  23

<212>  PRT

<213>  Artificial sequence

<220>

<223>  Synthetic peptide

<400>  43

Thr Asp Lys Asp Leu Ser Pro His Thr Ser Pro Phe Gln Ala Gln Leu
1               5                   10                  15

Thr Asp Asp Ser Asp Ile Tyr
            20

<210>  44

<211>  16

<212>  PRT

<213>  Artificial sequence

<220>

<223>  Synthetic peptide

<400>  44

Asp Cys His Gly His Val Glu Thr Cys Pro Gly Pro Trp Lys Gly Gly
1               5                   10                  15


<210>  45

<211>  12

<212>  PRT

<213>  Artificial sequence



<220>

<223>  Synthetic peptide

<400>  45

Met Tyr Arg Pro Arg Pro Ala Asn Pro Asp Glu Ile
1               5                   10


<210>  46

<211>  19

<212>  DNA

<213>  Artificial sequence



<220>

<223>  Synthetic oligonucleotide

<400>  46
cttggagatg ctctgtggc                                                    19

<210>  47

<211>  20

<212>  DNA

<213>  Artificial sequence



<220>

<223>  Synthetic oligonucleotide

<400>  47
gcacttgctg tctgctggtc                                                   20

<210>  48

<211>  20

<212>  DNA

<213>  Artificial sequence

<220>

<223>  Synthetic oligonucleotide

<400>  48
catgcttgtt ctcctgtgtg                                                    20

<210>  49

<211>  20

<212>  DNA

<213>  Artificial sequence

<220>

<223>  Synthetic oligonucleotide

<400>  49
ctgtgacatc atctgtcttg                                                    20

<210>  50

<211>  20

<212>  DNA

<213>  Artificial sequence

<220>

<223>  Synthetic oligonucleotide

<400>  50
caaagagact acagcaatgg ac                                                 22

<210>  51

<211>  20

<212>  DNA

<213>  Artificial sequence

<220>

<223>  Synthetic oligonucleotide

<400>  51
ctgagtgagg acatctgcag                                                    20

<210>  52

<211>  19

<212>  DNA

<213>  Artificial sequence

<220>

<223>  Synthetic oligonucleotide

<400>  52
ctgggtgaca gagtgagac                                                    19

<210>  53

<211>  20

<212>  DNA

<213>  Artificial sequence

<220>

<223>  Synthetic oligonucleotide

<400>  53
cttcatggtg tactcagatc                                                   20

<210>  54

<211>  20

<212>  DNA

<213>  Artificial sequence

<220>

<223>  Synthetic oligonucleotide

<400>  54
ggttctagag gagatcattg tc                                            22

<210>  55

<211>  20

<212>  DNA

<213>  Artificial sequence

<220>

<223>  Synthetic oligonucleotide

<400>  55
gtcttgagag gtgagagctg                                                    20

<210>  56

<211>  20

<212>  DNA

<213>  Artificial sequence

<220>

<223>  Synthetic oligonucleotide

<400>  56
gcatgagcca ctgcatccag                                                    20

<210>  57

<211>  20

<212>  DNA

<213>  Artificial sequence

<220>

<223>  Synthetic oligonucleotide

<400>  57
gccctgaatg atgacatcag                                                    20

<210>  58

<211>  22

<212>  DNA

<213>  Artificial sequence

<220>

<223>  Synthetic oligonucleotide

<400>  58
caatctctat ggtaatcaga ac                                                 22

<210>  59

<211>  21

<212>  DNA

<213>  Artificial sequence

<220>

<223>  Synthetic oligonucleotide

<400>  59
catctcaact gtcctgcaca g                                                21

<210>  60

<211>  22

<212>  DNA

<213>  Artificial sequence

<220>

<223>  Synthetic oligonucleotide

<400>  60
cagtgactct tacctattta tg                                               22

<210>  61

<211>  20

<212>  DNA

<213>  Artificial sequence

<220>

<223>  Synthetic oligonucleotide

<400>  61
catcctgccg ctgtgtatac                                                  20

<210>  62

<211>  20

<212>  DNA

<213>  Artificial sequence

<220>

<223>  Synthetic oligonucleotide

<400>  62
cagccatagt gctgagactg                                                  20

<210>  63

<211>  20

<212>  DNA

<213>  Artificial sequence

<220>

<223>  Synthetic oligonucleotide

<400>  63
cacccatgag ccagtgcttc                                                    20

<210>  64

<211>  20

<212>  DNA

<213>  Artificial sequence

<220>

<223>  Synthetic oligonucleotide

<400>  64
gcttctgctc tcagagtcag                                                    20

<210>  65

<211>  19

<212>  DNA

<213>  Artificial sequence

<220>

<223>  Synthetic oligonucleotide

<400>  65
gtagacaggg ctggagttg                                                     19

<210>  66

<211>  20

<212>  DNA

<213>  Artificial sequence

<220>

<223>  Synthetic oligonucleotide

<400>  66
cagagctctg ctctaggatc                                                    20

<210>  67

<211>  21

<212>  DNA

<213>  Artificial sequence

<220>

<223>  Synthetic oligonucleotide

<400>  67
ctgttcagtg agcagattct c                                                  21

<210>  68

<211>  21

<212>  DNA

<213>  Artificial sequence

<220>

<223>  Synthetic oligonucleotide

<400>  68
cagtagcaag aaatctcatg c                                                  21

<210>  69

<211>  21

<212>  DNA

<213>  Artificial sequence

<220>

<223>  Synthetic oligonucleotide

<400>  69
caataggctc atctaggtct c                                                  21

<210>  70

<211>  20

<212>  DNA

<213>  Artificial sequence

<220>

<223>  Synthetic oligonucleotide

<400>  70
gactaacact acctcctctg                                                    20

<210>  71

<211>  20

<212>  DNA

<213>  Artificial sequence

<220>

<223>  Synthetic oligonucleotide

<400>  71
gtccatgaat gtctatgatc                                                    20

<210>  72

<211>  19

<212>  DNA

<213>  Artificial sequence

<220>

<223>  Synthetic oligonucleotide

<400>  72
gatgtcatag gcgctctgct g                                              21

<210>  73

<211>  19

<212>  DNA

<213>  Artificial sequence

<220>

<223>  Synthetic oligonucleotide

<400>  73
gtcgcggcag ctgcttcac                                                     19

<210>  74

<211>  20

<212>  DNA

<213>  Artificial sequence

<220>

<223>  Synthetic oligonucleotide

<400>  74
gcagagagtg aaggaggctg                                                    20

<210>  75

<211>  20

<212>  DNA

<213>  Artificial sequence

<220>

<223>  Synthetic oligonucleotide

<400>  75
gtactgagga ggctgaggag                                                    20

## Description

### FIELD AND BACKGROUND OF THE INVENTION

The present invention relates to methods and pharmaceutical compositions for modulating hair growth, and, more particularly, to methods and pharmaceutical compositions for inducing hair growth in cases of alopecia and methods and pharmaceutical compositions for inhibiting hair growth at locations where hair is unwanted, using modulators of P-cadherin.

Alopecia (baldness) is a deficiency of hair, either normal or abnormal, and is primarily a cosmetic problem in humans, although the negative psychological impact of hair loss is well known. See C. H. Mortimer et al., Clin. Exp. Dermatol. 9, 342-350 (1984). Dermatologists recognize many different types of alopecia, with androgenic alopecia being the most common cause of hair loss in both men and women. As this type of hair loss is more common and more severe in males, it is typically referred to as "male pattern baldness". However, it is thought that androgenic alopecia affects more that one third of individuals of either sex who have a strong family history of hair loss. See W. F. Bergfield, Clin. Dermatol. 6, 102-107 (1988).

One traditional treatment for alopecia is the method of hair transplantation. Typically, this method involves transplanting plugs of natural hair from areas of the scalp where hair is growing to bald areas. This procedure is costly, time-consuming, painful, and meets with only limited success.

Another common treatment for hair loss is the application of a chemical or drug for the purpose of stimulating hair growth. For example, U.S. Pat. No. 5,177,061 to Pickart proposes the topical application of glycyl-L-histidyl-L-lycine:copper(II) (GHL-Cu) and its derivatives to promote hair growth in warm-blooded animals. U.S. Pat. No. 4,832,946 to Green proposes a composition for topical application to mammalian hair or skin, comprising an amount of the cell-free supernatant from a culture of dermal papilla fibroblasts, which is said to increase hair growth in the rat. U.S. Pat. No. 5,358,714 to Green proposes the use of diacylglycerol activators of protein kinase C in order to increase or maintain hair growth in mammals, while U.S. Pat. No. 5,068,315 to Buultjens et al. proposes the application of purified hair growth regulating peptides (HGRP) to stimulate hair growth. It has also been suggested that retinoids, substituted pyrimidines, and immunosuppressants be used as possible treatments for hair loss, although methods utilizing these compounds have not been entirely successful in producing a reliable and safe method of inducing hair growth. See G. Bazzano et al., J. Invest. Dermatol. 101 (1 Supplement), 138S-142S (1993); H. Jiang et al., J. Invest. Dermatol. 104(4), 523-525 (1995).

In recent years, the topical application of minoxidil has been a widely-used method for treating androgenic alopecia. See A. R. Zapacosta, N. Eng. J. Med. 303, 1480-81 (1980). U.S. Pat. No. 4,139,619 to Chidsey, proposes a topical composition of minoxidil and related iminopyrimidines to stimulate the conversion of vellus hair to terminal hair and increase the rate of growth of terminal hair. However, despite its popularity, minoxidil has not performed in a completely satisfactory fashion in promoting hair growth in all target populations.

The following provides further insight with respect to pharmaceuticals used with limited success to treat alopecia.

Thymosin fraction 5 (TF5) is a partially purified mixture of polypeptides prepared from calf thymus glands. TF5 has been routinely prepared from calf thymus. However, it may also be prepared from porcine, ovine, murine, goat, rat, chicken, and human thymus tissues. Preparation and isolation of TF5 have been described (Hooper et al., "The purification and properties of bovine thymosin", Ann. NY Acad Sci. 249:125, 1975). TF5 consists of at least 40 to 50 distinct polypeptides on isoelectric focusing on polyacrylamide gel plates (pH 3.5-9.5). TF5 is essentially free of lipids, carbohydrates and endotoxins. TF5 has been demonstrated to be effective in reconstituting immune functions in thymic-deprived or immunodeprived animals, in humans with primary immunodeficiencies, and in immunosuppressed cancer patients. A primary effect of this mixture of peptides is to stimulate cell-mediated immunity. Two of the major biologically active ingredients in TF5 are thymosin alphal (Talphal) an immunomodulatory peptide of 28 amino acids (molecular weight 3,108 daltons) (Low et al., "The chemistry and biology of Thymosin I. Isolation and characterization and biological activities of Tα₁ and polypeptide beta1 from calf thymus," J. Bio. Chem. 254:981, 1979), and thymosin β₄ (Tβ₄), an actin-sequestering peptide of 43 amino acids (molecular weight 4,963 daltons) (Low, T. L. K., and Goldstein, A. L., "Chemical characterization of thymosin β₄," J. Bio. Chem. 257:1000, 1982). Tα₁ and Tα₄ are highly conserved in nature and their amino acid sequences are identical in most mammalian species. More than a dozen TF5-like preparations have been prepared from calf or porcine thymus tissue. These thymic extracts such as thymostimulin (TP-1), TFX, thymalin, thymoject, thym-Uvocal, and others, are variations of the TF5 formulation and are all partially purified preparations composed primarily of polypeptide mixtures with molecular weights of 15,000 or less. The major biologically active components of TF5 contain Tα₁ and Tα₄, as well as lower concentrations of other purified well characterized thymosin peptides such as prothymosin a (Pro Tα₁), Tα₂ to Tα₁ and Tβ₃, Tβ to Tβ₁₃, MB3S, MB40, ubiquitin, thymulin (FTS), thymic humoral factor (THFα₂) and thymopoietin (TP). The TF5-like extracts prepared by variations of the procedure used originally to prepare TF5 may also contain alpha and beta as key ingredients and smaller quantities of the other peptides described in TF5 such as Pro Tα₃, FTS, THFα₂, TP, ubiquitin and MB 35 and MB 40. Thymosin fraction 5 was found useful in the treatment of alopecia.

Substances that block DHT, testosterone, estradiol and EGF are thus believed to be of value in the prevention and treatment of alopecia. Systemic antiestrogens that have been used include tamoxiten citrate, a variety of triphenylethylene-based compounds and testolaotone.

Various azoles, especially ketoconazole have been found to have a significant role in the treatment of alopecia. Ketoconazole is important because it also blocks testosterone, DHT, and estrudiol non-specifically. However, systemic treatment to this compound over a long period of time results in loss of libido in men and women. In the context of topical treatment, this problem does not occur, and the effect relative to alopecia is much more significant. Undecylenic acid and a variety of systemic preparations may also be employed. These include grisocfulvia, terbinafine and fluconazole and other azoles, as well as ampotercin B and ampotercin like compounds.

Surprisingly, bioflavanoids can inhibit the production of epidermal growth factor (EGF). The most powerful of these, quercetin methyl chalcone, is water soluble. This compound effectively blocks EGF in relatively low concentrations. This greatly reduces hair loss and contributes significantly to hair growth. Polyamines also have this ability. Putrescine, protamine, etc., all will promote hair regrowth by blocking EGF. However, these substances are not cosmetically preferable for topical use because of their odor. It has been found that compounds containing bioflavanoids, especially quercetin methyl chalcone, greatly reduce hair loss and facilitate hair regrowth.

The presence of an ectoparasite and its role in alopecia prompted the development of an effective mitocide. Using fragrance-based chemicals, a skin penetrant, preferably PX-13, and a surfactant, it was discovered that this parasite could be effectively eliminated. Concomitantly, it was discovered that this composition was capable of effectively killing any mite, insect or chitin-coated organism. This was completely unexpected. Although others have recognized the efficacy of fragrance moieties in an aerosolized format, the novelty represented by this invention is inherent in the concomitant administration of a surfactant and an antilipase composition (such as PX-13, U.S. Pat. No. 5,659,055).

Certain indole-based compounds have a significant effect on hair loss. These include but are not limited to indole, skatole, indole-3-carbinol, and melatonin. They exert their effect by blocking the effects of virtually all estrogens. Melatonin has been used in high doses orally as an effective birth control agent, and a combination of indole-3-carbinol and melatonin is more powerful than either alone. Further, these compounds have antifungal properties. It should also be noted that very high concentrations of indole are found in jasmine fragrance and citrus flower based fragrances such as orange and lemon.

Melatonin has been found to alter the cyclic pattern of hair growth in rodents. Melatonin compositions and methods of using these melatonin compositions have been developed for treating the cosmetic and physical appearance of the scalp. (Pierpaoli, W., Regelson, W., Melatonin Compositions and Uses Thereof. U.S. Pat. No. 4,746,674 (1988)).

Melatonin was found to increase the 5-α reductase of seminiferous tubules for both progesterone and testosterone. Melatonin decreased androgen synthesis in both testicular interstitial cells and tubules. Currently, 5-α reductase modulating agents are being used to treat male pattern baldness.

Melatonin inhibits estrogen-mediated cell proliferation in MCF-7 cancer cells (Cos, S. Blask, D. E., Melatonin Modulates Growth Factor Activity in MCF-7 Human Breast Cancer Cells. J. Pineal Research 17:25-32 (1994). It was shown that melatonin down-regulates estrogen receptor expression. This group also showed that messenger RNA (MRNA) estrogen-receptor-mediated expression is inhibited by melatonin in MCF-7 breast cancer cells (Molis, T. M., Spriggs, L. L. Hill, S. M., Modulation of Estrogen Receptor mRNA Expression by Melatonin in MCF-7 Human Breast Cancer Cells. Mol. Endocrinol. 8:1681-90 (1994).

The inhibitory mechanism of melatonin relates to effects on cell cycle response resulting from a block to estrogenic growth stimulation, perhaps through effects on estrogen receptor availability.

Although a variety of treatments are presently offered to treat alopecia, not all subjects are responsive to such treatments, whereas some treatments are associated with unwanted side effects

Hence, there is still a great need for an efficient treatment for alopecia, which will overcome the limitations of the presently employed treatments and will offer an alternative to at least a subset of the patients.

While alopecia affects some individuals, other individuals suffer excessive hair growth and/or are culturally influenced by the trend of hairless body and hence treatments for the removal of hair are at their highest demand.

Various methods of hair removal are known. For example, the hair can be shaved from the body or can be removed by the use of tweezers or other instruments which pluck the hairs from the skin, such as devices including bent rotating coil springs and the like. In addition, chemical depilatory preparations and waxes have been formulated for the purpose of hair removal. Conventional depilatory preparations, often containing sulphide chemicals, act by weakening the structure of the hair to such an extent that scraping the cream off the skin breaks the hair at skin level and thus removes it. Alternatively, waxes can be applied to the skin which can then be peeled away with the hairs embedded therein.

Each of these methods has attendant disadvantages. Shaving brings only temporary alleviation since the roots of the hair are still present and the hair will grow again after a very short period. Also, there is the danger of cutting the skin on shaving. Chemical depilatory preparations tend to have an unpleasant smell and the use of waxes and coil spring devices can cause some discomfort.

Currently, the most common methods for hair removal involve the use of hair removal creams, as well as shaving, waxing and electrolysis. Although creams and shaving are popular because they can be readily used at home, they are inadequate because they must be used on a regular basis. Waxing and electrolysis offer longer term hair removal. Both methods, however, can be time-consuming and are often quite painful. For example, removing a typical mustache which contains 1,000 to 2,000 hairs by electrolysis may take up to 50 visits before the hair removal is complete.

More recently, lasers alone or in conjunction with topical formulations containing carbon particles, hair dyes, hematoporphyrin derivatives or aminolevulinic acid have been used for hair removal (See, U.S. Pat. Nos. 5,226,907 and 5,425,728; Grossman, M. et al. Lasers Surg. Med. Suppl. 7:44 (1995)). Such treatments are generally not selective in that they result in only partial destruction of hair follicles and may promote skin reaction.

All of these hair removal treatments fail to prevent new hair growth. Hirsutism is defined as terminal hair growth in women in a pattern typical of men. Current modalities include the use of cosmetic means, anti-androgen therapy such as oral contraceptives, cyproterone aceate, spironolactone with moderate success rate and many associated side effects.

Accordingly, there exists a great need for an efficient method of inhibiting hair growth.

The present invention emerges from a novel discovery that a mutation in the *CDH3* gene which encodes P-cadherin is the cause for the autosomal recessive disorder congenital hypotrichosis which is associated with juvenile macular dystrophy (HJMD; MIM601553), and is characterized by hair loss heralding progressive macular degeneration and early blindness (Souied, E. et al. *Ophthalmic Genet.* 16, 11-15 (1995); Raison-Peyron, N. et al. Br. J. Dermatol. 143, 902-904 (2000); Da Cruz, L. & McAllister, I.L. Br. J. Ophthalmol. 85, 239 (2001)).

Using homozygosity mapping in 4 consanguineous families, the HJMD gene was localized to 16q22.1. This region harbors CDH3 encoding P-cadherin, which is expressed in the retinal pigment epithelium and hair follicles. Mutation analysis revealed in all families revealed a common homozygous deletion in exon 8 of *CDH3.* These results establish the molecular etiology of HJMD and implicate for the first time a cadherin molecule in the pathogenesis of a human hair and retinal disorder.

### SUMMARY OF THE INVENTION

According to one aspect of the present invention there is provided a method of identifying a hair growth modulator (i.e., hair growth inhibitor or inducer) comprising identifying a P-cadherin modulator (i.e., P-cadherin inhibitor or inducer); and testing whether the P-cadherin modulator is functional as a hair growth modulator.

According to another aspect of the present invention there is provided a method of identifying a hair growth modulator comprising identifying a molecule being capable of specifically binding to P-cadherin; and testing whether the molecule is functional as a hair growth modulator.

According to yet another aspect of the present invention there is provided a method of modulating (i.e., inhibiting or inducing) hair growth, the method comprising administering to a subject in need a therapeutically effective amount of a P-cadherin modulator (i.e., P-cadherin inhibitor or inducer) functional as a hair growth modulator.

According to still another aspect of the present invention there is provided a pharmaceutical composition for modulating hair growth, the pharmaceutical composition comprising, as an active ingredient, a therapeutically effective amount of a P-cadherin modulator functional as a hair growth modulator.

According to further features in preferred embodiments of the invention described below, the pharmaceutical composition further comprising, as an additional active ingredient, a therapeutically effective amount of an additional hair growth modulator (i.e., an additional hair growth inhibitor or inducer, respectively).

According to still further features in the described preferred embodiments, the P-cadherin modulator is an antisense oligonucleotide capable of specifically binding to P-cadherin gene, pre-messenger RNA or messenger RNA under physiological conditions and hence serves as a P-cadherin inhibitor.

According to still further features in the described preferred embodiments the P-cadherin modulator is an antisense construct encoding an antisense transcript capable of specifically binding to P-cadherin gene, pre-messenger RNA or messenger RNA under physiological conditions.

According to still further features in the described preferred embodiments the P-cadherin modulator is a polynucleotide capable of directing P-cadherin expression in hair follicle cells and hence serves as a P-cadherin inducer.

According to still further features in the described preferred embodiments the P-cadherin modulator or the molecule capable of binding P-cadherin is an anti-P-cadherin antibody and hence serves as a P-cadherin inhibitor.

According to still further features in the described preferred embodiments the P-cadherin modulator or the molecule capable of binding P-cadherin is an a small molecular weight organic compound, which may serve as either a P-cadherin inhibitor or inducer.

According to still further features in the described preferred embodiments identifying the molecule being capable of specifically binding to P-cadherin is by a two hybrid system.

According to an additional aspect of the present invention there is provided a hair growth modulator identified by the method described herein.

According to yet an additional aspect of the present invention there is provided a method of modulating hair growth comprising administering to a subject in need a therapeutically effective amount of the hair growth modulator described herein.

The present invention successfully addresses the shortcomings of the presently known configurations by providing new means with which to modulate hair growth.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

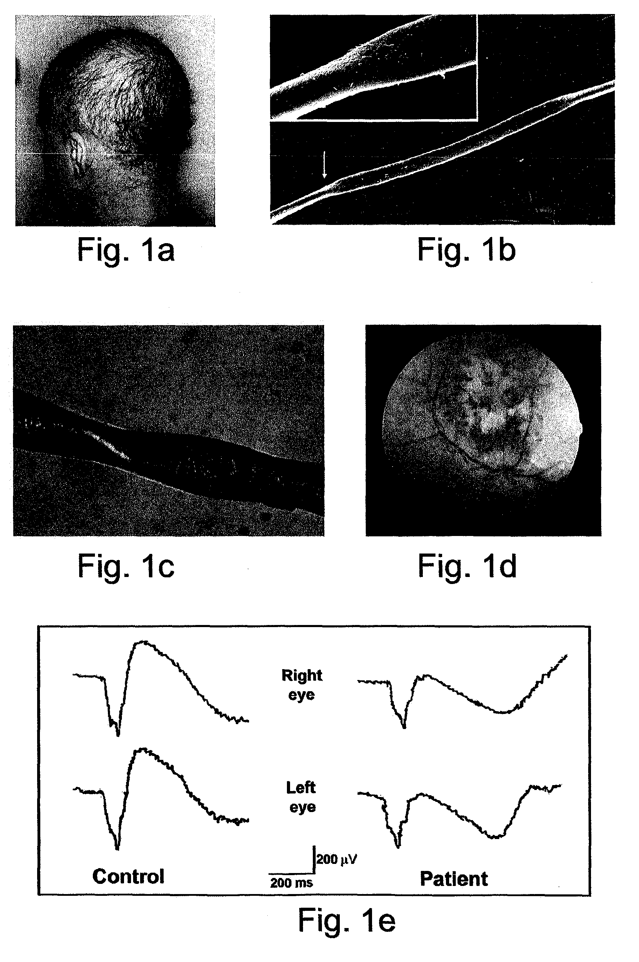

FIGs. 1a-e demonstrate clinical spectrum of HJMD. 1a, Sparse, short hair on the scalp of a 17-year old affected individual; 1b, Scanning electron microscopy of a hair shaft. Note the fusiform beading along the hair shaft (original magnification X70), reminiscent of pseudomonilethrix (MIM177750). This abnormality is due to flattening of the shaft (arrow) seen in details in insert (original magnification X 500); 1c, Pili torti (180° twisting of the hair) apparent by light microscopy (original magnification X100); 1d, Eye fundus examination in HJMD. Note atrophic scars of the macular area surrounded by degenerative pigmentary changes; 1e, Electroretinogram of a HJMD patient (left) compared to a normal profile (right) demonstrating reduced wave amplitude, consistent with macular dysfunction.

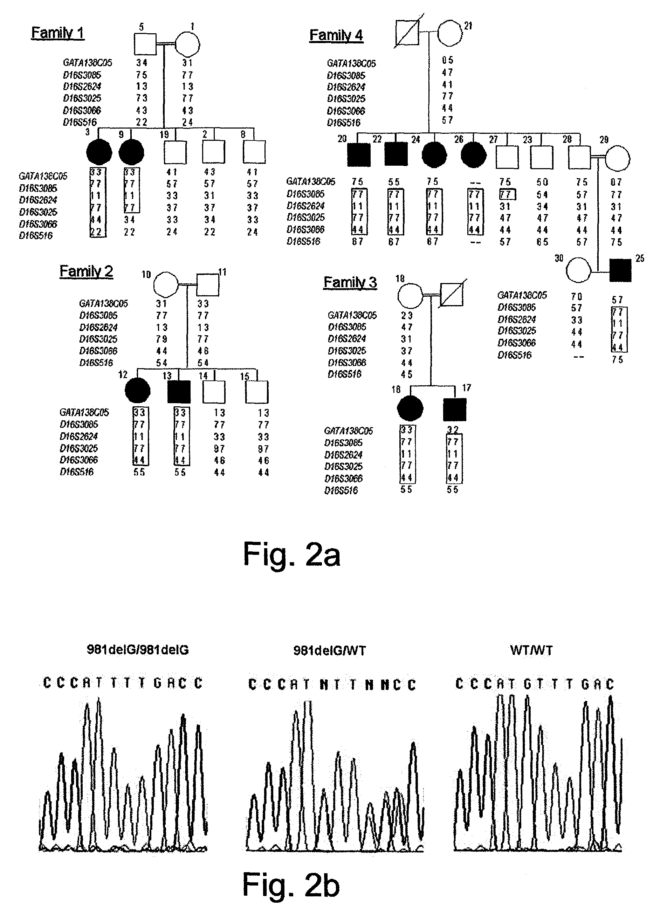

FIGs. 2a-g demonstrates a mutation in CDH3 which underlies HJMD. 2a, Haplotype analysis in 4 HJMD families using 6 polymorphic markers on 16q22.1. The shared disease-associated haplotype is boxed; 2b, Sequence analysis reveals a homozygous G deletion at cDNA position 981 of CDH3 in patient 22 (left panel); each parent carries this mutation in a heterozygous state (middle panel); the wildtype (WT) sequence is shown in the right panel; 2c, Segregation of the 981delG in family 1 is illustrated by restriction fragment analysis. 981delG causes loss of an enzyme recognition site for NlaIII. Upon digestion, amplicons of exon 8 of CDH3 (320 bp), normally resulting in three fragments (individuals 8 and 19), yields only two fragments in affected individuals (3 and 9) and four fragments in heterozygous carriers of the mutation (individuals 5 and 1); 2d, Predicted wildtype (black) and mutant (red) amino acid sequence of P-cadherin; 2e, Expression of CDH3 in the skin of a patient (P) and a control (C) determined by RT-PCR amplification of RNA using gene-specific intron-crossing primers for CDH3 and β-actin; 2f, Schematic representation of the wildtype and predicted mutant protein structures; 2g, Immunostaining of fresh frozen skin biopsies obtained from a patient and a control with antibodies specific for P-cadherin (P-cad) or E-cadherin (E-cad) (Santa Cruz) (original magnification X 630). E-cadherin is expressed both in control and patient skin. Note reduced staining for P-cadherin in the patient epidermis (left upper panel) and follicular epithelium (right upper panel).

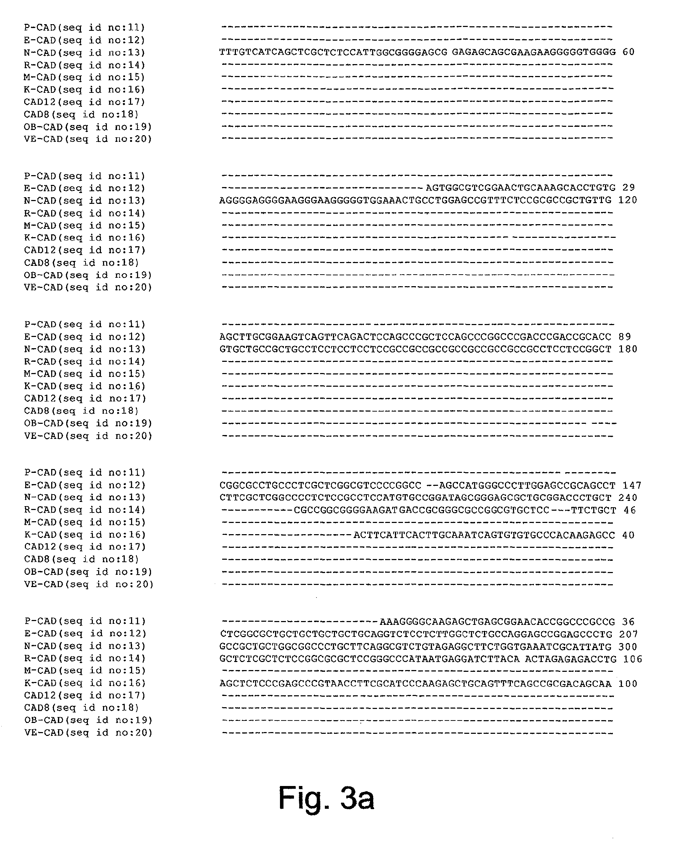

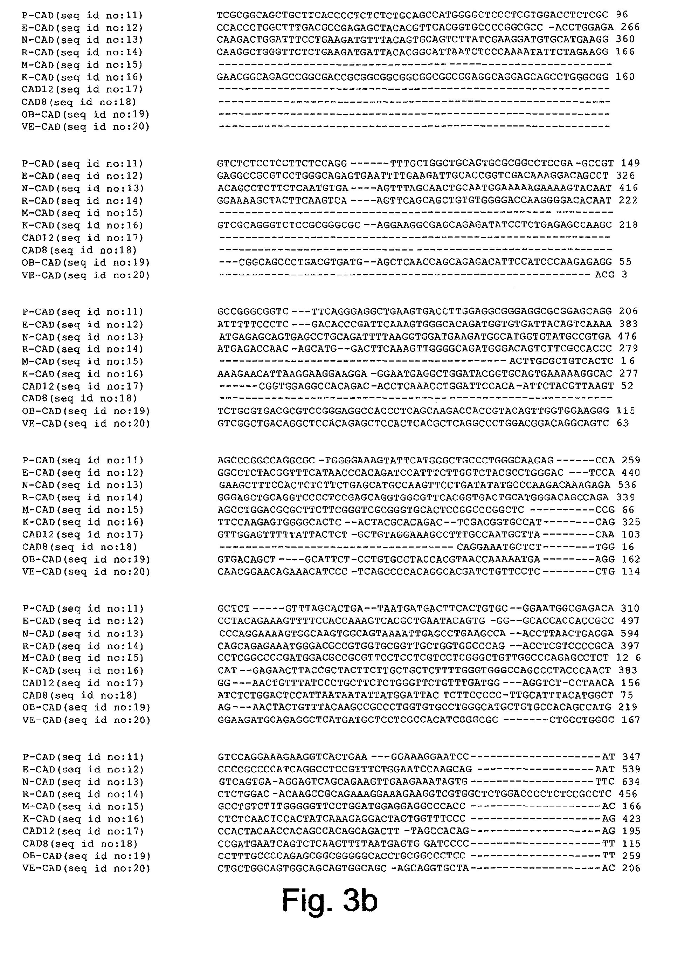

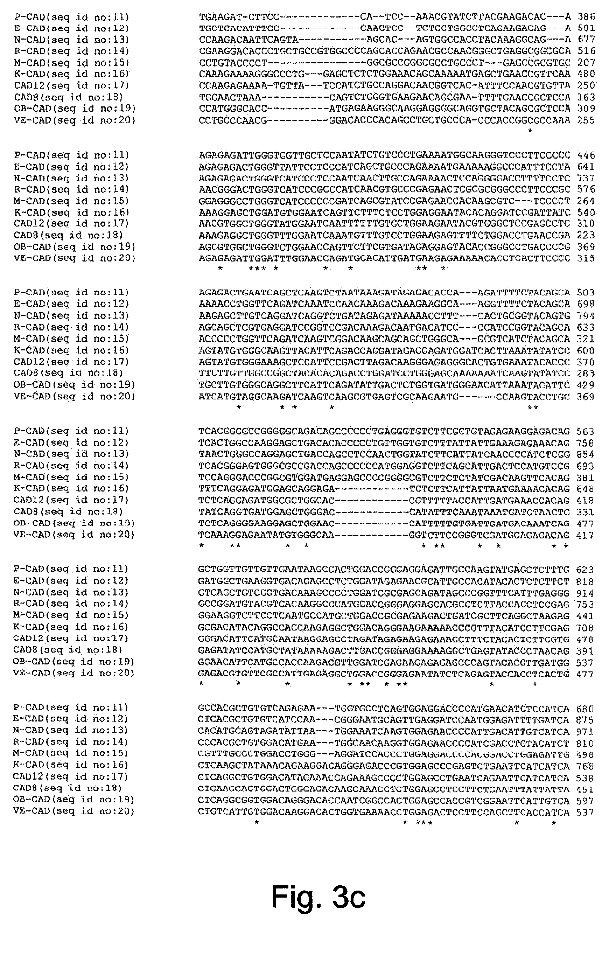

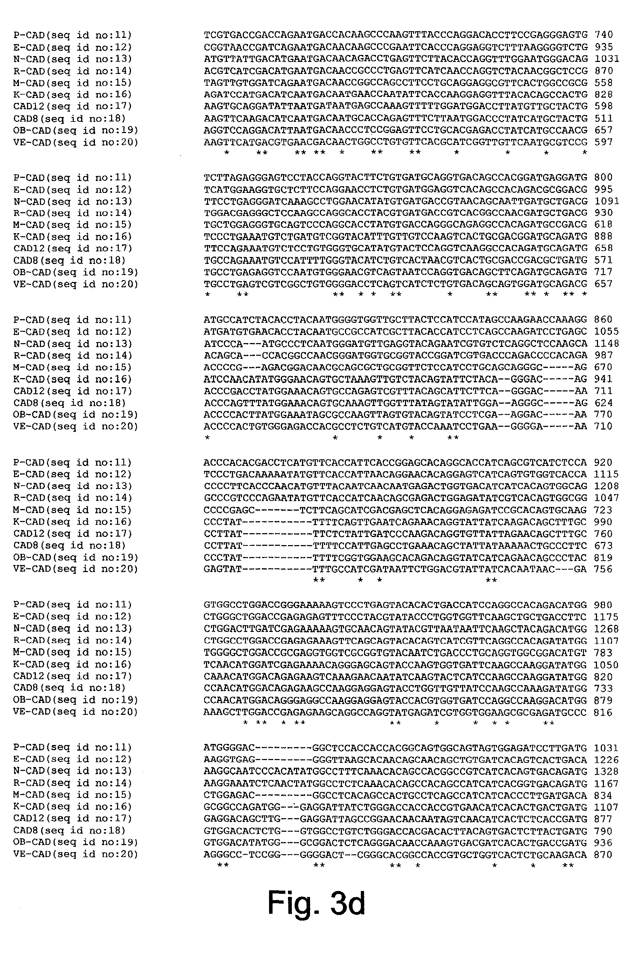

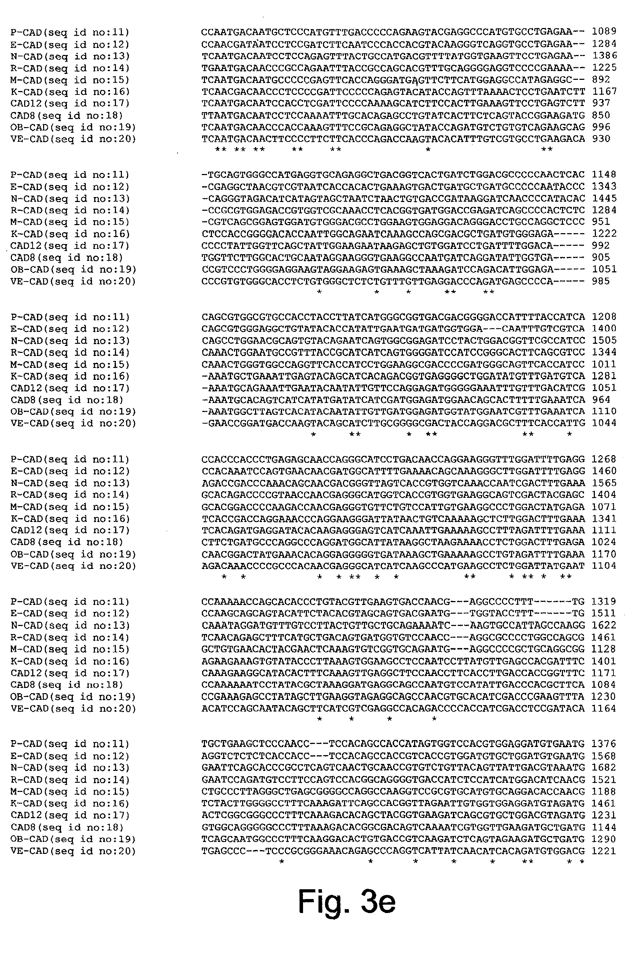

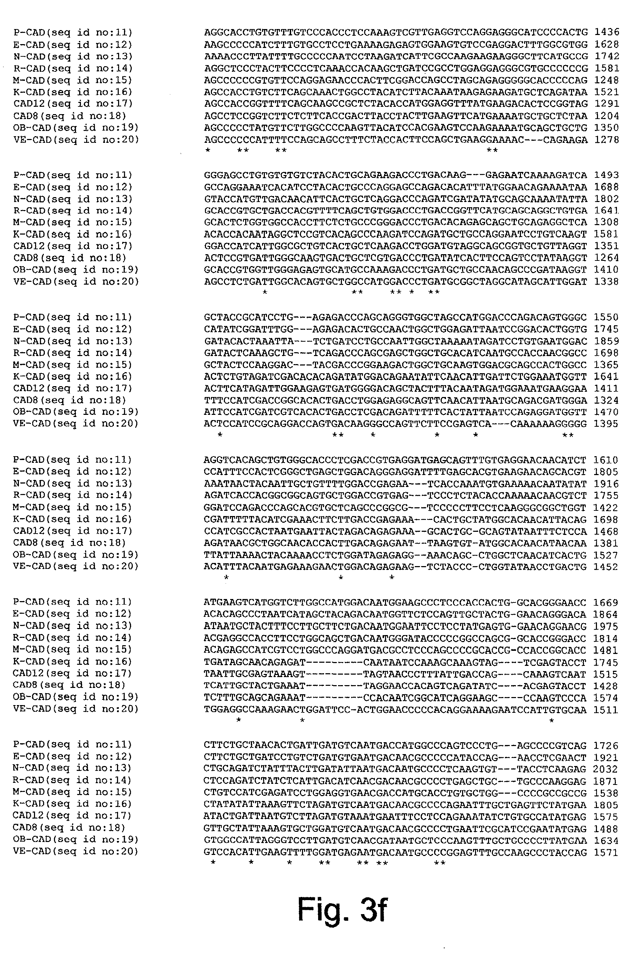

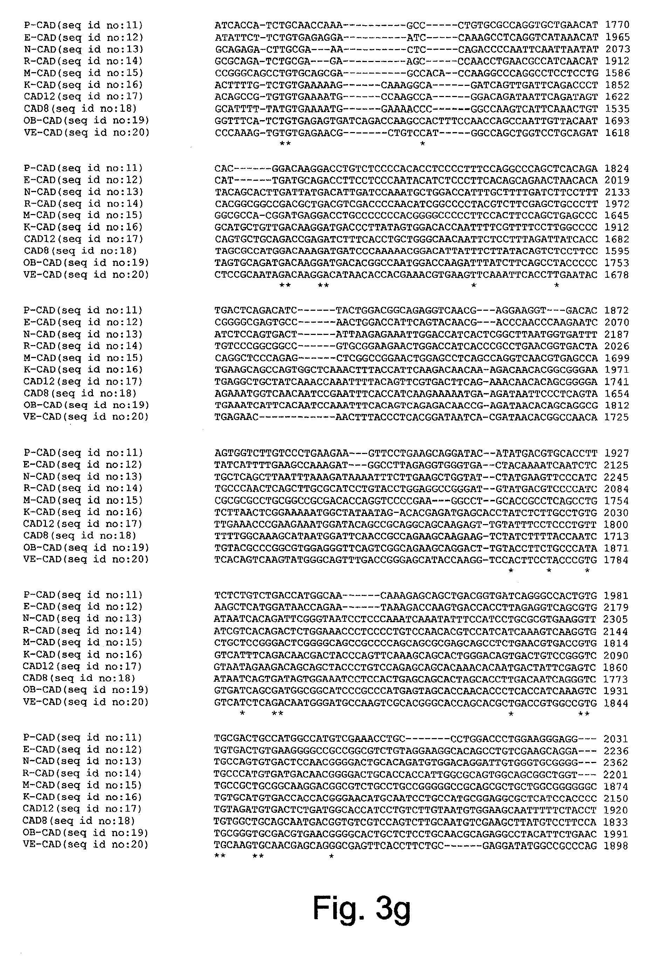

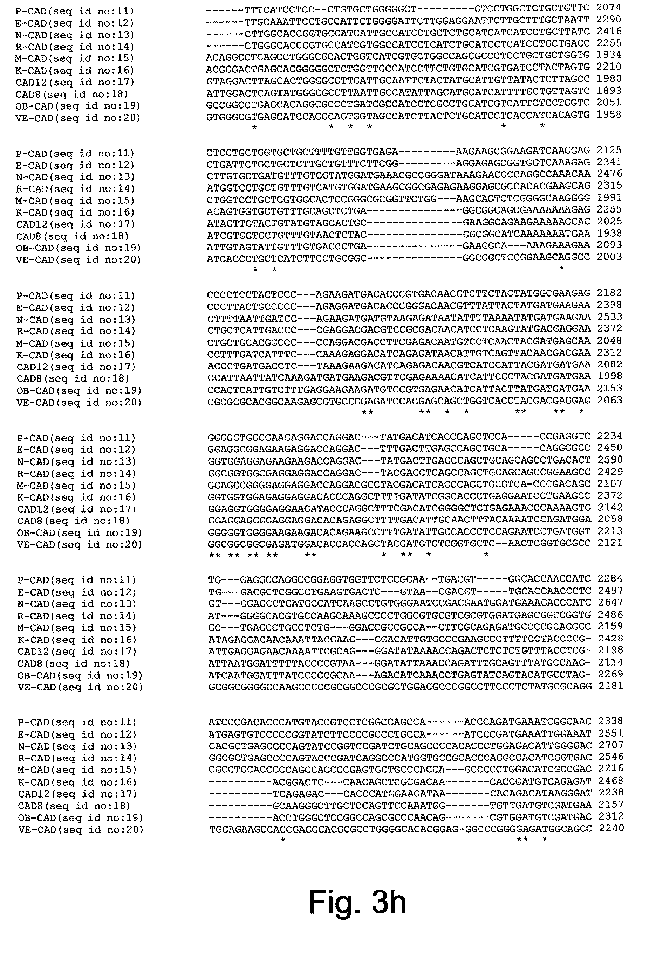

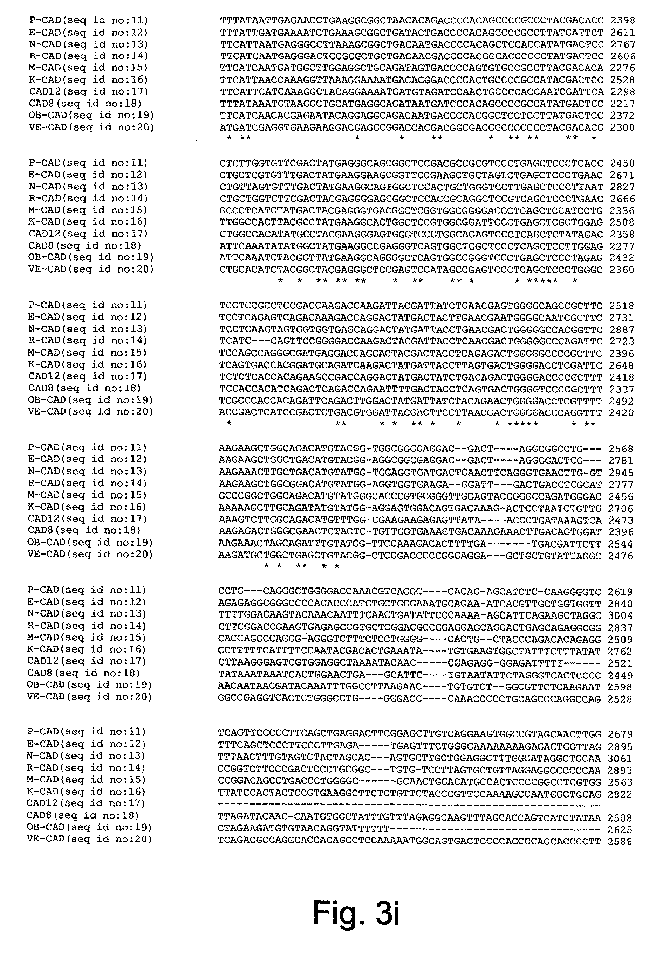

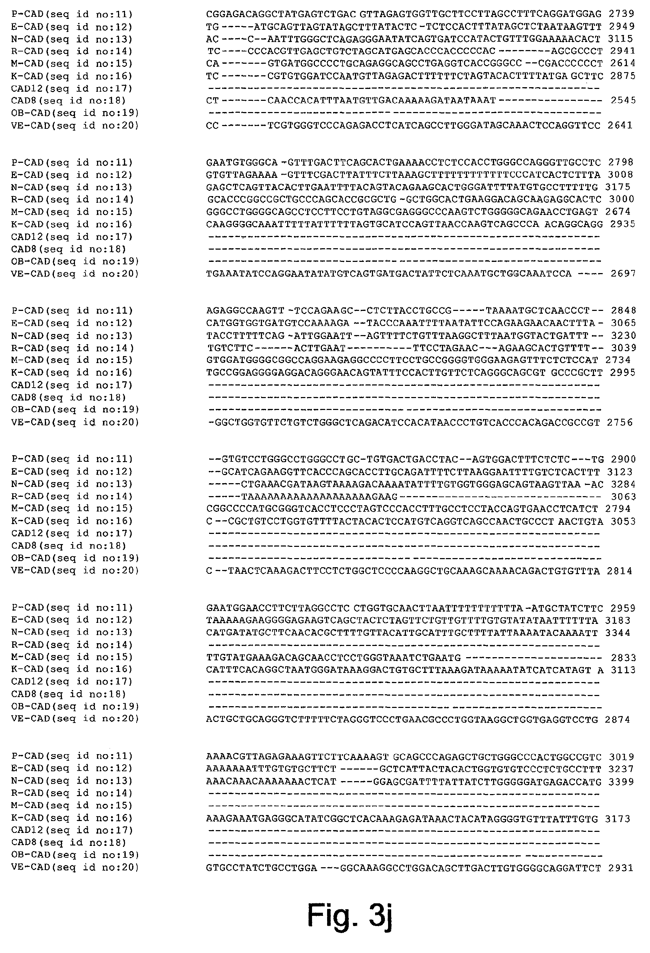

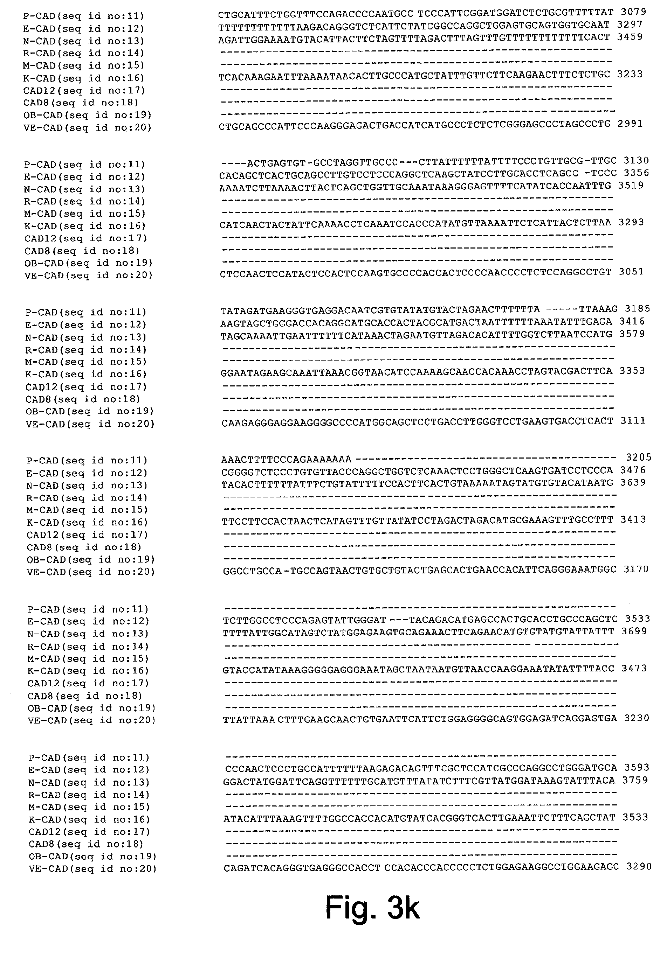

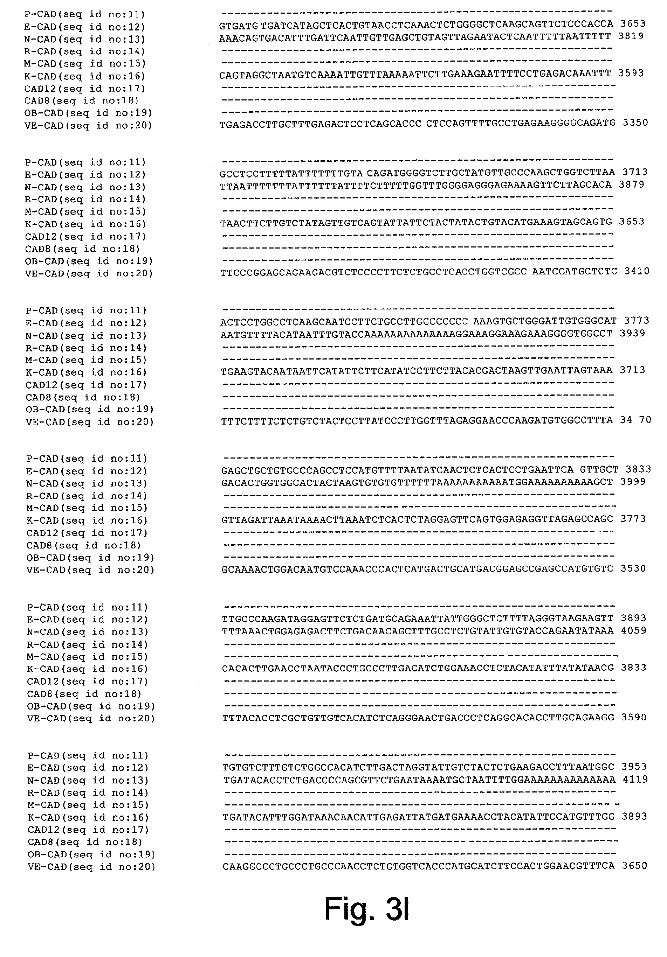

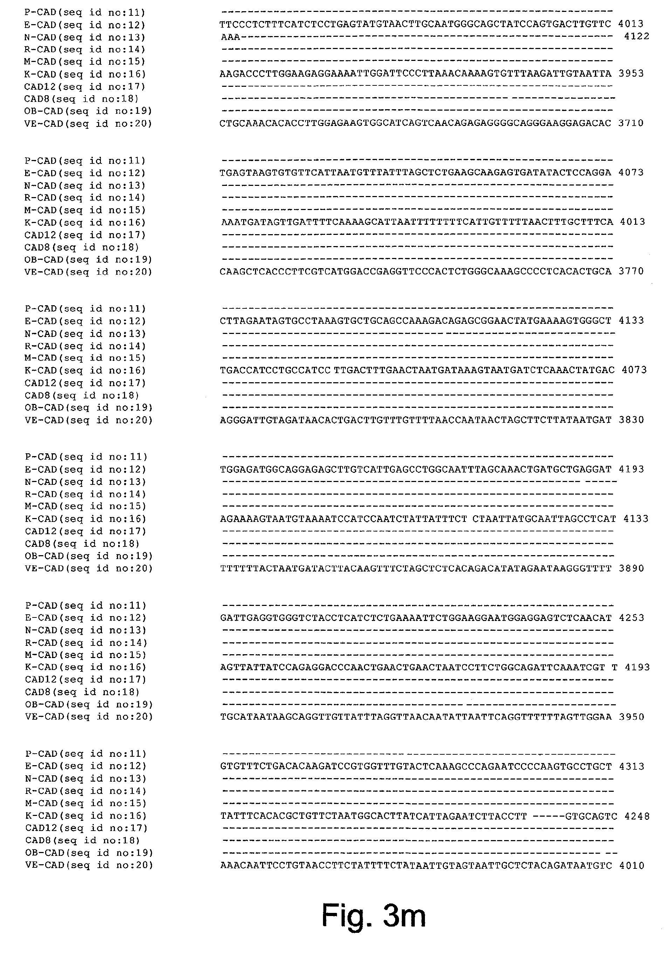

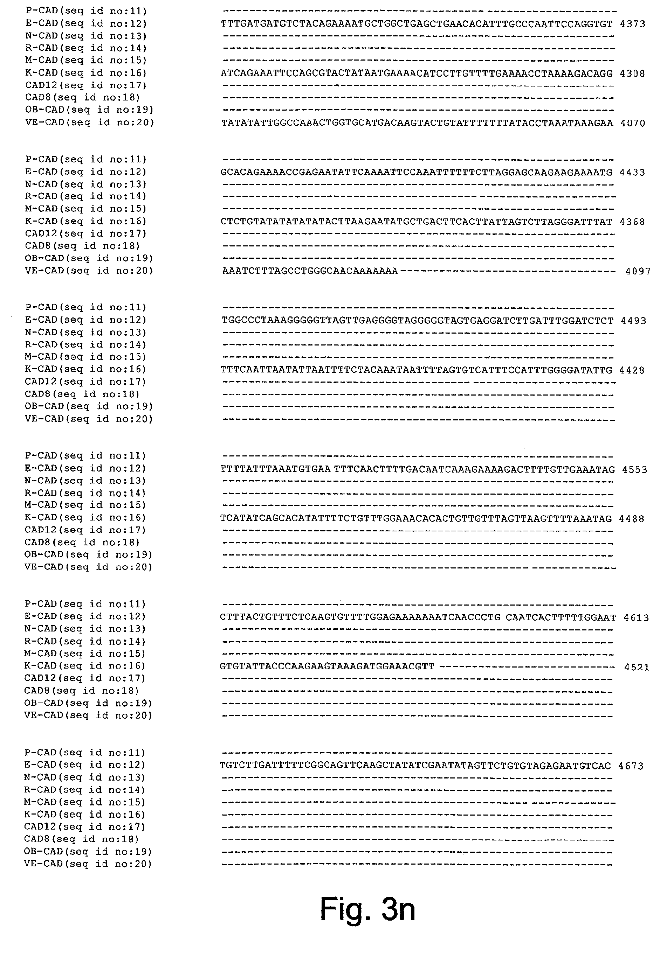

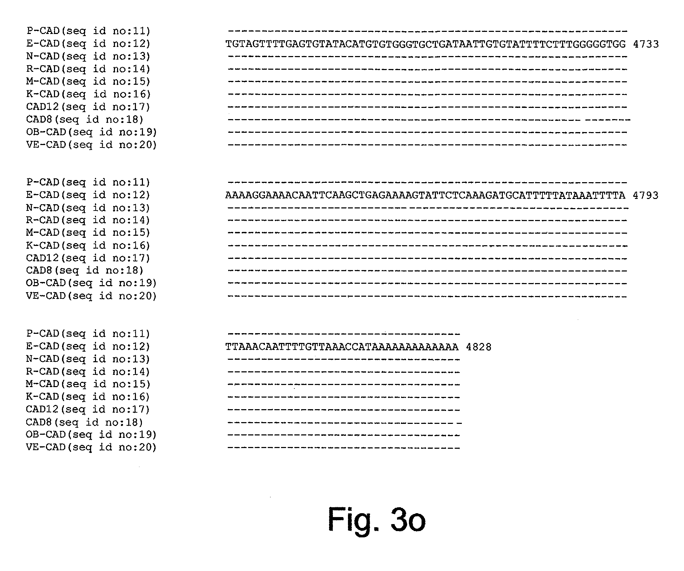

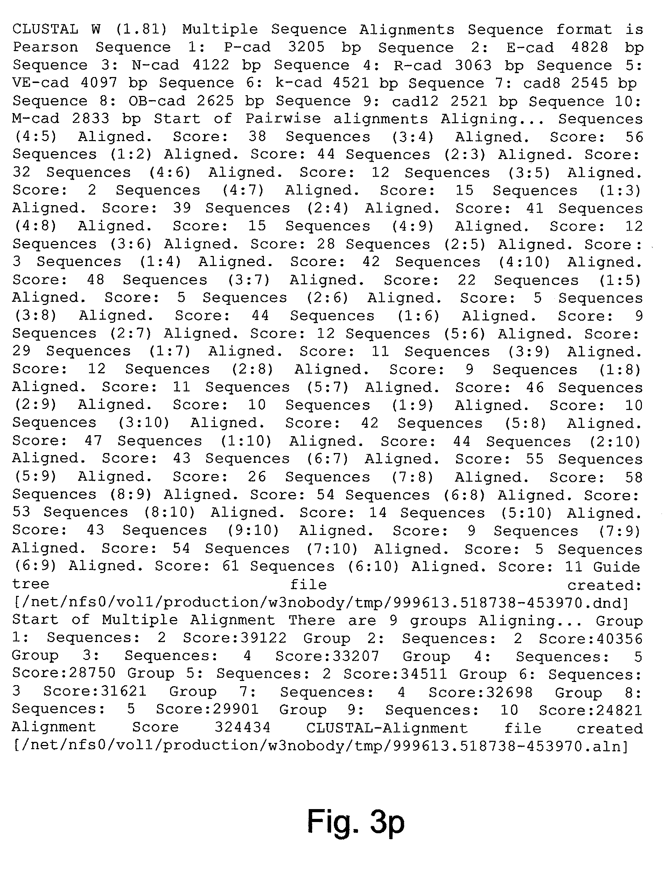

FIGs. 3a-p show multiple alignment of human cadherin cDNAs. Multiple alignment was made using 'clustalW' software (from EMBL) with all parameters set on default. Bases common to all cadherins are marked with an asterisks.

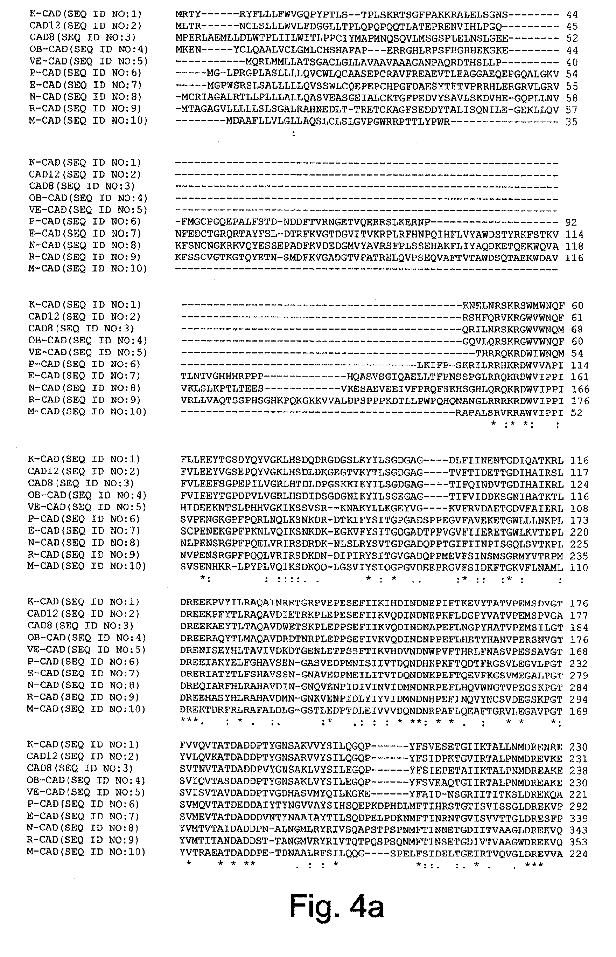

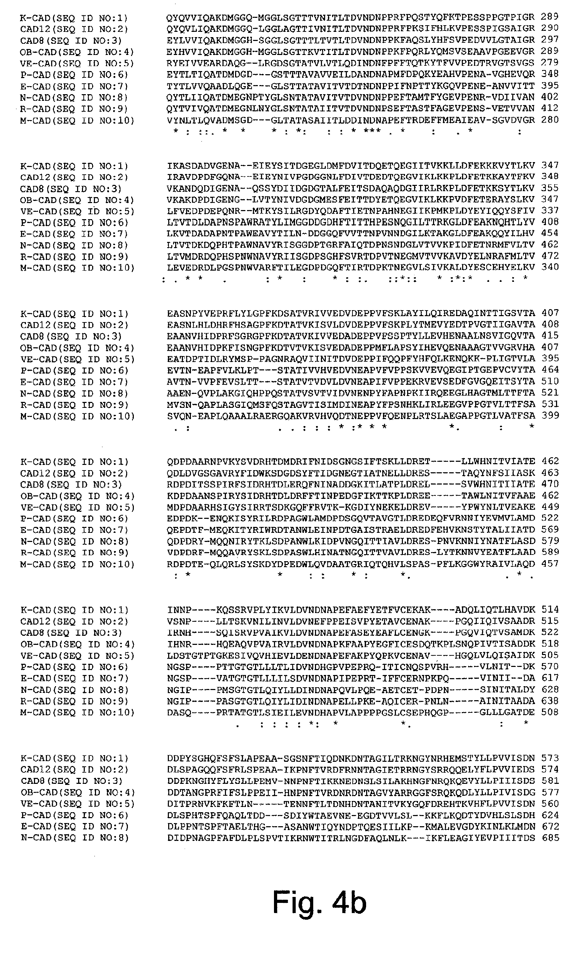

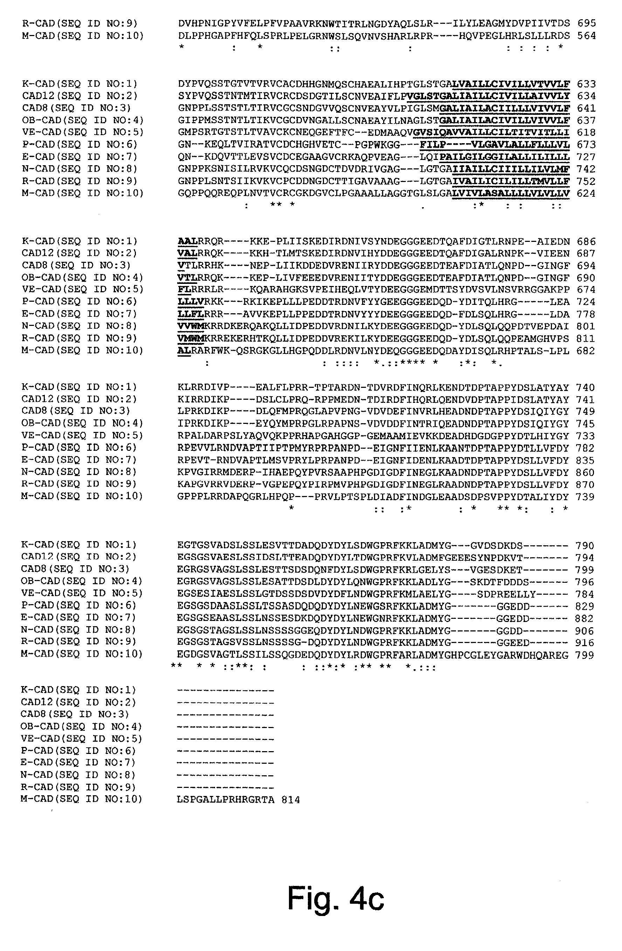

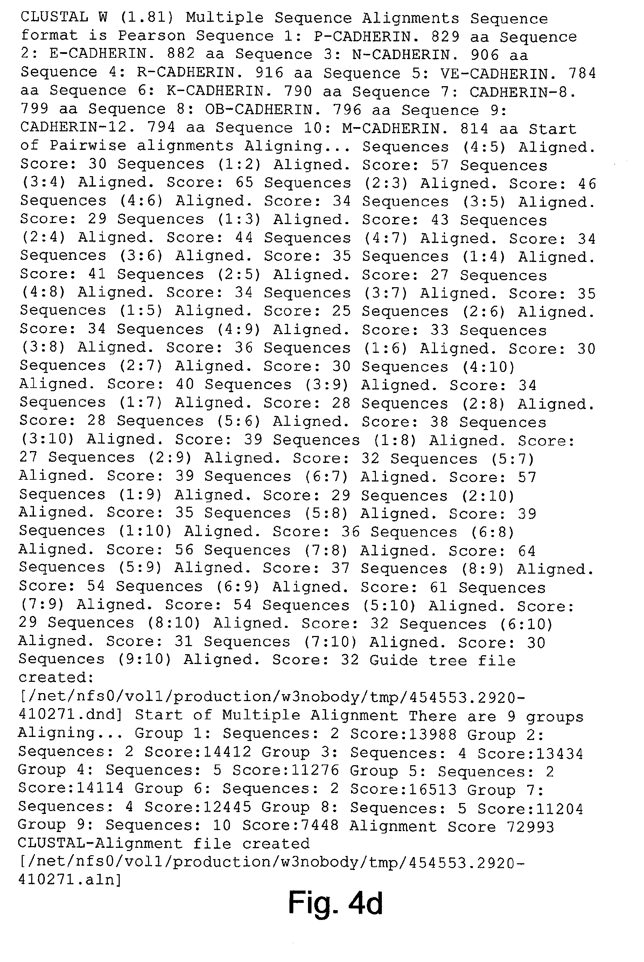

FIGs. 4a-d show multiple alignment of human cadherin cDNAs. Multiple alignment was made using 'clustalW' software (from EMBL) with all parameters set on default. For each precursor protein the first 21 amino acids from the N' serve as signal peptide. The bold and underlined letters in each sequence represent the transmembrane domain. The sequence up-stream to the trans membrane domain is the extracellular. The sequence down stream is the cytoplasmic part of the protein. Perfect alignment between cadherin family members is marked at the bottom of every cluster. In order to select for immunogenic peptides of P-cadherin regions of low similarity were analyzed for immugenicity using the 'peptidestructure' software of the 'GCG package'.

### DESCRIPTION OF THE PREFERRED EMBODIMENTS

The present invention is of methods and pharmaceutical compositions which can be used to modulate hair growth. Specifically, the present invention can be used to (i) treat alopecia (boldness) or otherwise induce hair growth on the one hand; and to (ii) inhibit hair growth, in cases of excessive hairiness or for cosmetic purposes, on the other hand. The invention is further of methods of identifying P-cadherin modulators effective in either inducing hair growth in cases of alopecia and inhibiting hair growth in cases of excessive hairiness and/or for cosmetic reasons.

The principles and operation of methods and pharmaceutical composition according to the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Although P-cadherin was originally identified more than 10 years ago and was shown to be expressed in the mouse hair follicles, nothing was known until recently about its role in the morphogenesis of the hair follicle. The inventors of the present invention identified 4 families affected with congenital hypotrichosis associated with juvenile macular dystrophy (HJMD; MIM601553). Juvenile macular dystrophy is an autosomal recessive disorder of unknown etiology characterized by hair loss heralding progressive macular degeneration and early blindness (Souied, E. *et al. Ophthalmic Genet.* 16, 11-15 (1995); Raison-Peyron, N. *et al. Br. J. Dermatol.* 143, 902-904 (2000); Da Cruz, L. & McAllister, I.L. *Br. J. Ophthalmol.* 85, 239 (2001)). Using homozygosity mapping in these consanguineous families, the HJMD gene was localized to chromosome 16q22.1. This region harbors the *CDH3* gene encoding P-cadherin, which is expressed in the retinal pigment epithelium and hair follicles. Mutation analysis revealed in all families a common homozygous deletion in exon 8 of *CDH3.* These results establish the molecular etiology of HJMD and positively demonstrate for the first time the importance of P-cadherin in the morphogenesis of the hair follicle. These findings pave the way for various novel therapeutic strategies based on the modulation of P-cadherin in hair disorders such as the design of P-cadherin inhibitors for the treatment of unwanted hair growth, such as hirsutism.

Given the fact that P-cadherin is necessary for the morphogenesis of the hair follicle; and given the fact that lack of functional P-cadherin is not associated with any skin phenotype, it is clear that modulation of P-cadherin function represents an attractive strategy for modulating hair growth in for example hirsutism or for cosmetic reasons.

Hirsutism is defined as terminal hair growth in women in a pattern typical of men. Current modalities include the use of cosmetic means, anti-androgen therapy such as oral contraceptives, cyproterone aceate, spironolactone with moderate success rate and many associated side effects. The design of such inhibitors may be based on the use of specific antisense oligonucleotides transferred using novel and efficient methods targeted to the hair follicle Domashenko et al, *Nature Biotechnol* 18, 43-47 (2000), which is incorporated herein by reference). Such a strategy has been successful with another regulator of hair growth, the hairless protein, in a murine model (Cserhalmi-Friedman, P.B. & Christiano, A.M. *J Invest Dermatol,* in press, and incorporated by reference herein). Alternatively, the well-known structure of P-cadherin may be amenable to computer-based inhibitor designing.

On the other hand, correction or partial correction of hair loss in HJMD and other alopecia patients may be achieved by the use of a P-cadherin inducer. Partial correction of hair loss in HJMD patients during puberty indicates that P-cadherin expression is involved in the androgen-mediated regulation of hair growth. Indeed, expression of several cadherins have been shown to be controlled by sex hormones.

Hence, according to one aspect of the present invention there is provided a method of identifying a hair growth modulator (i.e., hair growth inhibitor or inducer). The method according to this aspect of the present invention is materialized by identifying a P-cadherin modulator (i.e., P-cadherin inhibitor or inducer); and thereafter testing whether the P-cadherin modulator is functional as a hair growth modulator.

According to another aspect of the present invention there is provided a method of identifying a hair growth modulator. The method according to this aspect of the present invention is materialized by identifying a molecule capable of specifically binding to P-cadherin; and thereafter testing whether the molecule is functional as a hair growth modulator.

According to yet another aspect of the present invention there is provided a method of modulating (i.e., inhibiting or inducing) hair growth. The method according to this aspect of the present invention is materialized by administering to a subject in need a therapeutically effective amount of a P-cadherin modulator (i.e., P-cadherin inhibitor or inducer) functional as a hair growth modulator.

According to still another aspect of the present invention there is provided a pharmaceutical composition for modulating hair growth. The pharmaceutical composition comprising, as an active ingredient, a therapeutically effective amount of a P-cadherin modulator functional as a hair growth modulator.

Preferably, the pharmaceutical composition further comprises, as an additional active ingredient, a therapeutically effective amount of an additional hair growth modulator (i.e., an additional hair growth inhibitor or inducer, respectively). Such hair growth modulators (both hair growth inhibitors and hair growth inducers) are discussed at length at the Background section and elsewhere hereinabove.

As used herein, the phrase "P-cadherin modulator" includes any and all molecules capable of increasing or decreasing specifically P-cadherin expression and/or P-cadherin function, such as binding β-catenin and/or other cellular skeleton components.

As used herein the term "specifically" refers to an effect which is unique to P-cadherin expression of activity and not to other cadherins or other cell components.

As used herein, the phrase "P-cadherin inhibitor" includes any and all molecules capable of decreasing specifically P-cadherin expression and/or P-cadherin function, such as binding β-catenin and/or other cellular skeleton components.

As used herein, the phrase "P-cadherin inducer" includes any and all molecules capable of increasing specifically P-cadherin expression and/or P-cadherin function, such as binding β-catenin and/or other cellular skeleton components.

As used herein, the phrase "hair growth modulator" includes any and all molecules capable of increasing (e.g., accelerating) or decreasing (e.g., suppressing) hair growth.

As used herein, the phrase "hair growth inhibitor" includes any and all molecules capable of decreasing or suppressing hair growth.

As used herein, the phrase "hair growth inducer" includes any and all molecules capable of increasing or accelerating hair growth.

Several assays are known for monitoring P-cadherin function, such as binding β-catenin and/or other cellular skeleton components. These assays include immunoprecipitation of cell extracts with an anti-Pcadherin antibody and immunoblotting of this reaction products to reveal a 116kD band representing P-cadherin as well as three smaller bands corresponding in decreasing size order to α-, β-, γ-catenins; microscopic examination of cell cultures in the presence of anti-E cadherin in which further inhibition of P-cadherin function leads to cell-cell interaction disruption and inhibition of keratinocyte differentiation; inhibition of actin cytoskeleton formation under changing Ca⁺⁺ concentrations in keratinocyte cell-cultures (Lewis, J.E., Jensen, P.J. & Wheelock, M.J *J. Invest. Dermatol.* **102,** 870-877 (1994)). According to one embodiment of the present invention, the P-cadherin modulator is an antisense oligonucleotide capable of specifically binding to P-cadherin gene, pre-messenger RNA or messenger RNA under physiological conditions and hence serves as a P-cadherin inhibitor, reducing its level of expression.

Figures 3a-p present an alignment of human cadherin cDNAs (SEQ ID NOs:11-20). Those regions for which no or low homology exists between P-cadherin and other human cadherins were identified. The following oligonucleotides are exemplary oligonucleotides capable of specifically binding to P-cadherin gene, pre-messenger RNA or messenger RNA under physiological conditions and hence serve as P-cadherin inhibitors, via inhibiting P-cadherin expression:
1. GAGAGGTCCACGAGGGAGCCC (74-94) (SEQ ID NO:21)
2. CACGGCTCGGAGGCCGCGCA (131-150) (SEQ ID NO:22)
3. CGCCTCCAAGGTCACTTCAG (171-191) (SEQ ID NO:23)
4. CTAAACAGAGCTGGCTCTTG (251-270) (SEQ ID NO:24)
5. AGTGACCTTCTTTCCTGGAC (311-330) (SEQ ID NO:25)
6. GTTTGGATGGGAAGATCTTC (349-368) (SEQ ID NO:26)
7. CTTGTGTCTTCGTAAGATAC (369-388) (SEQ ID NO:27)
8. CTGGGGGAAGGGACCCTTGC (429-448) (SEQ ID NO:28)
9. CTTCAGCACAAAAGGGGCCT (1308-1027) (SEQ ID NO:29)
10. CAACGACTTTGGAGGGTGGGAC (1391-1412) (SEQ ID NO:30)
11. GTTGTTCCTCACAAACTGCTC (1586-1606) (SEQ ID NO:31)
12. GTGGTGGGAGGGCTTCCATTG (1636-1656) (SEQ ID NO:32)
13 GATCTGACGGGGCTCAGGGAC (1709-1729) (SEQ ID NO:33)
14. CATCTGTGAGCTGGGCCTGG (1807-1826) (SEQ ID NO:34)
15. CCTTCCTCGTTGACCTCTGCC (1846-1866) (SEQ ID NO:35)
16. CTTTGTTGCCATGGTCAGACAG (1931-1952) (SEQ ID NO:36)
17. GCAGCACCAGCAGGAGGAAC (2071-2090) (SEQ ID NO:37)
18. GGTTGGTGCCACGTCATTGCG (2261-2281) (SEQ ID NO:38)
19. GTTGGCTGGCCGAGGACGGTAC (2278-2298) (SEQ ID NO:39)

As used herein, unless otherwise indicated, the term "antisense" or "antisense therapeutic" refers to oligonucleotides, modified oligonucleotides or other chemical compositions that bind in a sequence specific manner to a specified gene, its pre-mRNA, or its mRNA.

As used herein, unless otherwise indicated, the term "oligonucleotide" includes both oligomers of ribonucleotides, i.e., oligoribonucleotides, and oligomers of deoxyribonucleotides, i.e., oligodeoxyribonucleotides or oligodeoxynucleotides.

Unless otherwise indicated, the term "oligonucleotide" also includes oligomers that may be large enough to be termed "polynucleotides."

The terms "oligonucleotide", "oligodeoxynucleotide" and "oligodeoxyribonucleotide" include oligomers and polymers of the biologically significant nucleotides, adenine, deoxyadenine, guanine, deoxyguanine, thymidine, uridine, cytosine and deoxycytosine, as well as oligomers and polymers that contain other novel nucleotides and are capable of forming hybrids with the mRNA transcripts that encode P-cadherin. These terms also include oligomers and polymers having one or more purine or pyrimidine moieties, sugar moieties, or internucleotide linkage(s) that have been chemically modified. These terms include any oligomers and polymers that are composed of nucleotides or nucleotides containing any modifications listed above which also contain bases or modified bases that are joined to sugar moieties in the alpha and not the beta configuration (known in the art as "alpha anomers") or any oligonucleotide or polynucleotide that contains one or more of these modifications. The oligonucleotides can be linear or circular and include oligomers that are modified at the 5'-end, 3'-end, or anywhere in the middle of the chain. Modifications may also involve the backbone or may occur through the nucleobases with reporter groups. These reporter groups can be lipids, phospholipids, sugarlipids, etherlipids, peptides, ligands to known or unknown receptors or any other hydrophobic moiety that can enhance or regulate the cellular uptake or the targeting of the oligonucleotide to a particular cell type. The reporter groups can also be a cross-linking group that can form covalent linkages between the oligonucleotide and the targeted mRNA with or without biological or chemical activation. The sugar-phosphate backbone can be joined by 3'-5' or 2'-5' linkages. The backbone modifications of the oligonucleotides may include those known in the art including phosphotriesters, methylphosphonates, phosphodiesters or phosphorothioates and also such backbone modifications which are based on peptides or any other non-phosphate linkages that are currently being employed or might be used by those skilled in the art. These terms also include any oligomer or polymer that has nucleosides, whether natural or containing modifications, that are joined together in linkages that are not 3'-5', such as 3'-2' phosphodiester, 5'-2' phosphodiester, or phosphorothioate linkages.

The term "downstream" is used herein to indicate the 5'-3' direction in a nucleotide sequence. Similarly, the term "upstream" indicates the 3'-5' direction.

Unless otherwise indicated, the term "mRNA" is used herein to indicate either the mature or processed messenger RNA, or the unprocessed nuclear pre-mRNA that encodes the human P-cadherin.

Antisense oligodeoxynucleotides or ribozymes have been successfully employed to decrease mRNA translation (van der Krol, et. al., 1988; Cohen, 1991; Calabretta, 1991; Calabretta, et. al., 1991; Saison-Behmoraras, et. al., 1991). Once the oligonucleotides are taken up by the cells they can elicit an antisense effect by binding to the correct sequences on the target mRNA. The concept behind antisense therapy is based on the assumption that antisense oligonucleotides are taken up by cells and interact with a specific mRNA resulting in the formation of a stable heteroduplex. The interaction of the antisense oligonucleotide with its target mRNA is highly specific and is determined by the sequence of bases complementary to the antisense oligonucleotide as determined by Watson/Crick base pairing.

Antisense oligonucleotides used for therapeutic purposes were first proposed in 1978 by M. L Stephenson and P. C. Zamecnik (PNAS 75: 280-284). The concept behind antisense therapy relies on the ability of antisense oligonucleotides to be taken up by cells and form a stable heteroduplex with the target mRNA, thereby down regulating the targeted protein's synthesis.

It has been demonstrated in a number of systems by a number of investigators that oligonucleotides containing an antisense sequence targeting a portion of a particular mRNA are capable of hybridizing to the mRNA and inhibiting the translation of the transcript.

The interaction of an antisense oligonucleotide with target mRNA is highly specific, as hybridization is determined by the sequence of bases complementary to the antisense oligonucleotide (Watson/Crick base pairing of the two strands of nucleic acid). This results in multiple points of contact between the antisense oligonucleotide and the mRNA target, which increases the specificity for hybridization to the correct sequence.

Evidence for down regulation of protein synthesis by antisense oligonucleotides has been well documented *in vitro* (for reviews see van der Krol, A. R., et al. BioTechniques 6: 958-976, 1988; Milligen et. al. J. Med. Chem 36:1923-1937, 1993). In vivo studies using antisense oligonucleotides have demonstrated that injection of radiolabeled antisense oligonucleotides into the blood of mice results in distribution of full-length labeled oligonucleotide to the various tissues. Once in the tissue, oligonucleotides can elicit an antisense effect by binding to the correct mRNA and, thus, be suitable for a therapeutic (Miller, P. S. and Ts'o, P. O. P. Anticancer Drug Design 2: 117-128, 1987).

An example of antisense alopecia therapy is known in the art. The development and progression of androgenic alopecia is associated with the local accumulation of DHT. The enzyme steroid 5α-reductase type 1 is expressed in the inner epithelial sheath of the hair follicle. This enzyme functions to catalyze the conversion of testosterone to dihydrotestosterone. U.S. Patent No. 5,994,319 teaches that antisense inhibition of steroid 5α-reductase type 1 expression, alone or in combination with other agents that decrease steroid 5α-reductase activity (i.e. Propecia™) or through the inhibition of the expression of other steroid 5α-reductase genes, is an effective means for treating androgenic alopecia.

Antisense therapy, is used according to the present invention, alone or in combination of other hair growth inhibitors or hair removers to inhibit hair growth by selectively binding to P-cadherin nucleic acids (e.g., pre-mRNA, m-RNA or gene encoding P-cadherin), thereby inhibiting P-cadherin expression and inhibiting hair growth.

Antisense oligonucleotides (at a concentration of 0.01 µg to 100 g per kg/body weight) capable of down regulating the expression of P-cadherin is administered to patients at locations where hair removal is desired in a topical application optionally containing at least one additional hair growth inhibitor or hair remover substance.

Recent evidence suggests that it is possible to deliver DNA molecules to the hair follicle by using the hair shaft appendage as an integral component of the delivery strategy (Li L, Hoffman RM. (1995) The feasibility of targeted selective gene therapy of the hair follicle.Nat Med. 1995 Jul;1(7):705-6). The formulation used for delivery can be comprised of any suitable delivery vehicle that is compatible with the physical properties of antisense oligonucleotides. For example, such agents are soluble in a solution of 60 % ethanol, propylene glycol, water and, thus, the formulation may be comprised of these components. Additionally, various liposomal formulations may be added to the delivery vehicle to promote delivery to the hair follicle.

The oligonucleotides of the present invention can be constructed and purified by methods known in the art. The specific oligonucleotide sequences are constructed so as to have a nucleotide sequence that is complementary to a nucleotide sequence that comprises a portion of the gene that encode human P-cadherin. The described sequences are most often 21 bases in length but may include as few as 3 bases, typically, at least 10, 11, 12, 13, 14, 15, 16, 17, 18, 19, 20, 21, 22, 23, 24 or at least 25-40 bases and as many as 100 bases or more. The targeted sequences have been selected because it is believed that they are essential for the translation of the P-cadherin transcript. The oligonucleotides of the present invention have been selected because they are capable of hybridizing with a high degree of specificity to regions of the transcript including the translation initiation site along with sequences 5' or 3' to the translation initiation site. Other oligonucleotides may be selected that hybridize to the 5' cap region of the mRNA or sequences 3' or 5' to the cap site. Additional oligonucleotide sequences of the present invention are complementary to sequences found in the 3' untranslated region of the P-cadherin gene and are unique to the P-cadherin gene. Such sequences are capable of hybridizing with specificity to sequences found in the 3'-untranslated region of the P-cadherin mRNA transcripts. In addition to the sequences described above, other sequences contained within the P-cadherin transcript are targeted. This strategy has been adopted because, as yet, there is no method currently available that can predict, with precision, sequences that will become effective therapeutics. Moreover, this invention further contemplates antisense oligonucleotides made complementary to any portion of the P-cadherin gene and which are capable of cross-linking DNA, intercalating DNA or binding more tightly by mechanisms such as, for example, triple stranding. Furthermore, the invention contemplates that any oligonucleotide capable of substantially inhibiting the expression of P-cadherin can be used.

Oligonucleotides of varying lengths have been successfully used to inhibit gene expression. For example, in U.S. Pat. No. 4,806,463 oligonucleotides ranging in size from 12 bases to 26 bases were shown to be incorporated by cells and to be capable of inhibiting the expression of a target mRNA.

In order for the described antisense oligonucleotides to function therapeutically, the oligonucleotides or modified oligonucleotides must be taken up by the cell that expresses the target gene, pre-mRNA, or mRNA. The oligonucleotides of the present invention are constructed so as to ensure that the oligonucleotide will pass through the plasma membrane and achieve an intracellular concentration that is sufficient to decrease the expression of P-cadherin.

Oligonucleotides that are constructed to bind to the P-cadherin gene are further modified, if necessary, to enable them to pass through the nuclear membrane in levels that are sufficient to reduce transcription. Recent attempts at enhancing the cellular uptake of antisense oligonucleotides have employed a wide variety of techniques including the use of lipoproteins, and a wide variety of conjugates, such as poly-L-lysine, polyethylene glycol and cholesterol.

Conjugation of cholesterol to the 5' end of an oligonucleotide has been reported to result in a molecule that exhibited reduced serum clearance due to reduction in renal excretion, compared to that observed with control oligodeoxynucleotides. As a result, the conjugation of cholesterol to deoxynucleotides may allow an increase in the delivery of drug to liver cells via the LDL transport mechanism. Liposomes containing antisense oligonucleotides can also be targeted to specific cell types by the addition of cell-specific antibodies. These and other methods of achieving and maintaining adequate intracellular concentrations of the oligonucleotides are contemplated by this invention and include other methods and compositions that have the capacity to enhance cellular uptake or decrease the efflux of internalized oligonucleotides. Such modifications should not alter the specificity of the oligonucleotide for its target sequence.

Antisense oligonucleotides that are intended for use as drugs must achieve sufficient concentrations in order to decrease the expression of a target protein in a manner that provides therapeutic benefit. The oligonucleotides contemplated in this invention are constructed, or otherwise modified, so as to increase their stability by enhancing resistance to various degradative enzymes (e.g., nucleases). Such modifications will function to permit the concentration of the oligonucleotide therapeutic to be maintained at a level that is sufficient so as to realize therapeutic benefit but cannot substantially alter the specificity of the oligonucleotide for its target sequence. Modifications that improve oligonucleotide stability or efficacy include but are not limited to modifications to the phosphate backbone, termini, sugar moieties and the individual nucleic acid bases. Conjugations to peptides, proteins, carbohydrates, lipids, vitamins or any other conjugation that increases therapeutic potency or efficacy can also be used. Also, any modifications resulting in stable secondary structures including circularization of the oligonucleotide and target sequence, and intrastrand joining of the 3' to the 5' termini through covalent bonds or hybridization and triple stranded binding to mRNA can also be made. Any modifications that reduce nuclease sensitivity while substantially maintaining the affinity and substrate specifically and solubility exhibited by unmodified oligonucleotides are within the scope of the invention.

Several chemically modified oligonucleotides have been developed which substantially block or improve resistance to nuclease activity. These oligonucleotide modifications include phosphorothioate oligonucleotides wherein one of the phosphate oxygens is replaced by sulfur. Another type of modification of oligonucleotides is accomplished by replacing the charged phosphate oxygen with a methyl group or other alkyl group. These nonionic DNA analogs include, for example, methyl phosphonates, alkyl-phosphorothioates, and O-alkyl phosphotriesters. A preferred O-alkylphosphotriester is O-methylphosphotriester. Other DNA backbone modifications at the phosphate group include for example, phosphorodithioate, and phosphotriester oligonucleotides or oligonucleotides based on protein-nucleic acid structures or morpholino-like structures.

Various chemical modifications to either or both the 3'- or 5'-termini and the individual nucleic acid bases are known to improve stability of oligonucleotides to nucleases, stabilize the interaction of oligonucleotides with their specific target molecule, or enhance uptake of the oligonucleotides by cells. Moreover, chemical modifications to the 3' or 5' termini or modifications internal to the oligonucleotide can also be introduced as reporter molecules for example, to allow tracking of the oligonucleotide or as lipophilic moieties to enhance cell uptake. Such molecules can be introduced to both unmodified and backbone modified synthetic oligonucleotides. These moieties can be introduced for example, through thio or amino linkages to terminal hydroxyl or phosphate groups or to specific bases.

Other modifications to the oligonucleotides contemplated in this invention include for example, DNA intercalators, photochemically activated cross-linking or cleaving agents, alkylating agents and redox active nucleic acid cleaving groups.

In vivo and in vitro studies of the degradation of chemically modified oligonucleotides have clearly illustrated that modifications to the phosphate backbone, termini, sugar moiety and individual nucleic acids improve oligonucleotide efficacy or stability or both. Moreover, acute toxicity studies in mice have demonstrated that some modified oligomers are tolerated at about the same concentrations without undesirable side effects as unmodified oligomers.

Regardless of the modifications that are contemplated by this invention, a successful antisense therapeutic that is designed to inhibit the expression of P-cadherin must hybridize with sufficient specificity so as to reduce the potential of non-mechanistic-based toxicity. Investigations into the toxicity of other antisense oligonucleotides have not revealed significant damage or lethality to cells. To date, in vitro studies examining toxicity of antisense oligonucleotides have been limited primarily to modified oligomers wherein the phosphodiester linkages between the nucleosides have been replaced with either phosphorothioates or methylphosphonates. Under the conditions tested, exposure of a variety of cell lines to phosphorothioate oligomers has not resulted in any significant toxicity.

Antisense oligonucleotides are one way of delivering antisense therapy. However, antisense gene therapy, whereby a nucleic acid construct encoding an antisense transcript is used to introduce antisense therapy into cells. Hence, according to another embodiment of the present invention the P-cadherin inhibitor is an antisense construct encoding an antisense transcript capable of specifically binding to P-cadherin gene, pre-messenger RNA or messenger RNA under physiological conditions.

On the other hand, gene therapy can also be used in accordance with the teachings of the present invention to express or overexpress P-cadherin in hair follicle cells of alopecia patients in order to induce hair growth. Hence, according to another embodiment of the present invention the P-cadherin modulator is a polynucleotide capable of directing P-cadherin expression in hair follicle cells and hence serves as a P-cadherin inducer.

Gene therapy as used herein refers to the transfer of genetic material (e.g., DNA or RNA) of interest into a host to treat or prevent a genetic or acquired disease or condition or phenotype. The genetic material of interest encodes a product (e.g., a protein, polypeptide, peptide, functional (sense) RNA, antisense RNA, ribozyme, etc.) whose production *in vivo* is desired. For example, the genetic material of interest can encode a P-cadherin protein, a peptide capable of binding P-cadherin and modulate its function, a functional (sense) P-cadherin RNA, antisense P-cadherin RNA, P-cadherin ribozyme, etc. For review see, in general, the text "Gene Therapy" (Advanced in Pharmacology 40, Academic Press, 1997). In *vivo* gene therapy (as opposed to ex vivo gene therapy), the genetic material to be transferred into the cells is introduced into the cells of the recipient organism *in situ*, that is within the recipient. In an alternative embodiment, if the host gene is defective, the gene is *repaired in situ* (Culver, 1998. (Abstract) Antisense DNA & RNA based therapeutics, February 1998, Coronado, CA). These genetically altered cells have been shown to express the transfected genetic material *in situ.*

The gene expression vehicle is capable of delivery/transfer of heterologous nucleic acid into a host cell. The expression vehicle may include elements to control targeting, expression and transcription of the nucleic acid in a cell selective manner as is known in the art. It should be noted that often the 5'UTR and/or 3'UTR of the gene may be replaced by the 5'UTR and/or 3'UTR of the expression vehicle. Therefore, as used herein the expression vehicle may, as needed, not include the 5'UTR and/or 3'UTR of the actual gene to be transferred and only include the specific amino acid coding region.

The expression vehicle can include a promoter for controlling transcription of the heterologous material and can be either a constitutive or inducible promoter to allow selective transcription. Enhancers that may be required to obtain necessary transcription levels can optionally be included. Enhancers are generally any nontranslated DNA sequence which works contiguously with the coding sequence (in cis) to change the basal transcription level dictated by the promoter. The expression vehicle can also include a selection gene as described herein below.

Vectors can be introduced into cells or tissues by any one of a variety of known methods within the art. Such methods can be found generally described in Sambrook *et al*., Molecular Cloning: A Laboratory Manual, Cold Springs Harbor Laboratory, New York 1989, 1992), in Ausubel *et al*., Current Protocols in Molecular Biology, John Wiley and Sons, Baltimore, Maryland 1989), Chang *et al*., Somatic Gene Therapy, CRC Press, Ann Arbor, MI 1995), Vega *et al*., Gene Targeting, CRC Press, Ann Arbor MI (995), Vectors: A Survey of Molecular Cloning Vectors and Their Uses, Butterworths, Boston MA 1988) and Gilboa *et al*. (Biotechniques 4 (6): 504-512, 1986) and include, for example, stable or transient transfection, lipofection, electroporation and infection with recombinant viral vectors.

Introduction of nucleic acids by infection offers several advantages over the other listed methods. Higher efficiency can be obtained due to their infectious nature. Moreover, viruses are very specialized and typically infect and propagate in specific cell types. Thus, their natural specificity can be used to target the vectors to specific cell types *in vivo.* Viral vectors can also be modified with specific receptors or ligands to alter target specificity through receptor mediated events.

A specific example of DNA viral vector introducing and expressing recombination sequences is the adenovirus-derived vector Adenop53TK. This vector expresses a herpes virus thymidine kinase (TK) gene for either positive or negative selection and an expression cassette for desired recombinant sequences. This vector can be used to infect cells that have an adenovirus receptor which includes cells of epithelial origin as well as others. This vector as well as others that exhibit similar desired functions can be used to treat a mixed population of cells and can include, for example, a tissue, e.g., skin tissue, or a human subject.

Features that limit expression to particular cell types can also be included. Such features include, for example, promoter and regulatory elements that are specific for the desired cell type. The P-cadherin promoter can be used to direct gene expression in hair follicle cells.

In addition, recombinant viral vectors are useful for *in vivo* expression of a desired nucleic acid because they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

As described above, viruses are very specialized infectious agents that have evolved, in may cases, to elude host defense mechanisms. Typically, viruses infect and propagate in specific cell types. The targeting specificity of viral utilizes its natural specificity of viral vectors utilizes its natural specificity to specifically target predetermined cell types and thereby introduce a recombinant gene into the infected cell. The vector to be used in the methods of the invention will depend on desired cell type to be targeted and will be known to those skilled in the art.

Retroviral vectors can be constructed to function either as infectious particles or to undergo only a single initial round of infection. In the former case, the genome of the virus is modified so that it maintains all the necessary genes, regulatory sequences and packaging signals to synthesize new viral proteins and RNA. Once these molecules are synthesized, the host cell packages the RNA into new viral particles which are capable of undergoing further rounds of infection. The vector's genome is also engineered to encode and express the desired recombinant gene. In the case of non-infectious viral vectors, the vector genome is usually mutated to destroy the viral packaging signal that is required to encapsulate the RNA into viral particles. Without such a signal, any particles that are formed will not contain a genome and therefore cannot proceed through subsequent rounds of infection. The specific type of vector will depend upon the intended application. The actual vectors are also known and readily available within the art or can be constructed by one skilled in the art using well-known methodology.

The recombinant vector can be administered in several ways. If viral vectors are used, for example, the procedure can take advantage of their target specificity and consequently, do not have to be administered locally at the diseased site. However, local administration can provide a quicker and more effective treatment, administration can also be performed by, for example, intravenous or subcutaneous injection into the subject.

According to another embodiment of the present invention, the P-cadherin modulator, or the molecule capable of binding P-cadherin, is an anti-P-cadherin antibody and hence serves as a P-cadherin inhibitor.

Figures 4a-d shows an alignment of the intracellular and extracellular portions of human cadherins. Short sequences of low similarity between P-cadherin and the other human cadherins, especially E-cadherin, were identified. These sequences are used in accordance with the teachings of the present invention to generate antibodies specific to P-cadherin.

The following peptides are thought to have a potential of eliciting antibodies specific to P-cadherin as they share low or no similarity with corresponding sequences of other human cadherins and/or mouse cadherins and were identified as immunogenic by the peptidestructure algorithm from the GCG package:

For the extracellular domain of P-cadherin:
1. VPENGKGPFP (117-124) (SEQ ID NO:40) both immunogenic and not homologous to either mouse P-cadherin or other human cadherins;
2. QEPKDPHDLMFTIHRSTGT (259-277)(SEQ ID NO:41);
3. DNGSPPTTGT (522-531)(SEQ ID NO:42);
4. TDKDLSPHTSPFQAQLTDDSDIY(568-590)(SEQ ID NO:43);
5. DCHGHVETCPGPWKGG (639-654)(SEQ ID NO:44);

For the cytoplasmic domain of P-cadherin:
6. MYRPRPANPDEI(743-754)(SEQ ID NO:45)

These or similar peptides are used according to the present invention to elicit P-cadherin specific antibodies which are used for inhibiting hair growth by topical application onto the skin in a formulation that enhances the penetration of such antibodies into cells of the hair follicle.

As used herein, the term "antibody" includes any monoclonal or polyclonal immunoglobulin, or a fragment of an immunoglobin such as sFv (single chain antigen binding protein), Fab1 or Fab2. The immunoglobulin could also be a "humanized", in which murine variable regions are fused to human constant regions, or in which murine complementarity-determining regions are grafted onto a human antibody structure (Wilder, R.B. et al., J. Clin. Oncol., 14:1383-1400, 1996). Unlike mouse or rabbit antibodies, "humanized" antibodies often do not undergo an undesirable reaction with the immune system of the subject. The terms "sFv" and "single chain antigen binding protein" refer to a type of a fragment of an immunoglobulin, an example of which is sFv CC49 (Larson, S.M. et al., Cancer, 80:2458-68, 1997).

The elicitation of an anti-P-cadherin antibody is through *in vivo* or *in vitro* techniques, the antibody having been prepared by a process comprising the steps of (a) exposing cells capable of producing antibodies to P-cadherin or an immonological part thereof (e.g., a peptide fragment or synthetic peptide derived therefrom) and thereby generating antibody producing cells; (b) immortalizing the antibody producing cells by, for example, either fusing the antibody producing cells with myeloma cells or infecting the antibody producing cells with an immortalizing (transforming) virus and thereby generating a plurality of immortalized (e.g., transformed or hybridoma) cells each producing a monoclonal antibody; and (c) screening a plurality of monoclonal antibodies to identify a monoclonal antibody which specifically binds P-cadherin.

The cDNA encoding the monoclonal antibody can then be isolated by conventional techniques (e.g., screening a cDNA library with a probe that hybridizes to the portion encoding the constant region of the antibody). Portions of the cDNA encoding the variable regions of the antibody can be fused in-frame to other polypeptides such as the constant region of an antibody derived from a human being, to thereby obtain a humanized single chain antibody.

In another approach a phage display library presenting variable regions of antibodies fused to one or more of their coat proteins is enriched for those phages presenting antibodies that bind P-cadherin. Individual phage clones are then isolated and their genetic material sequenced to determine the amino acid sequence of the antibody they display. Then, a corresponding peptide is synthesized using solid phase techniques and tested for binding P-cadherin. General protocols for antibody-phage display technology are available from the Pharmacia Biotech (Uppsala, Sweden) Recombinant Phage Antibody System (RPAS).

Methods of generating, screening and characterizing the specificity of binding of an antibody are well known in the art. Further insight on these topics is available in, for example, "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521.

Antibodies that are constructed to bind to P-cadherin may be further modified, if necessary, to enable them to pass through the cell membrane in levels that are sufficient to reduce P-cadherin function. Recent attempts at enhancing the cellular uptake of antibodies have employed a wide variety of techniques including the use of lipoproteins, polyethylene glycol and cholesterol. Liposomes containing antibodies can also be targeted to specific cell types by the addition of cell-specific antibodies on the outside of the liposome structure. These and other methods of achieving and maintaining adequate intracellular concentrations of the antibodies are contemplated by this invention and include other methods and compositions that have the capacity to enhance cellular uptake or decrease the efflux of internalized antibodies. Such modifications should not alter the specificity of the antibody for its target protein.

The present invention further contemplates the use of low molecular weight (e.g., up to 1,500 Da) organic compounds as either P-cadherin inhibitors or inducers as hair growth inducers or inhibitors, respectively. Chemical libraries of hundred of thousands of low molecular weight organic compounds are presently available on the market for use in highthroughput binding/screening assays. Such libraries can be screened for ligands that bind P-cadherin and modulate P-cadherin function. Such ligands can thereafter be tested *in vivo* to determine their effect on hair growth. Following the identification of a ligand as binding to P-cadherin, tests are conducted to establish whether it also modulates P-cadherin function (e.g., binding to β-catenin or other cellular skeleton components) and thereafter tests are conducted to establish whether it also modulates hair growth. Structure optimization and retesting are thereafter practiced to increase modulation activity. During structure optimization advantage can be taken of the 3D structure of P-cadherin. Similarly, rational drug design can take advantage of the 3D structure of P-cadherin.

Yet another type of candidate P-cadherin modulators are peptides. The present invention contemplates the use of a two hybrid system to identify peptides that specifically bind P-cadherin.

One approach for elucidating protein-protein binding in cells is the yeast-based two-hybrid system (Fields and Song (1989) Nature 340:245). That system utilizes chimeric genes and detects protein-protein interactions via the activation of reporter-gene expression. Reporter-gene expression occurs as a result of reconstitution of a functional transcription factor caused by the association of fusion proteins encoded by the chimeric genes. Typically, polynucleotides encoding two-hybrid proteins are constructed and introduced into a yeast host cell. The first hybrid protein consists of the yeast Gal4 DNA-binding domain fused to a polypeptide sequence of a known protein (often referred to as the "bait"). The second hybrid protein consists of the Gal4 activation domain fused to a polypeptide sequence of a second protein (often referred to as the "prey"). Binding between the two-hybrid proteins reconstitutes the Gal4 DNA-binding domain with the Gal4 activation domain, which leads to the transcriptional activation of a reporter gene (e.g., lacZ or HIS3), which is operably linked to a Gal4 binding site.

Homo- and heterodimeric protein complexes mediate many cellular processes and abnormal protein interactions underlie various medical conditions. Yan et al. (1995) Cancer-Res. 55: 3569-75. Research on such complexes has led to efforts to understand disease at the molecular level and to a search for small molecule effectors of such complexes. Such effectors could modulate protein interactions and are potential therapeutic agents. Gibbs & Oliff (1994) Cell 79: 193-198. Most often, such effectors have been identified using various biochemical and immunological in vitro approaches. The advantages of genetic approaches in drug discovery, however, have received increased attention. Liuzzi et al. (1994), Nature 372: 695-8. These advantages include both cost-effectiveness and simplicity. Several such genetic systems, in particular the yeast-two hybrid system, meets all these criteria and is also equally suitable for the detection of both homo- and heterodimeric protein interactions. Another unique feature of the yeast two-hybrid system is its ability to detect the desired protein-protein interaction without interference by competing interactions. Fields & Song (1989) Nature 340: 245-6. The system has been successfully used for the analysis of protein interactions and for the isolation of interacting proteins through interaction cloning. For a review, see Allen et al. (1995), Trends in Biochem. Sci. 20: 511-16.

Prokaryote two-hybrid systems are also available. E. coli strains can be hyperpermeable. Nakamura & Suganuma (1972) J. Bacteriol. 110: 329-35. One can use this hyperpermeability to maximize the number of small molecules that can be evaluated. In addition, E. coli has a rapid growth rate, permitting shorter turnaround times during drug screening. Furthermore, one can transform E. coli at high frequencies, facilitating interaction cloning. U.S. Patent No. 6,051,381, teaches a prokaryote two-hybrid system. U.S. Patent No. 6,251,676, teaches a mammalian two-hybrid system. Both of which are incorporated herein by reference.

In another approach a phage display library presenting short peptides (e.g., 6-8 amino acids) fused to one or more of the phage's coat proteins is enriched for those phages presenting peptides that bind P-cadherin. Individual phage clones are then isolated and their genetic material sequenced to determine the amino acid sequence of the short peptide they display. Then, a corresponding peptide is synthesized using solid phase techniques and tested for binding P-cadherin. Further insight regarding phage display libraries, their enrichment and screening is present in, for example, Frenkel and Solomon, J. of Neuroimmunol. 88:85-90, 1998.

A peptide that binds P-cadherin can be an inhibitor or inducer of its activity. Once this is established, such a peptide is tested for hair growth modulation.

As used herein in the specification and in the claims section below the term "peptide" includes native peptides (either degradation products, synthetically synthesized peptides or recombinant peptides) and peptido-mimetics (typically, synthetically synthesized peptides), such as peptoids and semipeptoids which are peptide analogs, which may have, for example, modifications rendering the peptides more stable while in a body, or more immunogenic. Such modifications include, but are not limited to, cyclization, N terminus modification, C terminus modification, peptide bond modification, including, but not limited to, CH₂-NH, CH₂-S, CH₂-S=O, O=C-NH, CH₂-O, CH₂-CH₂, S=C-NH, CH=CH or CF=CH, backbone modification and residue modification. Methods for preparing peptido-mimetic compounds are well known in the art and are specified, for example, in Quantitative Drug Design, C.A. Ramsden Gd., Chapter 17.2, F. Choplin Pergamon Press (1992), which is incorporated by reference as if fully set forth herein. Further detail in this respect are provided hereinunder.

Thus, a peptide according to the present invention can be a cyclic peptide. Cyclization can be obtained, for example, through amide bond formation, e.g., by incorporating Glu, Asp, Lys, Orn, di-amino butyric (Dab) acid, di-aminopropionic (Dap) acid at various positions in the chain (-CO-NH or -NH-CO bonds). Backbone to backbone cyclization can also be obtained through incorporation of modified amino acids of the formulas H-N((CH₂)ₙ-COOH)-C(R)H-COOH or H-N((CH₂)ₙ-COOH)-C(R)H-NH₂, wherein n = 1-4, and further wherein R is any natural or non-natural side chain of an amino acid.

Cyclization via formation of S-S bonds through incorporation of two Cys residues is also possible. Additional side-chain to side chain cyclization can be obtained via formation of an interaction bond of the formula -(-CH₂-)ₙ-S-CH₂-C-, wherein n = 1 or 2, which is possible, for example, through incorporation of Cys or homoCys and reaction of its free SH group with, e.g., bromoacetylated Lys, Om, Dab or Dap.

Peptide bonds (-CO-NH-) within the peptide may be substituted, for example, by N-methylated bonds (-N(CH₃)-CO-), ester bonds (-C(R)H-C-O-O-C(R)-N-), ketomethylen bonds (-CO-CH₂-), α-aza bonds (-NH-N(R)-CO-), wherein R is any alkyl, e.g., methyl, carba bonds (-CH₂-NH-), hydroxyethylene bonds (-CH(OH)-CH₂-), thioamide bonds (-CS-NH-), olefinic double bonds (-CH=CH-), retro amide bonds (-NH-CO-), peptide derivatives (-N(R)-CH₂-CO-), wherein R is the "normal" side chain, naturally presented on the carbon atom.

These modifications can occur at any of the bonds along the peptide chain and even at several (2-3) at the same time.

Natural aromatic amino acids, Trp, Tyr and Phe, may be substituted for synthetic non-natural acid such as TIC, naphthylelanine (Nol), ring-methylated derivatives of Phe, halogenated derivatives of Phe or o-methyl-Tyr.

Tables 1-2 below list all the naturally occurring amino acids (Table 1) and non-conventional or modified amino acids (Table 2).

**TABLE 1**

| Amino Acid | Three-Letter Abbreviation | One-letter Symbol |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic Acid | Glu | E |
| Glycine | Gly | G |
| Histidine | His | H |
| Isoleucine | Iie | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any amino acid as above | Xaa | X |

**TABLE 2**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgin |
| carboxylate | | L-N-methylglutamic acid | Nmglu |
| cyclohexylalanine | Chexa | L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcyclopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N- amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cyclododeclglycine | Ncdod |
| D-α-methylalnine | Dnmala | N-cyclooctylglycine | Ncoct |
| D-α-methylarginine | Dnmarg | N-cyclopropylglycine | Ncpro |
| D-α-methylasparagine | Dnmasn | N-cycloundecylglycine | Ncund |
| D-α-methylasparatate | Dnmasp | N-(2,2-diphenylethyl)glycine | Nbhm |
| D-α-methylcysteine | Dnmcys | N-(3,3-diphenylpropyl)glycine | Nbhe |
| D-N-methylleucine | Dmnleu | N-(3-indolylyethyl) glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nva |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(p-hydroxyphenyl)glycine | Nhtyr |
| L-t-butylglycine | Thug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-t-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomo phenylalanine | Mhphe |
| L-α-methylisoleucine | Mile | N-(2-methylthioethyl)glycine | Nmet |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl)glycine | Nser |
| D-N-methylisoleucine | Dnmile | N-(imidazolylethyl)glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmom | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(p-hydroxyphenyl)glycine | Nhtyr |
| L-t-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-t-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Mom |
| L-α-methylphenylalanine | Mphe | L-α-methylproline | Mpro |
| L-α-methylserine | mser | L-α-methylthreonine | Mthr |
| L-α-methylvaline | Mtrp | L-α-methyltyrosine | Mtyr |
| L-α-methylleucine | Mval Nnbhm | L-N-methylhomophenylalanine | Nmhphe |
| N-(N-(2,2-diphenylethyl) | | N-(N-(3,3-diphenylpropyl) | |
| carbamylmethyl-glycine | Nnbhm | carbamylmethyl(1)glycine | Nnbhe |
| 1-carboxy-1-(2,2-diphenyl ethylamino)cyclopropane | Nmbc | | |

A peptide according to the present invention can be used in a self standing form or be a part of a larger moiety such as a protein or a display moiety such as a display bacterium, a display phage or a display cell.

A peptide according to the present invention includes at least five, optionally at least six, optionally at least seven, optionally at least eight, optionally at least nine, optionally at least ten, optionally at least eleven, optionally at least twelve, optionally at least thirteen, optionally at least fourteen, optionally at least fifteen, optionally at least sixteen or optionally at least seventeen, optionally between seventeen and twenty five or optionally between twenty five and at least thirty amino acid residues (also referred to herein interchangeably as amino acids).

Accordingly, as used herein the term "amino acid" or "amino acids" is understood to include the 20 naturally occurring amino acids; those amino acids often modified post-translationally *in vivo*, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

According to an additional aspect of the present invention there is provided a hair growth modulator identified by the methods described herein.

According to yet an additional aspect of the present invention there is provided a method of modulating hair growth comprising administering to a subject in need a therapeutically effective amount of the hair growth modulator described herein.

A compound (active ingredient) according to the present invention can be administered to an organism, such as a human being or any other mammal, *per se*, or in a pharmaceutical composition where it is mixed with suitable carriers or excipients.

As used herein a "pharmaceutical composition" refers to a preparation of one or more of the compounds described herein, or physiologically acceptable salts or prodrugs thereof, with other chemical components such as physiologically suitable carriers and excipients. The purpose of a pharmaceutical composition is to facilitate administration of a compound to an organism. In particular, the purpose of a pharmaceutical composition in accordance with the present invention is to facilitate administration of a compound to the skin organism, specifically to hair follicles.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

Pharmaceutical compositions may also include one or more additional active ingredients, such as, but not limited to, anti inflammatory agents, antimicrobial agents, vitamins, anesthetics and the like in addition to the compounds described herein.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active compounds into preparations which, can be used pharmaceutically.

The pharmaceutical compositions herein described may comprise suitable solid of gel phase carriers or excipients. Examples of such carriers or excipients include, but are not limited to, calcium carbonate, calcium phosphate, various sugars, starches, cellulose derivatives, gelatin and polymers such as polyethylene glycols.

Pharmaceutical compositions suitable for use in context of the present invention include compositions wherein the active ingredients are contained in an amount effective to achieve the intended purpose. More specifically, a therapeutically effective amount means an amount of active ingredient effective in modulating hair growth of the subject being treated.

Determination of a therapeutically effective amount is well within the capability of those skilled in the art, especially in light of the detailed disclosure provided herein.

Toxicity and therapeutic efficacy of the compounds described herein can be determined by standard pharmaceutical procedures in cell cultures or experimental animals, e.g., by determining the IC₅₀ and the LD₅₀ (lethal dose causing death in 50 % of the tested animals) for a subject compound. The data obtained from these cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Depending on the severity and responsiveness of the condition to be treated, dosing can also be a single administration of a slow release composition using for example skin patches, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

The present invention can be used to treat any one of a plurality of diseases, disorders or conditions associated with modulation of hair growth.

A skin absorption enhancer can be used in a composition of the present invention. Skin absorption enhancer include, for example, khellin, methyl nicotinate, MSM-Decy methyl sulfoxide, diethylene glycol, citric acid, pyruvic acid, phenoxyethanol, transcutol, GEMTEK surfactant, phosphatidyl choline, MCT oil and water.

The following Table 3 provides a range of concentrations of ingredients that may be used in the skin absorption enhancer.

**TABLE 3**

| SKIN ABSORTION ENHANCER | Weight % |
|---|---|
| Khellin | 0-10 |
| Methyl nicotinate | 0-20 |
| Decy methyl sulfoxide | 0-60 |
| Diethylene glycol | 0-90 |
| Citric acid | 0-45 |
| Pyruvic acid | 0-45 |
| Phenoxyethanol | 0-85 |
| Transcutol | 0-90 |
| GEMTEK surfactant | 0-20 |
| Phosphatidyl choline | 0-10 |
| MCT oil | 0-30 |
| Water | 0-80 |

The above ingredients are shown in weight percent, and are available from commercial suppliers such as Brooks, Sigma (St. Louis, Mo.) and Aldrich (Milwaukee, Wis.).

The following Table 4 provides a preferred formulation of the skin absorption enhancer.

**TABLE 4**

| SKIN ABSORTION ENHANCER | Weight % |
|---|---|
| Khellin | 0.1 |
| Methyl nicotinate | 0.2 |
| MSM-Decy methyl sulfoxide | 2 |
| Diethylene glycol | 4 |
| Citric acid | 4 |
| Pyruvic acid | 2 |
| Phenoxyethanol | 6 |
| Transcutol | 4.7 |
| GEMTEK surfactant | 0.25 |
| Phosphatidyl choline | 0.1 |
| MCT oil | 2 |
| Water | 74.65 |

The above ingredients are shown in weight percent, and are available from commercial suppliers such as Brooks, Sigma (St. Louis, Mo.) and Aldrich (Milwaukee, Wis.).

In the method of the present invention, for modulating hair growth, the following steps are performed preferably in the order noted: (i) cleansing the scalp or other body portion treated with a cleansing agent; (ii) optionally, treating the cleansed scalp or body portion with a keratin solvent system; (iii) optionally, applying a topical anesthetic; (iv) optionally, applying an acid peel solution; (v) optionally, applying a hyperactive urea gel formula and (vi) applying a hair growth modulating composition.

When the hair growth modulating composition includes a hair growth inducer, treatment can be applied to individuals with, for example, alopecia androgenetica, alopecia totalis, alopecia universalis and alopecia areata.

When the hair growth modulating composition includes a hair growth inhibitor, treatment can be applied to individuals with, for example, excessive hair growth, such as in hirsutism or for cosmetic purposes.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### Demonstration of the role of P-cadherin in hair follicle morphogenesis

Four large consanguineous HJMD families with 11 affected individuals were selected for this study. All families originated from a small region of Northern Israel and belonged to the Druze population, a religious minority of Muslim origin, living in mountainous areas of the Middle East as a closed society almost from its inception in Cairo around 1017 A. C. (Qumsiyeh, M.B., Dasouki M.J. & Teebi, A.S. In: Genetic disorders among Arab populations, Teebi, A.S. & Farag, T.I. eds., p.232, Oxford University Press, Oxford (1997)). Affected individuals were born with normal-appearing hair but developed alopecia of the scalp at about 3 months of age. During puberty, however, partial regrowth of short and sparse hair occurred (Fig. la). Histological examination of scalp skin biopsies showed normal findings except for a reduced ratio of terminal vs. vellus hair follicles while distinct structural aberrations of the hair shafts were evident by light and scanning electron microscopic examinations (Fig. 1b-c). Between the age of 3 and 21 years, affected individuals developed progressive macular degeneration with slight peripheral retinal dystrophy (Fig. 1d). Electrophysiological evaluation of the visual system disclosed anomalies consistent with impaired macular function (Fig. 1e).

With informed consent of all participants, DNA was obtained from peripheral blood samples for molecular studies. To map the HJMD gene, a genome wide scan was performed by genotyping 202 fluorescently-labeled microsatellite markers (Research Genetics). Consanguinity of the families enabled to apply homozygosity mapping to identify a 20 cM segment on chromosome 16q22.1 identical by descent in affected individuals of families 1-3. Subsequent haplotype analysis and multipoint linkage analysis (HOMOZ software, Kruglyak, L., Daly, M.J. & Lander, E. S. *Am. J. Hum. Genet.* **56**, 519-527 (1995)) using 5 additional polymorphic markers in all members of the 4 families further refined the disease gene locus to a 5 cM interval flanked by D16S3085 and D16S3066 (Fig. 2a) with a maximum Iod score of 10.4 at marker D16S3025.

Three contigs were identified in the unfinished High Throughput Genomic Sequences (htgs) database that contained at least one of the 4 microsatellite markers flanking or located within the HJMD critical interval. Together these contigs harbored at least 45 different genes, including CDH3 encoding P-cadherin. Following are the Genbank accession numbers of contigs within the critical disease interval: NT_010478; NT_024792; NT_010556; CDH3 cDNA: NM_001793.

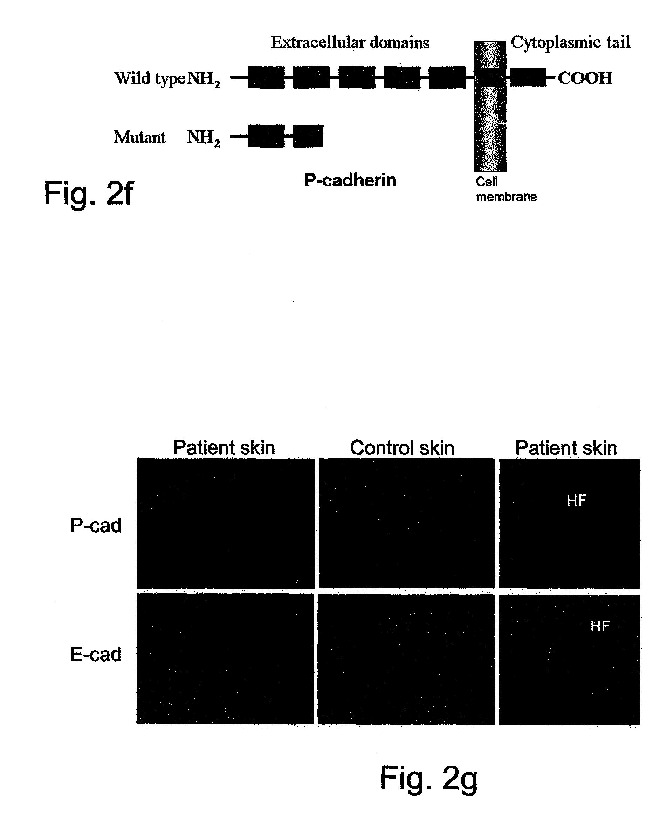

Classical cadherins are thought to be involved in the regulation of hair (Fukumi, F. et al. Microsc. Res. Tech. 38, 343-352 (1997); Muller-Rover, S. et al. Exp. Dermatol. 8, 237-246 (1999)) as well as retinal (Riehl, R. et al. Neuron 17, 837-848 (1996)) development. CDH3 spans 55.45 kb, comprises 16 exons and is part of a cluster of cadherin genes located on 16q (Kremmidiotis, G., Baker, E., Crawford, J., Eyre, H.J., Nahmias, J. & Callen, D.F. *Genomics* 49, 467-471 (1998)). The organization of P-cadherin conforms to the general structure of classical cadherins with 5 extracellular domains, a transmembrane region and a short intracellular tail (Yagi, T. & Takeishi, M. Genes Dev. 14, 1169-1180 (2000)) (Fig. 2f).

The entire coding region of CDH3 was PCR-amplified and directly sequenced, including exon-intron boundaries, in one affected individual. The following primer pairs (presented in a 5' to 3' orientation) were employed:

CDH3/16F CTTGGAGATGCTCT GTGGC (SEQ ID NO:46)

CDH3/16R    GCACTTGCTGTCTGCTGGTC (SEQ ID NO:47)

CDH3/15F CATGC TTGTTCTCCT GTGTG (SEQ ID NO:48)

CDH3/15R CTGTGACATCATCTGTCTTG (SEQ ID NO:49)

CDH3/14F CAAAGAGACTACAGCAATGGAC (SEQ ID NO:50)

CDH3/14R CTGAGTGAGGACATCTGCAG (SEQ ID NO:51)

CDH3/13F CTGGGTGACAGAGTGAGAC (SEQ ID NO:52)

CDH3/13R    CTTCATGGTGTACTCAGATC (SEQ ID NO:53)

CDH3/12F GGTTCTAGAGGAGATCATTGTC (SEQ ID NO:54)

CDH3/12R GTCTTGAGAGGTGAGAGCTG (SEQ ID NO:55)

CDH3/11F GCATGAGCCACTGCATCCAG (SEQ ID NO:56)

CDH3/11R GCCCTGAATGATGACATCAG (SEQ ID NO:57)

CDH3/10F CAATCTCTATGGTAATCAGAAC (SEQ ID NO:58)

CDH3/10R    CATCTCAACTGTCCTGCACAG (SEQ ID NO:59)

CDH3/9F CAGTGACTCTTACCTATTTATG (SEQ ID NO:60)

CDH3/9R CATCCTGCCGCTGTGTATAC (SEQ ID NO:61)

CDH3/8F CAGCCATAGTGCTGAGACTG (SEQ ID NO:62)

CDH3/8R CACCCATGAGCCAGTGCTTC (SEQ ID NO:63)

CDH3/7F GCTTCTGCTCTCAGAGTCAG (SEQ ID NO:64)

CDH3/7R GTAGACAGGGCTGGAGTTG (SEQ ID NO:65)

CDH3/5+6F  CAGAGCTCTGCTCTAGGATC (SEQ ID NO:66)

CDH3/5+6R CTGTTCAGTGAGCAGATTCTC (SEQ ID NO:67)

CDH3/4F CAGTAGCAAGAAATCTCATGC (SEQ ID NO:68)

CDH3/4R CAATAGGCTCATCTAGGTCTC (SEQ ID NO:69)

CDH3/3F GACTAACACTACCTCCTCTG (SEQ ID NO:70)

CDH3/3R GTCCATGAATGTCTATGATC (SEQ ID NO:71)

CDH3/2F GATGTCATAGGCGCTCTGCTG (SEQ ID NO:72)

CDH3/2R GTCGCGGCAGCTGCTTCAC (SEQ ID NO:73)

CDH3/1F GCAGAGAGTGAAGGAGGCTG (SEQ ID NO:74)

CDH3/1R GTACTGAGGAGGCTGAGGAG (SEQ ID NO:75)

PCR conditions were optimized for each primer pair.

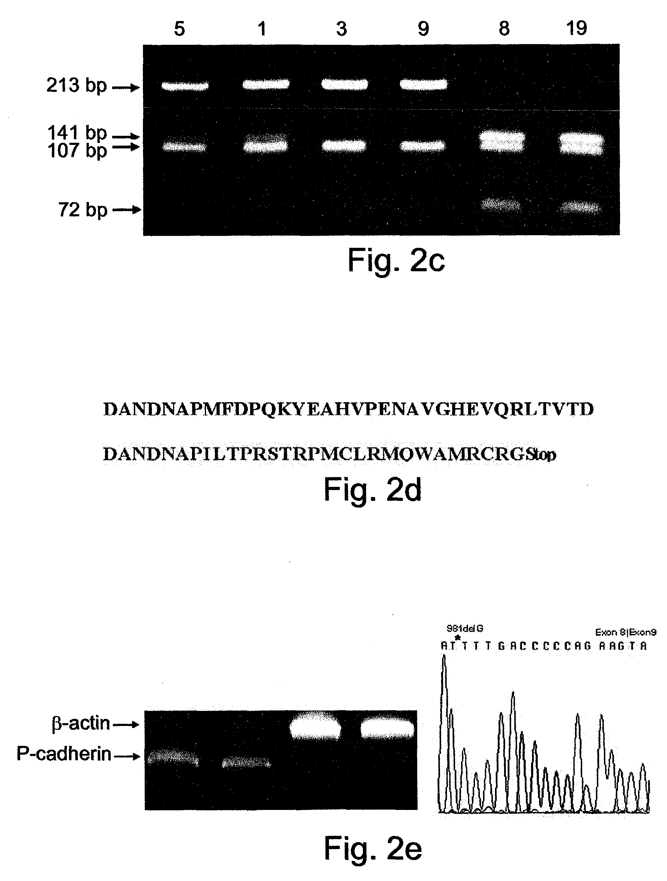

A homozygous deletion of a guanine nucleotide was identified in exon 8 at position 981 from the translation start site (ATG) of CDH3 (Fig. 2b). The 981delG mutation abolishes a recognition site for NlaIII (Fig. 2c) and is predicted to result in a frameshift that introduces a premature termination codon 23 residues downstream of the mutation site (Fig. 2d). Using direct DNA sequencing and restriction fragment analysis, it was determined that all affected individuals were homozygous for the 981delG mutation, and that their parents were carriers of the mutant allele. In contrast, the mutation was not found in a pool of 248 chromosomes of healthy unrelated Druze, Arab-Israeli and Caucasians individuals, excluding the possibility that the 981delG mutation represents a non-consequential polymorphism. Affected individuals also shared an ancestral haplotype for markers D16S3085, D16S3025 and D16S2624 (Fig. 2a), although a genealogical relationship could only be defined between families 2 and 3. These results strongly suggest a founder effect for 981delG in the Druze population.

To study the consequences of the 981delG mutation, a skin biopsy was obtained from a homozygous HJMD patient. The level of CDH3 mRNA expression determined by semi-quantitative RT-PCR was equivalent to that of a normal control sample suggesting either absence of nonsense-mediated RNA decay (Frischmeyer, P.A. & Dietz, H.C. *Hum. Mol. Genet.* 8, 1893-1900 (1999)) or RNA decay with compensatory overexpression of CDH3 (Fig. 2e). Direct sequence analysis of RT-PCR products confirmed the presence of the CDH3 mutation in the patient's cDNA and did not provide evidence for exon skipping (Fig. 2e). The 981delG mutation is predicted to result in translation of a truncated protein lacking its cytoplasmic tail and 3 out of 5 extracellular domains (Fig. 2f). P-cadherin membranal expression was assessed by immunofluorescence staining and shown to be markedly reduced in patient skin biopsies (Fig. 2g), suggesting either protein degradation or loss of antigenic epitope.

These results indicate that HJMD is caused by the loss of P-cadherin function due to a frameshift mutation in CDH3. P-cadherin expression has been demonstrated in the retinal pigment epithelium (Burke, J.M., Cao, F., Irving, P.E. & Skumatz, C.M. Invest. Ophthalmol. Vis. Sci. 40, 2963-2970 (1999)), although the exact role of P-cadherin in retina development remains elusive. Interestingly, two other forms of retinal dystrophy (Usher syndromes type 1D and IF) have been shown to result from mutations in unrelated cadherin genes (Ahmed, Z.M. et al. Am. J. Hum. Genet. 69, 25-34 (2001); Bolz, H. et al. Nature Genet. 27, 108-112 (2001)). In the hair follicle, P-cadherin (but not E-cadherin) is expressed in a subset of epithelial cells involved in hair shaft growth regulation (Muller-Rover, S. et al. Exp. Dermatol. 8, 237-246 (1999)), an observation which may help understanding the peculiar HJMD phenotype. In contrast, most other epithelia co-express both P-cadherin and E-cadherin, and the latter might be able to compensate, at least in part, for P-cadherin deficiency in epidermal cells (Lewis, J.E., Jensen, P.J. & Wheelock, M.J J. Invest. Dermatol. 102, 870-877 (1994)), thus explaining the absence of skin phenotype in HJMD patients. Some form of functional redundancy may also explain the characteristic regrowth of hair in HJMD patients during puberty. Indeed gene expression of various cadherins and cadherin-related proteins, such as E-cadherin (Chen, G.T., Getsios, S. & MacCalman, C.D. Endocrine 9, 263-267 (1998))¹⁶ and β-catenin (Monks, D.A., Getsios, S., MacCalman, C.D. & Watson, N.V. Proc. Natl. Acad. Sci. U.S.A. 98, 1312-1316 (2001)), has been shown to be controlled by sex hormones. It is of interest to note that loss of P-cadherin in mice does not result in obvious hair or ophthalmological abnormalities (Radice, G.L. et al. J. Cell Biol. 139, 1025-1032 (1997)). Such phenotypic discrepancies between mice and humans carrying mutations in orthologous genes are not uncommon: mutations in another cadherin gene, PCDH15, cause retinitis pigmentosa in humans but not in mice (Ahmed, Z.M. et al. Am. J. Hum. Genet. 69, 25-34 (2001)), and humans, but not mice, carrying recessive mutations in GJB3 display severe deafness (Plum, A. et al. Dev. Biol. 231, 334-347 (2001)).

Classical cadherins maintain cell-cell adhesion at adherens junctions through Ca⁺²-dependant homophilic interactions (Yagi, T. & Takeishi, M. Genes Dev. 14, 1169-1180 (2000)). β-catenin physically links the actin cytoskeleton to the cytoplasmic tail of P-cadherin (Yagi, T. & Takeishi, M. Genes Dev. 14, 1169-1180 (2000)), which is truncated as a result of the 981delG mutation. Since β-catenin was shown to control hair follicle mophogenesis (Huelsken, J., Vogel, R., Erdmann, B., Cotsarelis, G. & Birchmeier, W. Cell 105, 533-545 (2001)) and since constitutive expression of the β-catenin gene in mice leads to exuberant hair growth (Gat, U., DasGupta, R., Degenstein, L. & Fuchs, E. Cell 95, 605-614 (1998)), abnormal interactions between β-catenin and non-functional P-cadherin might play a pivotal role in the pathogenesis of HJMD.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

The following numbered claims are not claims but summarise aspects of the invention.
1. A method of identifying a hair growth modulator comprising:
   identifying a P-cadherin modulator; and
   testing whether said P-cadherin modulator is functional as a hair growth modulator.
2. The method of claim 1, wherein said P-cadherin modulator is an antisense oligonucleotide capable of specifically binding to P-cadherin gene, pre-messenger RNA or messenger RNA under physiological conditions.
3. The method of claim 1, wherein said P-cadherin modulator is an antisense construct encoding an antisense transcript capable of specifically binding to P-cadherin gene, pre-messenger RNA or messenger RNA under physiological conditions.
4. The method of claim 1, wherein said P-cadherin modulator is a polynucleotide capable of directing P-cadherin expression in hair follicle cells.
5. The method of claim 1, wherein said P-cadherin modulator is an anti-P-cadherin antibody.
6. The method of claim 1, wherein said P-cadherin modulator is a small molecular weight organic compound.
7. The method of claim 1, wherein said P-cadherin modulator is a peptide.
8. A hair growth modulator identified by the method of claim 1.
9. A method of modulating hair growth comprising administering to a subject in need a therapeutically effective amount of the hair growth modulator of claim 8.
10. A method of identifying a hair growth modulator comprising:
   identifying a molecule being capable of specifically binding to P-cadherin; and
   testing whether said molecule is functional as a hair growth modulator.
11. The method of claim 10, wherein said molecule is an anti-P-cadherin antibody.
12. The method of claim 10, wherein said molecule is an a small molecular weight organic compound.
13. The method of claim 10, wherein said molecule is a peptide.
14. A hair growth modulator identified by the method of claim 10.
15. A method of modulating hair growth comprising administering to a subject in need a therapeutically effective amount of the hair growth modulator of claim 14.
16. The method of claim 10, wherein identifying said molecule being capable of specifically binding to P-cadherin is by a two hybrid system.
17. A method of identifying a hair growth inhibitor comprising:
   identifying a P-cadherin inhibitor; and
   testing whether said P-cadherin inhibitor is functional as a hair growth inhibitor.
18. The method of claim 17, wherein said P-cadherin inhibitor is an antisense oligonucleotide capable of specifically binding to P-cadherin gene, pre-messenger RNA or messenger RNA under physiological conditions.
19. The method of claim 17, wherein said P-cadherin inhibitor is an antisense construct encoding an antisense transcript capable of specifically binding to P-cadherin gene, pre-messenger RNA or messenger RNA under physiological conditions.
20. The method of claim 17, wherein said P-cadherin inhibitor is an anti-P-cadherin antibody.
21. The method of claim 17, wherein said P-cadherin inhibitor is an a small molecular weight organic compound.
22. The method of claim 17, wherein said P-cadherin inhibitor is a peptide.
23. A hair growth inhibitor identified by the method of claim 17.
24. A method of inhibiting hair growth comprising administering to a subject in need a therapeutically effective amount of the hair growth inhibitor of claim 23.
25. A method of identifying a hair growth inhibitor comprising:
   identifying a molecule being capable of specifically binding to P-cadherin; and
   testing whether said molecule is functional as a hair growth inhibitor.
26. The method of claim 25, wherein said molecule is an anti-P-cadherin antibody.
27. The method of claim 25, wherein said molecule is an a small molecular weight organic compound.
28. The method of claim 25, wherein said molecule is a peptide.
29. A hair growth inhibitor identified by the method of claim 25.
30. A method of inhibiting hair growth comprising administering to a subject in need a therapeutically effective amount of the hair growth inhibitor of claim 29.
31. The method of claim 26, wherein identifying said molecule being capable of specifically binding to P-cadherin is by a two hybrid system.
32. A method of identifying a hair growth inducer comprising:
   identifying a P-cadherin inducer; and
   testing whether said P-cadherin inducer is functional as a hair growth inducer.
33. The method of claim 32, wherein said P-cadherin inducer is a polynucleotide capable of directing P-cadherin expression in hair follicle cells.
34. The method of claim 32, wherein said P-cadherin inducer is an a small molecular weight organic compound.
35. The method of claim 32, wherein said P-cadherin inducer is a peptide.
36. A hair growth inducer identified by the method of claim 32.
37. A method of inducing hair growth comprising administering to a subject in need a therapeutically effective amount of the hair growth inducer of claim 36.
38. A method of identifying a hair growth inducer comprising:
   identifying a molecule being capable of specifically binding to P-cadherin; and
   testing whether said molecule is functional as a hair growth inducer.
39. The method of claim 38, wherein said molecule is an anti-P-cadherin antibody.
40. The method of claim 38, wherein said molecule is an a small molecular weight organic compound.
41. The method of claim 38, wherein said molecule is a peptide.
42. A hair growth inducer identified by the method of claim 38.
43. A method of inducing hair growth comprising administering to a subject in need a therapeutically effective amount of the hair growth inducer of claim 42.
44. The method of claim 39, wherein identifying said molecule being capable of specifically binding to P-cadherin is by a two hybrid system.
45. A method of modulating hair growth, the method comprising administering to a subject in need a therapeutically effective amount of a P-cadherin modulator functional as a hair growth modulator.
46. The method of claim 45, wherein said P-cadherin modulator is an antisense oligonucleotide capable of specifically binding to P-cadherin gene, pre-messenger RNA or messenger RNA under physiological conditions.
47. The method of claim 45, wherein said P-cadherin modulator is an antisense construct encoding an antisense transcript capable of specifically binding to P-cadherin gene, pre-messenger RNA or messenger RNA under physiological conditions.
48. The method of claim 45, wherein said P-cadherin modulator is a polynucleotide capable of directing P-cadherin expression in hair follicle cells.
49. The method of claim 45, wherein said P-cadherin modulator is an anti-P-cadherin antibody.
50. The method of claim 45, wherein said P-cadherin modulator is an a small molecular weight organic compound.
51. The method of claim 45, wherein said P-cadherin modulator is a peptide.
52. The method of claim 45, further comprising co-administering to the subject a therapeutically effective amount of an additional hair growth modulator.
53. A method of inhibiting hair growth, the method comprising administering to a subject in need a therapeutically effective amount of a P-cadherin inhibitor functional as a hair growth inhibitor.
54. The method of claim 53, wherein said P-cadherin inhibitor is an antisense oligonucleotide capable of specifically binding to P-cadherin gene, pre-messenger RNA or messenger RNA under physiological conditions.
55. The method of claim 53, wherein said P-cadherin inhibitor is an antisense construct encoding an antisense transcript capable of specifically binding to P-cadherin gene, pre-messenger RNA or messenger RNA under physiological conditions.
56. The method of claim 53, wherein said P-cadherin inhibitor is an anti-P-cadherin antibody.
57. The method of claim 53, wherein said P-cadherin inhibitor is an a small molecular weight organic compound.
58. The method of claim 53, wherein said P-cadherin inhibitor is a peptide.
59. The method of claim 53, further comprising co-administering to the subject a therapeutically effective amount of an additional hair growth inhibitor.
60. A method of inducing hair growth, the method comprising administering to a subject in need a therapeutically effective amount of a P-cadherin inducer functional as a hair growth inducer.
61. The method of claim 60, wherein said P-cadherin inducer is a polynucleotide capable of directing P-cadherin expression in hair follicle cells.
62. The method of claim 60, wherein said P-cadherin inducer is an a small molecular weight organic compound.
63. The method of claim 60, wherein said P-cadherin inducer is a peptide.
64. The method of claim 60, further comprising co-administering to the subject a therapeutically effective amount of an additional hair growth inducer.
65. A pharmaceutical composition for modulating hair growth, the pharmaceutical composition comprising, as an active ingredient, a therapeutically effective amount of a P-cadherin modulator functional as a hair growth modulator.
66. The pharmaceutical composition for claim 65, wherein said P-cadherin modulator is an antisense oligonucleotide capable of specifically binding to P-cadherin gene, pre-messenger RNA or messenger RNA under physiological conditions.
67. The pharmaceutical composition for claim 65, wherein said P-cadherin modulator is an antisense oligonucleotide capable of specifically binding to P-cadherin gene, pre-messenger RNA or messenger RNA under physiological conditions.
68. The pharmaceutical composition for claim 65, wherein said P-cadherin modulator is a polynucleotide capable of directing P-cadherin expression in hair follicle cells.
69. The pharmaceutical composition for claim 65, wherein said P-cadherin modulator is an anti-P-cadherin antibody.
70. The pharmaceutical composition for claim 65, wherein said P-cadherin modulator is an a small molecular weight organic compound.
71. The pharmaceutical composition for claim 65, wherein said P-cadherin modulator is a peptide.
72. The pharmaceutical composition for claim 65, further comprising, as an additional active ingredient, a therapeutically effective amount of an additional hair growth modulator.
73. A pharmaceutical composition for inhibiting hair growth, the pharmaceutical composition comprising, as an active ingredient, a therapeutically effective amount of a P-cadherin inhibitor functional as a hair growth inhibitor.
74. The pharmaceutical composition for claim 73, wherein said P-cadherin inhibitor is an antisense oligonucleotide capable of specifically binding to P-cadherin gene, pre-messenger RNA or messenger RNA under physiological conditions.
75. The pharmaceutical composition for claim 73, wherein said P-cadherin inhibitor is an antisense construct encoding an antisense transcript capable of specifically binding to P-cadherin gene, pre-messenger RNA or messenger RNA under physiological conditions.
76. The pharmaceutical composition for claim 73, wherein said P-cadherin inhibitor is an anti-P-cadherin antibody.
77. The pharmaceutical composition for claim 73, wherein said P-cadherin inhibitor is an a small molecular weight organic compound.
78. The pharmaceutical composition for claim 73, wherein said P-cadherin inhibitor is a peptide.
79. The pharmaceutical composition for claim 73, further comprising, as an additional active ingredient, a therapeutically effective amount of an additional hair growth inhibitor.
80. A pharmaceutical composition for inducing hair growth, the pharmaceutical composition comprising, as an active ingredient, a therapeutically effective amount of a P-cadherin inducer functional as a hair growth inducer.
81. The pharmaceutical composition for claim 80, wherein said P-cadherin inducer is a polynucleotide capable of directing P-cadherin expression in hair follicle cells.
82. The pharmaceutical composition for claim 80, wherein said P-cadherin inducer is an a small molecular weight organic compound.
83. The pharmaceutical composition for claim 80, wherein said P-cadherin inducer is a peptide.
84. The pharmaceutical composition for claim 80, further comprising, as an additional active ingredient, a therapeutically effective amount of an additional hair growth inducer.

## Claims

1. A method of identifying a hair growth modulator comprising:
identifying a P-cadherin modulator; and
testing whether said P-cadherin modulator is functional as a hair growth modulator.

2. The method of Claim 1, wherein said P-cadherin modulator is an antisense oligonucleotide capable of specifically binding to P-cadherin gene, pre-messenger RNA or messenger RNA under physiological conditions or is an antisense construct encoding an antisense transcript capable of specifically binding to P-cadherin gene, pre-messenger RNA or messenger RNA under physiological conditions.

3. The method of Claim 1 or Claim 2, wherein said P-cadherin modulator is a polynucleotide capable of directing P-cadherin expression in hair follicle cells.

4. The method of Claim 1, wherein said P-cadherin modulator is an anti-P-cadherin antibody.

5. The method of Claim 1, wherein said P-cadherin modulator is an a small molecular weight organic compound.

6. The method of Claim 1, wherein said P-cadherin modulator is a peptide.

7. A method of any preceding claim, wherein said modulator is identified as comprising:
a molecule capable of specifically binding to P-cadherins.

8. The method of Claim 7, wherein identifying said molecule as being capable of specifically binding to P-cadherin is by a two hybrid system.

9. A method of any preceding claim, wherein said modulator is identified as P-cadherin an inhibitor and is tested to determine whether said P-cadherin inhibitor is functional as a hair growth inhibitor.

10. A method of any one of Claims 1 to 8, wherein said modulator is identified as a P-cadherin inducer and is tested to determine whether said P-cadherin inducer is functional as a hair growth inducer.

11. A hair growth modulator identified by a method of any preceding claim.

12. A composition for modulating hair growth, the composition comprising, as an active ingredient, an effective amount of a P-cadherin modulator functional as a hair growth modulator.

13. The composition of Claim 12, wherein said P-cadherin modulator is an antisense oligonucleotide capable of specifically binding to P-cadherin gene, pre-messenger RNA or messenger RNA under physiological conditions, or is an antisense constant encoding an antisense transcript capable of specifically binding to P-cadherin gene, pre-messenger RNA or messenger RNA under physiological conditions.

14. The composition of Claim 12, wherein said P-cadherin modulator is a polynucleotide capable of directing P-cadherin expression in hair follicle cells.

15. The composition of Claim 12, wherein said P-cadherin modulator is an anti-P-cadherin antibody.

16. The composition of Claim 12, wherein said P-cadherin modulator is a small molecular weight organic compound.

17. The composition of Claim 12, wherein said P-cadherin modulator is a peptide.

18. The composition of Claim 12, further comprising, as an additional active ingredient, an effective amount of an additional hair growth modulator.

19. A composition of any one of Claims 12 t0 18, for inhibiting hair growth, the composition comprising, as an active ingredient, an effective amount of a P-cadherin inhibitor functional as a hair growth inhibitor.

20. The composition of Claim 19, further comprising, as an additional active ingredient, an effective amount of an additional hair growth inhibitor.

21. A composition of any one of Claims 10 to 18, the composition comprising, as an active ingredient, an effective amount of a P-cadherin inducer functional as a hair growth inducer.

22. The composition of Claim 21, further comprising as an additional active ingredient, an effective amount of an additional hair growth inducer.

23. A hair growth modulator of Claim 11, for use in a method of medical treatment.

24. A method of modulating hair growth for non-therapeutic cosmetic purposes comprising administering to a subject an effective amount of the hair growth modulator of Claim 11 or of a composition of any one of Claims 12 to 22.

25. A hair growth modulator of Claim 11, for use in the manufacture of a medicament for the therapeutic modulation of hair growth.
